# EUROPÄISCHE PATENTSCHRIFT

(11) **EP 2 033 959 B1**
(45) Veröffentlichungstag und Bekanntmachung des Hinweises auf die Patenterteilung: **27.04.2011**
(21) Anmeldenummer: 08014660.8
(22) Anmeldetag: 14.12.2004
(51) Int. Cl.: C07D 401/04, A61K 31/4741, A61P 35/00

(54) **Tetrahydropyranochinolinderivate**
Tetrahydropyranoquinoline derivatives
Tétrahydropyranochinolines

(30) Priorität: 20.12.2003 DE 10360154; 30.01.2004 US 539961 P; 27.05.2004 DE 102004026026
(43) Veröffentlichungstag der Anmeldung: 11.03.2009
(62) Teilanmeldung aus: 04803833.5
(73) Patentinhaber: Merck Patent GmbH, 64293 Darmstadt (DE)
(72) Erfinder: Schiemann, Kai, Dr., 64342 Seeheim-Jugenheim (DE); Emde,Ulrich, Dr., 64291 Darmstadt (DE); Finsinger, Dirk, Dr., 64291 Darmstadt (DE); Gleitz, Johannes, Dr., 64289 Darmstadt (DE); Zenke, Frank, Dr., 64291 Darmstadt (DE); Reubold, Helmut, 64732 Bad Koenig (DE)

(56) Entgegenhaltungen:
- MARIA VITTORIA SPANEDDA ET AL: "Aza- Diels Alder reaction in fluorinated alcohols" TETRAHEDRON LETTERS, Bd. 44, 2003, Seiten 217-219, XP002323173
- RAVINDRANATH, N. ET AL.: "A facile and convenient three-component coupling protocol for the synthesis of Pyrano and Furoquinolines" CHEMISTRY LETTERS, Bd. 32, Nr. 3, 2003, Seiten 222-223, XP002323174
- YUN MA ET AL: "Lanthanide Chloride Catalyzed imino Diels Alder reaction. One-pot synthesis of Pyrano and Furoquinolines" JOURNAL OF ORGANIC CHEMISTRY, Bd. 64, 1999, Seiten 6462-6467, XP002323175

## Beschreibung

### HINTERGRUND DER ERFINDUNG

Der Erfindung lag die Aufgabe zugrunde, neue Verbindungen mit wertvollen Eigenschaften aufzufinden, insbesondere solche, die zur Herstellung von Arzneimitteln verwendet werden können.

Die vorliegende Erfindung betrifft Verbindungen der Formel I A4 und deren Verwendung zur Behandlung und Prophylaxe von Krankheiten, bei denen die Hemmung, Regulierung und/oder Modulation der mitotische Motor-Proteine, insbesondere des mitotischen Motor-Protein Eg5 eine Rolle spielt, ferner pharmazeutische Zusammensetzungen, die diese Verbindungen enthalten.

Im einzelnen betrifft die vorliegende Erfindung Verbindungen der Formel I A4, die die bevorzugt eines oder mehrere mitotische Motor-Proteine hemmen, regulieren und/oder modulieren, Zusammensetzungen, die diese Verbindungen enthalten, sowie Verfahren zu ihrer Verwendung zur Behandlung von Krankheiten und Leiden wie Angiogenese, Krebs, Tumorentstehung, -wachstum und -verbreitung, Arteriosklerose, Augenerkrankungen, choroidale Neovaskularisierung und diabetische Retinopathie, Entzündungserkrankungen, Arthritis, Neurodegeneration, Restenose, Wundheilung oder Transplantatabstossung. Insbesondere eignen sich die erfindungsgemäßen Verbindungen zur Therapie oder Prophylaxe von Krebserkrankungen.

Während der Mitose regulieren verschiedenen Kinesine die Ausbildung und Dynamik des Spindelapparates, der für eine korrekte und koordinierte Ausrichtung und Separation der Chromosomen verantwortlich ist. Es wurde beobachtet, dass eine spezifische Inhibierung eines mitotischen Motor-Proteins - Eg5 - zu einem Kollaps der Spindelfasern führt. Daraus resultiert, dass die Chromosomen nicht mehr korrekt auf die Tochterzellen aufgeteilt werden können. Dies führt zu mitotischem Arrest und kann damit das Absterben der Zelle verursachen. Eine Hochregulierung des Motorproteins Eg5 wurde z.B. in Gewebe von Brust Lungen- und Colon- Tumoren beschrieben. Da Eg5 eine für die Mitose spezifische Funktion einnimmt, sind hauptsächlich sich schnell teilende Zellen und nicht vollständig ausdifferenzierte Zellen von einer Eg5 Inhibierung betroffen. Darüber hinaus regelt Eg5 ausschließlich die Bewegung mitotischer Mikrotubuli (Spindelapparat) und nicht die des Cytoskeletts. Dies ist entscheidend für das Nebenwirkungsprofil der erfindungsgemäßen Verbindungen, da z.B. Neuropathien, wie sie bei Taxol beobachtet werden, nicht oder nur abgeschwächt auftreten. Daher ist die Inhibierung von Eg5 durch die erfindungsgemäßen Verbindungen ein relevantes Therapiekonzept für die Behandlung von malignen Tumoren.

Generell können alle soliden und nicht soliden Tumore mit den Verbindungen der Formel I A4 behandelt werden, wie z.B. die Monozytenleukämie, Hirn-, Urogenital-, Lymphsystem-, Magen-, Kehlkopf-und Lungenkarzinom, darunter Lungenadenokarzinom und kleinzelliges Lungenkarzinom. Zu weiteren Beispielen zählen Prostata-, Bauchspeicheldrüsen- und Brustkarzinom.

Es wurde überraschend gefunden, daß die erfindungsgemäßen Verbindungen eine spezifische Inhibierung der mitotischen Moter-Proteine, insbesondere Eg5 bewirken. Die erfindungsgemäßen Verbindungen zeigen bevorzugt eine vorteilhafte biologische Aktivität, die in den zum Beispiel hierin beschrieben Assays leicht nachweisbar ist. In derartigen Assays zeigen und bewirken die erfindungsgemäßen Verbindungen bevorzugt einen inhibierenden Effekt, der gewöhnlich durch IC₅₀-Werte in einem geeigneten Bereich, bevorzugt im mikromolaren Bereich und bevorzugter im nanomolaren Bereich dokumentiert wird.

Wie hierin besprochen, sind Wirkungen der erfindungsgemäßen Verbindung für verschiedene Erkrankungen relevant. Dementsprechend sind die erfindungsgemäßen Verbindungen nützlich bei der Prophylaxe und/oder Behandlung von Erkrankungen, die durch eine Inhibierung eines oder mehreren mitotischer Motor-Proteine, insbesondere Eg5, beeinflusst werden.

Gegenstand der vorliegenden Erfindung sind deshalb erfindungsgemäße Verbindungen als Arzneimittel und/oder Arzneimittelwirkstoffe bei der Behandlung und/oder Prophylaxe der genannten Erkrankungen und die Verwendung von erfindungsgemäßen Verbindungen zur Herstellung eines Pharmazeutikums für die Behandlung und/oder Prophylaxe der genannten Erkrankungen wie auch ein Verfahren zur Behandlung der genannten Erkrankungen umfassend die Verabreichung eines oder mehrerer erfindungsgemäßer Verbindungen an einen Patienten mit Bedarf an einer derartigen Verabreichung.

Es kann gezeigt werden, dass die erfindungsgemäßen Verbindungen in einem Xenotransplantat-Tumor-Modell eine vorteilhafte Wirkung aufweisen.

Der Wirt oder Patient kann jeglicher Säugerspezies angehören, z. B. einer Primatenspezies, besonders Menschen; Nagetieren, einschließlich Mäusen, Ratten und Hamstern; Kaninchen: Pferden, Rindern, Hunden, Katzen usw. Tiermodelle sind für experimentelle Untersuchungen von Interesse, wobei sie ein Modell zur Behandlung einer Krankheit des Menschen zur Verfügung stellen.

Die Suszeptibilität einer bestimmten Zelle gegenüber der Behandlung mit den erfindungsgemäßen Verbindungen kann durch Testen in vitro bestimmt werden. Typischerweise wird eine Kultur der Zelle mit einer erfindungsgemäßen Verbindung bei verschiedenen Konzentrationen für eine Zeitdauer kombiniert, die ausreicht, um den Wirkstoffen zu ermöglichen, Zelltod zu induzieren oder Migration zu inhibieren, gewöhnlich zwischen ungefähr einer Stunde und einer Woche. Zum Testen in vitro können kultivierte Zellen aus einer Biopsieprobe verwendet werden. Die nach der Behandlung zurückbleibenden lebensfähigen Zellen werden dann gezählt.

Die Dosis variiert abhängig von der verwendeten spezifischen Verbindung, der spezifischen Erkrankung, dem Patientenstatus usw.. Typischerweise ist eine therapeutische Dosis ausreichend, um die unerwünschte Zellpopulation im Zielgewebe erheblich zu vermindern, während die Lebensfähigkeit des Patienten aufrechterhalten wird. Die Behandlung wird im Allgemeinen fortgesetzt, bis eine erhebliche Reduktion vorliegt, z. B. mindestens ca. 50 % Verminderung der Zelllast und kann fortgesetzt werden, bis im Wesentlichen keine unerwünschten Zellen mehr im Körper nachgewiesen werden.

### ZUSAMMENFASSUNG DER ERFINDUNG

Die Erfindung betrifft Verbindungen der Formel I A4 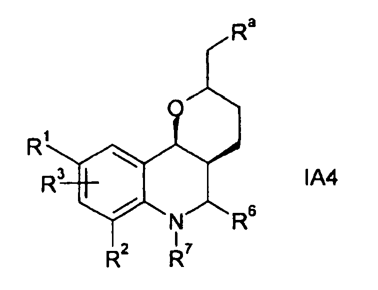 worin
- R¹: A, CF₃, OCF₃, SA, SCN, CH₂CN, -OCOA, Hal oder SCF₃,
- R²: F oder H,
- R³: F oder H,
- R^{a}: 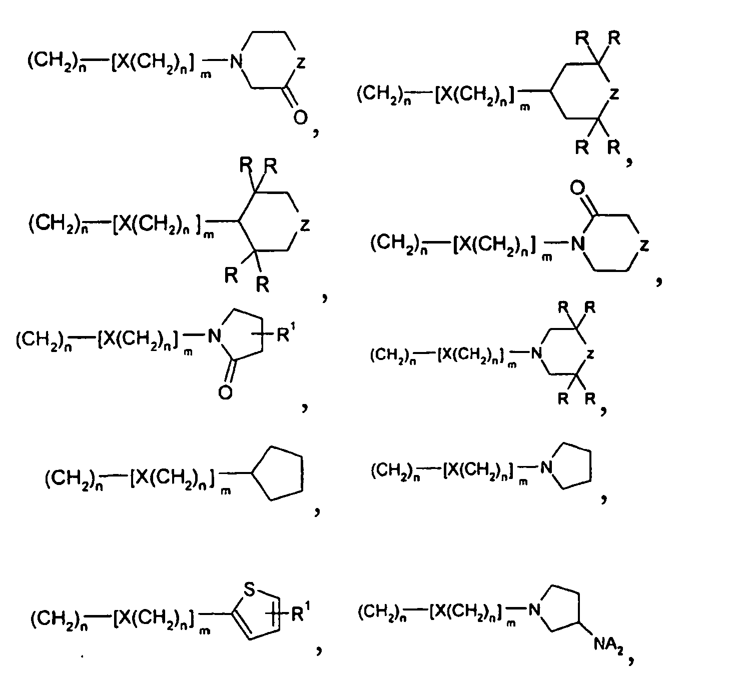 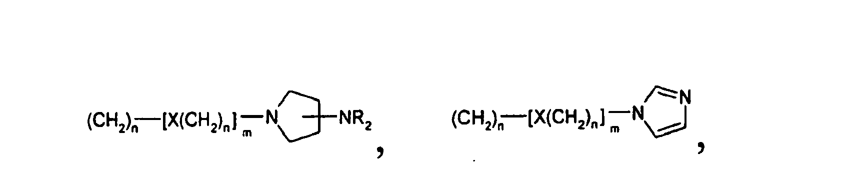 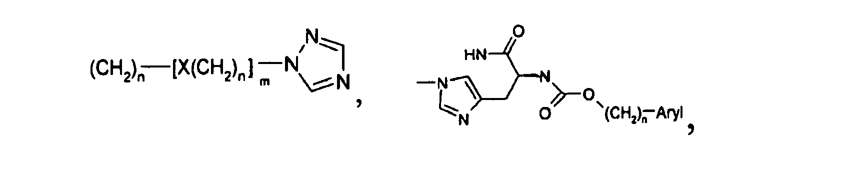 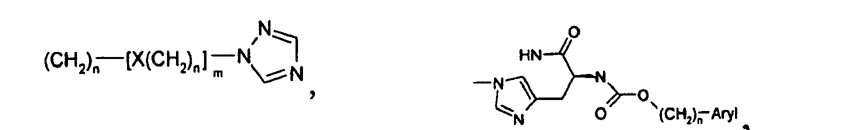 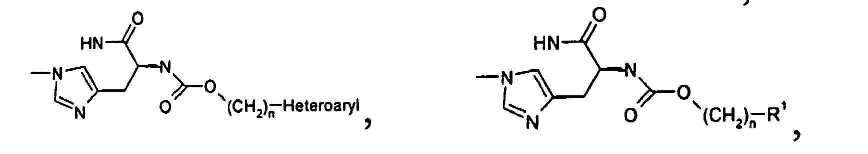 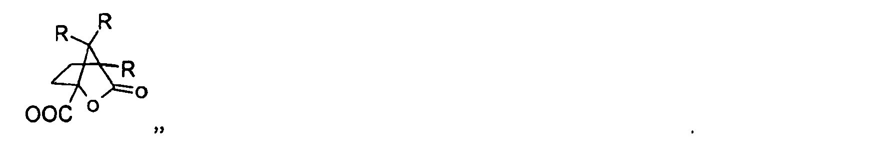 OR, NHR₂, NR₂, OR, NHR₂, NR₂, NR(CH₂),Aryl, NR(CH₂)ₙOR. COOR, N-Pyrrolidon-Rest, OCOR, NR(CH₂)ₙNR₂, N[(CH₂)ₙNR₂]CO(CH₂)ₙAryl, N[(CH₂)ₙNHCOOR]COAryl. R¹, N[CH₂(CH₂)ₙOR]₂, NR(CH₂)ₙNCOOR, X(CH₂)ₙX(CH₂)ₙXR, NR(CH₂)ₙX(CH₂)ₙOH, NR(CH₂)nO(CH₂)ₙOH, (CH₂)ₙCOOR, O(CO)NR(CH₂) nOR, O(CO)(CH₂)ₙNR₂, NR(CH₂)ₙNR₂, N[(CH₂)ₙNR₂]CO(CH₂)ₙAryl, N[(CH₂)ₙXR]CO(CH₂)ₙAryl, N[(CH₂)ₙXR]CO(CH₂)ₙHeteroaryl, N[(CH₂)ₙNR₂]CO(CH₂)ₙHetetoaryl, N[(CH₂)ₙNR₂]CO(CH₂)ₙR¹, N(R)(CH₂)ₙN(R)COOR, A XCOO(CH₂)ₙNR₂, OSO₂A, OSO₂CF₃, OSO₂Ar, OCONR₂, OCH₂(CH₂)ₙNR₂
- R: H oder A, bei geminalen Resten R zusammen auch - (CH₂)₅-, -(CH₂)4-, -(CH₂)ₙ-X-(CH₂)ₙ oder -(CH₂)ₙ-Z-(CH₂)ₙ, Alkyl oder Cycloalkyl, worin eines oder mehrere H-Atome durch Hal ersetzt sein können,
- Hal: F oder Cl,
- X: O, S oder NR,
- Z: CH₂. X, CHCONH₂, CH(CH₂)ₙNRCOOR, CHNRCOOR, NCO, CH(CH₂)ₙCOOR, NCOOR, CH(CH₂)ₙOH, N(CH₂)ₙOH, CHNH₂, CH(CH₂)ₙNR₂, CH(CH₂)ₙNR_{2,} C(OH)R, CHNCOR, CH(CH₂)ₙ-Aryl, CH(CH₂)ₙ-Heteroaryl, CH(CH₂)ₙR¹, N(CH₂)ₙCOOR, CH(CH₂)ₙX(CH₂)ₙ-Aryl, CH(CH₂)ₙX(CH₂)ₙ-Heteroaryl, N(CH₂)ₙCONR₂, XCONR(CH₂)ₙNR₂, N[(CH₂)ₙXCOOR]CO(CH₂)ₙ-Aryl, N[(CH₂)ₙXR]CO(CH₂)ₙ-Aryl, N[(CH₂)ₙXR]CO(CH₂)ₙX-Aryl, N[(CH₂)ₙXR]SO₂(CH₂)ₙ-Aryl, N[(CH₂₎ₙNRCOOR]CO(CH₂)ₙ-Aryl, N[(CH₂),NR₂]CO(CH₂)ₙ-Aryl, N[(CH₂)ₙNR₂]CO(CH₂)ₙNR- Aryl, N[(CH₂)ₙNR₂]SO₂(CH₂)ₙ-Aryl, N[(CH₂)ₙXR]CO(CH₂)ₙ- Heteroaryl, N[(CH₂)ₙXR]CO(CH₂)ₙX-Heteroaryl, N[(CH₂)ₙXR]SO₂(CH₂)ₙ-Heteroaryl, N[(CH₂)ₙNRCOOR]CO(CH₂)ₙ-Heteroaryl, N[(CH₂)ₙNR₂]CO(CH₂)ₙ-Heteroaryl, N[(CH₂)ₙNR₂]CO(CH₂)ₙNR-Heteroaryl, N[(CH₂)ₙNR₂]SO₂(CH₂)ₙ-Heteroaryl, O(CH₂)ₙNR₂, X(CH₂)ₙNR_{2,} NCO(CH₂)ₙNR₂.
- R⁶: unsubstituiertes oder einfach oder mehrfach durch Hal, NO₂, CN, OH, CF₃, OCH(CF₃)₂, OCOCH₃ oder A substituiertes Phenyl, 2-, 3- oder 4-Pyridyl, Pyrimidyl, Furyl oder Thienyl,
- R⁷: H,
- Aryl: unsubstituiertes oder ein-, zwei- oder dreifach durch Hal, A, OH, OA, NH₂, NO₂, CN, COOH, COOA, CONH₂, NHCOA, NHCONH₂, NHSO₂A, CHO, COA, SO₂NH₂, SO₂A, -CH₂-COOH oder -OCH₂-COOH substituiertes Phenyl, Naphthyl oder Biphenyl,
- Heteroaryl: einen ein- oder zweikernigen unsubstituierten oder ein-, zwei- oder dreifach durch Hal, A, NO₂, NHA, NA₂, OA, COOA oder CN substituierten aromatischen Heterocyclus mit einem oder mehreren N-, O- und/oder S-Atomen,
- m: 0, 1 oder 2 und
- n: 0, 1, 2, 3, 4, 5, 6 oder 7
bedeuten,
sowie ihre Solvate, Tautomere, Salze and Stereoisomere, einschließlich deren Mischungen in allen Verhältnissen.

Gegenstand der Erfindung sind auch die optisch aktiven Formen, die Enantiomere, die Racemate, die Diastereomere sowie die Hydrate und Solvate dieser Verbindungen. Unter Solvaten der Verbindungen werden Anlagerungen von inerten Lösungsmittelmolekülen an die Verbindungen der Formel I A4 verstanden, die sich aufgrund ihrer gegenseitigen Anziehungskraft ausbilden. Solvate sind z.B. Mono- oder Dihydrate oder Alkoholate.

Ähnliche Verbindungen sind z.B. in Tetrahedron Lett. 1988, 29, 5855-5858, Tetrahedron Lett. 2003, 44, 217-219, J. Org. Chem. 1997, 62, 4880-4882, J. Org. Chem. 1999, 64, 6462-6467, Chem. Lett. 1995, 423-424, J. Org. Chem. 2000, 65, 5009-5013, Chem. Lett. 2003, 32, 222-223, US2003149069A1 beschrieben, sind aber nicht im Zusammenhang mit Krebsbehandlungen genannt und/oder enthalten nicht die erfindungswesentlichen Merkmale.

Der Ausdruck "wirksame Menge" bedeutet die Menge eines Arzneimittels oder eines pharmazeutischen Wirkstoffes, die eine biologische oder medizinische Antwort in einem Gewebe, System, Tier oder Menschen hervorruft, die z.B. von einem Forscher oder Mediziner gesucht oder erstrebt wird.

Darüberhinaus bedeutet der Ausdruck "therapeutisch wirksame Menge" eine Menge, die in einem Menschen oder einem anderen Säuger mindestens eine der folgenden Wirkungen hervorruft (im Vergleich zu einem Subjekt, das diese Menge nicht erhalten hat): Verbesserung der Heilbehandlung, Heilung, Prävention oder Beseitigung einer Krankheit, eines Krankheitsbildes, eines Krankheitszustandes, eines Leidens, einer Störung oder von Nebenwirkungen oder auch die Verminderung des Fortschreitens einer Krankheit, eines Leidens oder einer Störung.

Die Bezeichnung "therapeutisch wirksame Menge" umfaßt auch die Mengen, die wirkungsvoll sind, die normale physiologische Funktion zu erhöhen oder zu verstärken.

Gegenstand der Erfindung ist auch die Verwendung von Mischungen der Verbindungen der Formel I A4? z.B. Gemische zweier Diastereomerer z.B. im Verhältnis 1:1, 1:2, 1:3, 1:4, 1:5, 1:10, 1:100 oder 1:1000.

Besonders bevorzugt handelt es sich dabei um Mischungen stereoisomerer Verbindungen.

Vor- und nachstehend haben die Reste R, R¹, R², R³, R⁶, R⁷, X, R^{a}, Z, m und n die bei der Formel I A4 angegebenen Bedeutungen, sofern nicht ausdrücklich etwas anderes angegeben ist. Bei mehrfachem Auftreten einzelner Reste innerhalb einer Verbindung nehmen die Reste unabhängig voneinander die angegebenen Bedeutungen an.

A bedeutet Alkyl, ist bevorzugt unverzweigt (linear) oder verzweigt, und hat 1, 2, 3, 4, 5, 6, 7, 8, 9 oder 10 C-Atome. A bedeutet vorzugsweise Methyl, weiterhin Ethyl, Propyl, Isopropyl, Butyl, Isobutyl, sek.-Butyl oder tert.-Butyl, ferner auch Pentyl, 1-, 2- oder 3-Methylbutyl, 1,1- , 1,2- oder 2,2-Dimethylpropyl, 1-Ethylpropyl, Hexyl, 1-, 2-, 3-oder4-Methylpentyl, 1,1-, 1,2-, 1,3-, 2,2- , 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl, weiter bevorzugt z.B. Trifluormethyl.

A bedeutet ganz besonders bevorzugt Alkyl mit 1, 2, 3, 4, 5 oder 6 C-Atomen, vorzugsweise Methyl, Ethyl, Propyl, Isopropyl, Butyl, Isobutyl, sek.-Butyl, tert.-Butyl, Pentyl, Hexyl, Trifluormethyl, Pentafluorethyl oder 1,1,1-Trifluorethyl. A bedeutet auch Cycloalkyl.
Cycloalkyl bedeutet vorzugsweise Cyclopropyl, Cyclobutyl, Cylopentyl, Cyclohexyl oder Cycloheptyl, insbesondere aber Cyclopentyl.

R¹ bedeutet vorzugsweise A, CF₃, OCF₃, SA, SCN, CH₂CN, -OCOA, Hal, SCF₃, bevorzugt auch t-Butyl, -CH(CH₃)CH₂CH₃, Isopropyl, Ethyl oder Methyl. Insbesondere bedeutet R¹ t-Butyl, Isopropyl, Ethyl, CF₃, Methyl, Br, Cl, SCF₃, CH(CH₃)CH₂CH₃, n-Propyl, OCH₃, SCH₃, n-Butyl, -SCN, CH₂CN. Besonders bevorzugt bedeutet R¹ t-Butyl, Isopropyl, Ethyl oder CF₃.

R² bedeutet insbesondere bevorzugt H oder F.

R³ steht bevorzugt in 5-Position. Insbesondere bedeutet R³ H oder F.

In besonders bevorzugten Verbindungen der Förmel I weisen R² und R³ gleichzeitig die Bedeutung H auf. In weiteren bevorzugten Verbindungen der Formel I weist einer der Reste R² und R³ die Bedeutung H und der andere Rest die Bedeutung F auf.

R^{a} bedeutet vorzugsweise 1-Piperazinyl, N-Morpholinyl, NHR oder NR₂.

Bei mehrfachem Auftreten der Reste und Indices wie z. B. n können die Reste und Indices unabhängig voneinander unterschiedliche Werte annehmen. In den Resten R^{a} bedeutet n vorzugsweise 2 oder 0 und m vorzugsweise 0.

R⁶ bedeutet vorzugsweise unsubstituiertes oder einfach oder mehrfach durch Hal, CN, NO₂, OH, CF₃, OCH(CF₃)₂, OCOCH₃ oder A substituiertes Phenyl, 2-, 3- oder 4-Pyridyl, Pyrimidyl, Furyl oder Thienyl. Vorzugsweise ist R⁶ kein heteroaromatischer Rest. Insbesondere bedeutet R⁶ eine der folgenden Gruppen: 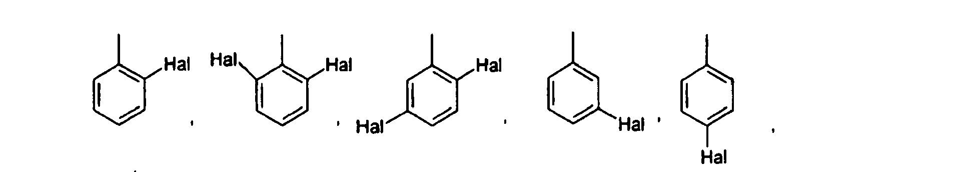 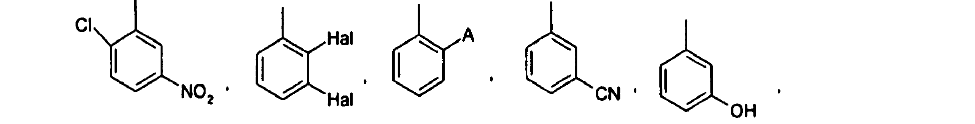 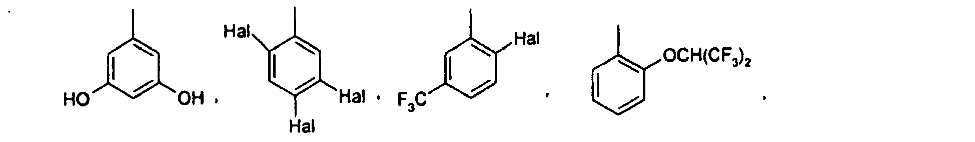 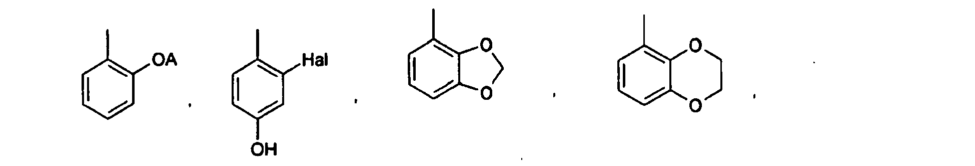 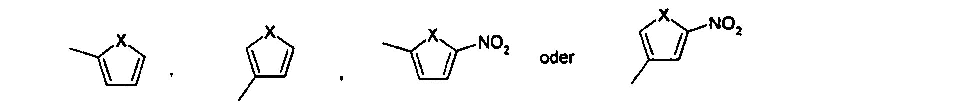 worin
- X: O, S oder NR und insbesondere O oder S bedeutet, A die oben angegebene Bedeutung aufweist, bevorzugt aber Methyl bedeutet und Hal bevorzugt F oder Cl bedeutet.

Weiterhin sind insbesondere Verbindungen der Formel I A4 bevorzugt, worin R⁶ eine der folgenden Bedeutungen aufweist:

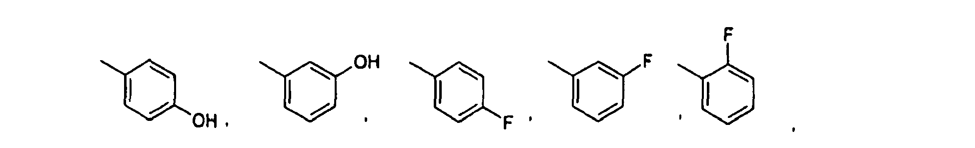

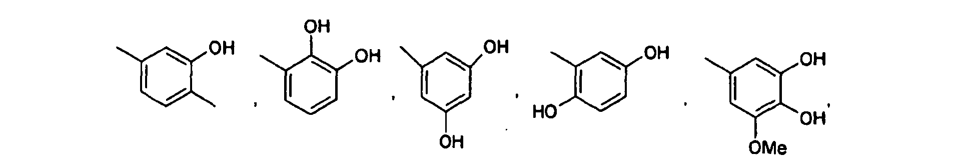

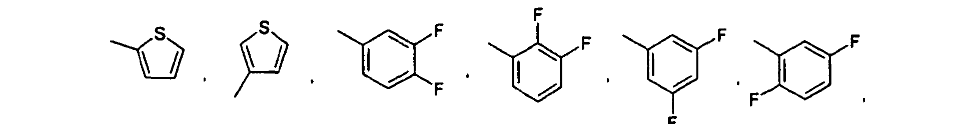

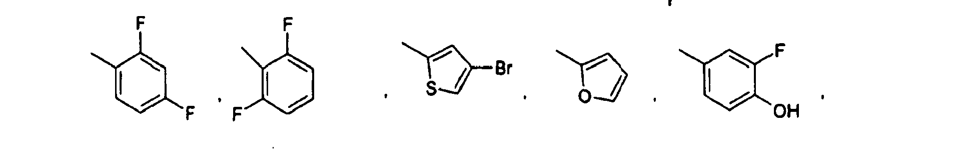

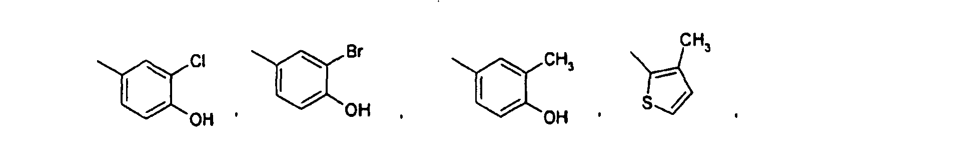

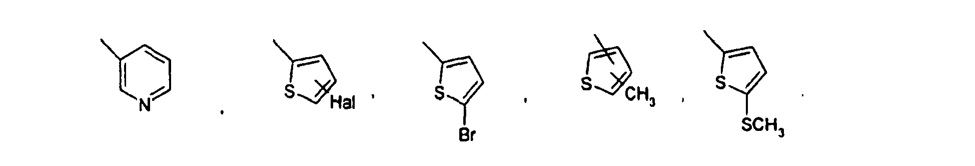

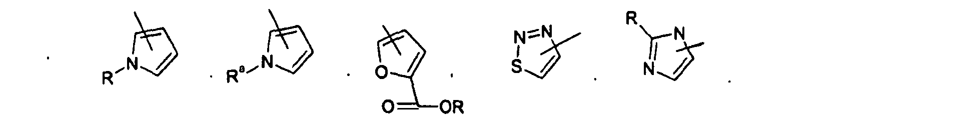

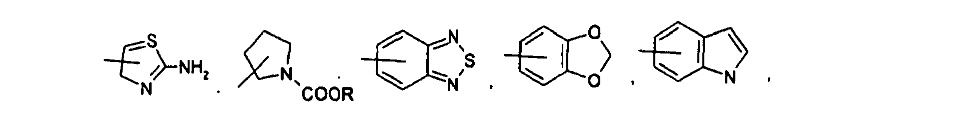

R⁷ bedeutet insbesondere H.

Aryl bedeutet vorzugsweise unsubstituiertes oder ein-, zwei- oder dreifach durch Hal, A, OH, OA, NH₂, NO₂, CN, COOH, COOA, CONH₂, NHCOA, NHCONH₂, NHSO₂A, CHO, COA, SO₂NH₂, SO₂A, -CH₂-COOH oder -OCH₂-COOH substituiertes Phenyl, Naphthyl oder Biphenyl.

Aryl bedeutet bevorzugt Phenyl, o-, m- oder p-Tolyl, o-, m- oder p-Ethylphenyl, o-, m- oder p-Propylphenyl, o-, m- oder p-Isopropylphenyl, o-, m-oder p-tert.-Butylphenyl, o-, m- oder p-Hydroxyphenyl, o-, m- oder p-Methoxyphenyl, o-, m- oder p-Nitrophenyl, o-, m- oder p-Aminophenyl, o-, m- oder p-(N-Methylarnino)-phenyl, o-, m- oder p-(N-Methylaminocarbonyl)-phenyl, o-, m- oder p-Acetamidophenyl, o-, m- oder p-Methoxyphenyl, o-, m- oder p-Ethoxyphenyl, o-, m- oder p-Ethoxycarbonylphenyl, o-, m- oder p-(N,N-Dimethylamino)-phenyl, o-, m- oder p-(N,N-Dimethylaminocarbonyl)-phenyl, o-, m- oder p-(N-Ethylamino)-phenyl, o-, m- oder p-(N,N-Diethylamino)-phenyl, o-, m- oder p-Fluorphenyl, o-, m- oder p-Bromphenyl, o-, m- oder p- Chlorphenyl, o-, m- oder p-(Methylsulfonamido)-phenyl, o-, m- oder p-(Methylsulfonyl)-phenyl, weiter bevorzugt 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2,4- oder 2,5-Dinitrophenyl, 2,5- oder 3,4-Dimethoxyphenyl, 3-Nitro-4-chlorphenyl, 3-Amino-4-chlor-, 2-Amino-3-chlor-, 2-Amino-4-chlor-, 2-Amino-5-chlor- oder 2-Amino-6-chlorphenyl, 2-Nitro-4-N,N-dimethylamino- oder 3-Nitro-4-N,N-dimethylaminophenyl, 2,3-Diaminophenyl, 2,3,4-, 2,3,5-, 2,3,6-, 2,4,6- oder 3,4,5-Trichlorphenyl, 2,4,6-Trimethoxyphenyl, 2-Hydroxy-3,5-dichlorphenyl, p-lodphenyl, 3,6-Dichlor-4-aminophenyl, 4-Fluor-3-chlorphenyl, 2-Fluor-4-bromphenyl, 2,5-Difluor-4-bromphenyl, 3-Brom-6-methoxyphenyl, 3-Chlor-6-methoxyphenyl, 3-Chlor-4-acetamidophenyl, 3-Fluor-4-methoxyphenyl, 3-Amino-6-methylphenyl, 3-Chlor-4-acetamidophenyl oder 2,5-Dimethyl-4-chlorphenyl.

Heteroaryl bedeutet vorzugsweise einen ein- oder zweikernigen unsubstituierten oder ein-, zwei- oder dreifach durch Hal, A, NO₂, NHA, NA₂, OA, COOA oder CN substituierten aromatischen Heterocyclus mit einem oder mehreren N-, O- und/oder S-Atomen.

Heteroaryl bedeutet besonders bevorzugt einen einkernigen gesättigten oder aromatischen Heterocyclus mit einem N, S oder O-Atom, der unsubstituiert oder ein-, zwei- oder dreifach durch Hal, A, NHA, NA₂, NO₂, COOA oder Benzyl substituiert sein kann.

Ungeachtet weiterer Substitutionen, bedeutet unsubstituiertes Heteroaryl z.B. 2- oder 3-Furyl, 2- oder 3-Thienyl, 1-, 2- oder 3-Pyrrolyl, 1-, 2, 4- oder 5-Imidazolyl, 1-, 3-, 4- oder 5-Pyrazolyl, 2-, 4- oder 5-Oxazolyl, 3-, 4- oder 5-lsoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, - 4- oder -5-yl, 1,2,4-Triazol-1-, -3- oder 5-yl, 1- oder 5-Tetrazolyl, 1,2,3-Oxadiazol-4- oder -5-yl, 1,2,4-Oxadiazol-3- oder -5-yl, 1,3,4-Thiadiazol-2-oder -5-yl, 1,2,4-Thiadiazol-3- oder -5-yl, 1,2,3-Thiadiazol-4- oder -5-yl, 3-oder 4-Pyridazinyl, Pyrazinyl, 1-, 2-, 3-, 4-, 5-, 6- oder 7-Indolyl, 4- oder 5-Isoindolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7-Benzisoxazolyl, 2-, 4-, 5-, 6- oder 7-Benzothiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7- oder 8-Isochinolyl, 3-, 4-, 5-, 6-, 7-oder 8-Cinnolinyl, 2-, 4-, 5-, 6-, 7- oder 8-Chinazolinyl, 5- oder 6-Chinoxalinyl, 2-, 3-, 5-, 6-, 7- oder 8-2H-Benzo[1,4]oxazinyl, weiter bevorzugt 1,3-Benzodioxol-5-yl, 1,4-Benzodioxan-6-yl, 2,1,3-Benzothiadiazol-4- oder -5-yl oder 2,1,3-Benzoxadiazol-5-yl.

Hal bedeutet vorzugsweise F, Cl oder Br, aber auch I, besonders bevorzugt F oder Cl.

Für die gesamte Erfindung gilt, daß sämtliche Reste, die mehrfach auftreten, gleich oder verschieden sein können, d.h. unabhängig voneinander sind.

Die Verbindungen der Formel I A4 können ein oder mehrere chirale Zentren besitzen und daher in verschiedenen stereoisomeren Formen vorkommen. Die Formel I umschließt alle diese Formen.

Besonders bevorzugte Verbindungen sind die der Teilforme IA4: 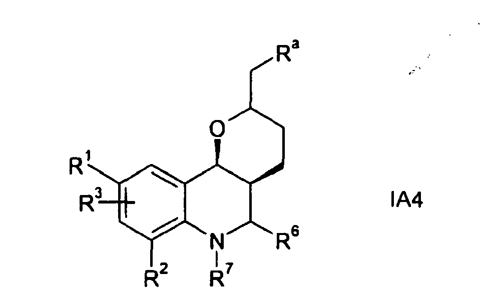 worin R, R^{a}, R¹, R², R³, R⁶ und R⁷ die oben angegebenen Bedeutungen aufweisen.

Dementsprechend sind Gegenstand der Erfindung insbesondere diejenigen Verbindungen der Formel I A4, in denen mindestens einer der genannten Reste eine der vorstehend angegebenen bevorzugten Bedeutungen hat. Einige bevorzugte Gruppen von Verbindungen können durch die folgenden Teilformeln I 12 bis 13a ausgedrückt werden: 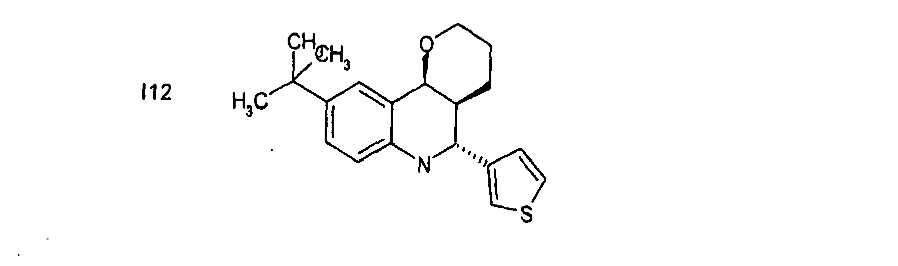 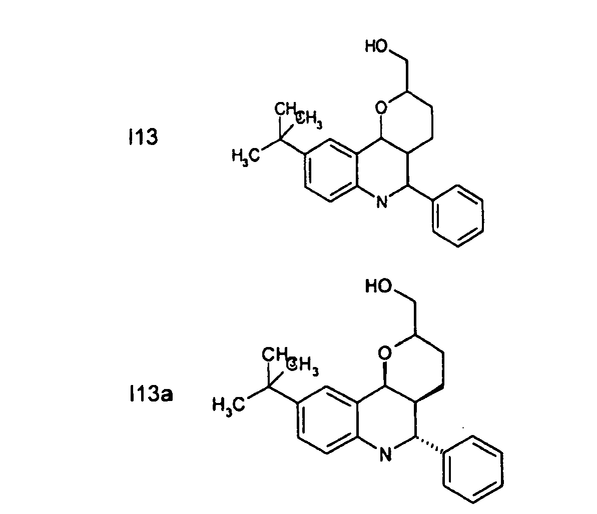 Die Verbindungen der Formel I A4 und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I A4 umsetzt.

Die Umsetzung erfolgt in der Regel in einem inerten Lösungsmittel, bevorzugt in Gegenwart einer Protonensäure oder Lewis-Säure wie TFA, HFIP, Bismut(III)-Salzen, Ytterhium(III)-Salzen oder CAN. Die Reaktionszeit liegt je nach den angewendeten Bedingungen zwischen einigen Minuten und 14 Tagen, die Reaktionstemperatur zwischen etwa 0° und 180°, normalerweise zwischen 0° und 100°, besonders bevorzugt zwischen 15° und 35°C.

Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Chloroform oder Dichlormethan; Nitrile wie Acetonitril; Schwefelkohlenstoff; Carbonsäuren wie Ameisensäure oder Essigsäure; Nitroverbindungen wie Nitromethan oder Nitrobenzol oder Gemische der genannten Lösungsmittel. Gewünschtenfalls kann in einer Verbindung der Formel IA4 eine funktionell abgewandelte Amino- und /oder Hydroxygruppe durch Solvolyse oder Hydrogenolyse nach üblichen Methoden in Freiheit gesetzt werden. Dies kann z.B. mit NaOH oder KOH in Wasser, Wasser-THF oder Wasser-Dioxan bei Temperaturen zwischen 0 und 100° erfolgen.

Die Reduktion eines Esters zum Aldehyd oder zum Alkohol, oder die Reduktion eines Nitrils zum Aldehyd oder Amin erfolgt nach Methoden wie sie dem Fachmann bekannt sind und in Standardwerken der organischen Chemie beschrieben sind.

Die genannten erfindungsgemäßen Verbindungen lassen sich in ihrer endgültigen Nichtsalzform verwenden. Andererseits umfaßt die vorliegende Erfindung auch die Verwendung dieser Verbindungen in Form ihrer pharmazeutisch unbedenklichen Salze, die von verschiedenen organischen und anorganischen Säuren und Basen nach fachbekannten Vorgehensweisen abgeleitet werden können. Pharmazeutisch unbedenkliche Salzformen der Verbindungen der Formel IA4 werden größtenteils konventionell hergestellt. Sofern die Verbindung der Formel I A4 eine Carbonsäuregruppe enthält, läßt sich eines ihrer geeigneten Salze dadurch bilden, daß man die Verbindung mit einer geeigneten Base zum entsprechenden Basenadditionssalz umsetzt. Solche Basen sind zum Beispiel Alkalimetallhydroxide, darunter Kaliumhydroxid, Natriumhydroxid und Lithiumhydroxid; Erdalkalimetallhydroxide wie Bariumhydroxid und Calciumhydroxid; Alkalimetallalkoholate, z.B. Kaliumethanolat und Natriumpropanolat; sowie verschiedene organische Basen wie Piperidin, Diethanolamin und N-Methylglutamin. Die Aluminiumsalze der Verbindungen der Formel I A4 zählen ebenfalls dazu. Bei bestimmten Verbindungen der Formel I A4 lassen sich Säureadditionssalze dadurch bilden, daß man diese Verbindungen mit pharmazeutisch unbedenklichen organischen und anorganischen Säuren, z.B. Halogenwasserstoffen wie Chlorwasserstoff, Bromwasserstoff oder Jodwasserstoff, anderen Mineralsäuren und ihren entsprechenden Salzen wie Sulfat, Nitrat oder Phosphat und dergleichen sowie Alkyl- und Monoarylsulfonaten wie Ethansulfonat, Toluolsulfonat und Benzolsulfonat, sowie anderen organischen Säuren und ihren entsprechenden Salzen wie Acetat, Trifluoracetat, Tartrat, Maleat, Succinat, Citrat, Benzoat, Salicylat, Ascorbat und dergleichen behandelt. Dementsprechend zählen zu pharmazeutisch unbedenklichen Säureadditionssalzen der Verbindungen der Formel I die folgenden: Acetat, Adipat, Alginat, Arginat, Aspartat, Benzoat, Benzolsulfonat (Besylat), Bisulfat, Bisulfit, Bromid, Butyrat, Kampferat, Kampfersulfonat, Caprylat, Chlorid, Chlorbenzoat, Citrat, Cyclopentanpropionat, Digluconat, Dihydrogenphosphat, Dinitrobenzoat, Dodecylsulfat, Ethansulfonat, Fumarat, Galacterat (aus Schleimsäure), Galacturonat, Glucoheptanoat, Gluconat, Glutamat, Glycerophosphat, Hemisuccinat, Hemisulfat, Heptanoat, Hexanoat, Hippurat, Hydrochlorid, Hydrobromid, Hydroiodid, 2-Hydroxyethansulfonat, lodid, Isethionat, Isobutyrat, Lactat, Lactobionat, Malat, Maleat, Malonat, Mandelat, Metaphosphat, Methansulfonat, Methylbenzoat, Monohydrogenphosphat, 2-Naphthalinsulfonat, Nicotinat, Nitrat, Oxalat, Oleat, Pamoat, Pectinat, Persulfat, Phenylacetat, 3-Phenylpropionat, Phosphat, Phosphonat, Phthalat, was jedoch keine Einschränkung darstellt.

Weiterhin zählen zu den Basensalzen der erfindungsgemäßen Verbindungen Aluminium-, Ammonium-, Calcium-, Kupfer-, Eisen(III)-, Eisen(II)-, Lithium-, Magnesium-, Mangan(III)-, Mangan(II), Kalium-, Natrium- und Zinksalze, was jedoch keine Einschränkung darstellen soll. Bevorzugt unter den oben genannten Salzen sind Ammonium; die Alkalimetallsalze Natrium und Kalium,sowie die Erdalkalimetalsalze Calcium und Magnesium. Zu Salzen der Verbindungen der Formel IA4, die sich von pharmazeutisch unbedenklichen organischen nicht-toxischen Basen ableiten, zählen Salze primärer, sekundärer und tertiärer Amine, substituierter Amine, darunter auch natürlich vorkommender substituierter Amine, cyclischer Amine sowie basischer lonenaustauscherharze, z.B. Arginin, Betain, Koffein, Chlorprocain, Cholin, N,N'-Dibenzylethylendiamin (Benzathin), Dicyclohexylamin, Diethanolamin, Diethylamin, 2-Diethyl-aminoethanol, 2-Dimethylaminoethanol, Ethanolamin, Ethylendiamin, N-Ethylmorpholin, N-Ethylpiperidin, Glucamin, Glucosamin, Histidin, Hydrabamin, Iso-propylamin, Lidocain, Lysin, Meglumin, N-Methyl-D-glucamin, Morpholin, Piperazin, Piperidin, Polyaminharze, Procain, Purine, Theobromin, Triethanolamin, Triethylamin, Trimethylamin, Tripropylamin sowie Tris-(hydroxymethyl)-methylamin (Tromethamin), was jedoch keine Einschränkung darstellen soll.

Verbindungen der vorliegenden Erfindung, die basische stickstoffhaltige Gruppen enthalten, lassen sich mit Mitteln wie (C₁-C₄) Alkylhalogeniden, z.B. Methyl-, Ethyl-, lsopropyl- und tert.-Butylchlorid, -bromid und -iodid; Di(C₁-C₄)Alkylsulfaten, z.B. Dimethyl-, Diethyl- und Diamylsulfat; (C₁₀-C₁₀)Alkylhalogeniden, z.B. Decyl-, Dodecyl-, Lauryl-, Myristyl- und Stearylchlorid, -bromid und -iodid; sowie Aryl-(C₁-C₄)Alkylhalogeniden, z.B. Benzylchlorid und Phenethylbromid, quarternisieren. Mit solchen Salzen können sowohl wasser- als auch öllösliche erfindungsgemäße Verbindungen hergestellt werden.

Zu den oben genannten pharmazeutischen Salzen, die bevorzugt sind, zählen Acetat, Trifluoracetat, Besylat, Citrat, Fumarat, Gluconat, Hemisuccinat, Hippurat, Hydrochlorid, Hydrobromid, Isethionat, Mandelat, Meglumin, Nitrat, Oleat, Phosphonat, Pivalat, Natriumphosphat, Stearat, Sulfat, Sulfosalicylat, Tartrat, Thiomalat, Tosylat und Tromethamin, was jedoch keine Einschränkung darstellen soll.

Die Säureadditionssalze basischer Verbindungen der Formel I A4 werden dadurch hergestellt, daß man die freie Basenform mit einer ausreichenden Menge der gewünschten Säure in Kontakt bringt, wodurch man auf übliche Weise das Salz darstellt. Die freie Base läßt sich durch In-Kontakt-Bringen der Salzform mit einer Base und Isolieren der freien Base auf übliche Weise regenerieren. Die freien Basenformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Basenformen.

Wie erwähnt werden die pharmazeutisch unbedenklichen Basenadditionssalze der Verbindungen der Formel I A4 mit Metallen oder Aminen wie Alkalimetallen und Erdalkalimetallen oder organischen Aminen gebildet. Bevorzugte Metalle sind Natrium, Kalium, Magnesium und Calcium. Bevorzugte organische Amine sind N,N'-Dibenzylethylendiamin, Chlorprocain, Cholin, Diethanolamin, Ethylendiamin, N-Methyl-D-glucamin und Procain.

Die Basenadditionssalze von erfindungsgemäßen sauren Verbindungen werden dadurch hergestellt, daß man die freie Säureform mit einer ausreichenden Menge der gewünschten Base in Kontakt bringt, wodurch man das Salz auf übliche Weise darstellt. Die freie Säure läßt sich durch In-Kontakt-Bringen der Salzform mit einer Säure und Isolieren der freien Säure auf übliche Weise regenerieren. Die freien Säureformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Säureformen.

Enthält eine erfindungsgemäße Verbindung mehr als eine Gruppe, die solche pharmazeutisch unbedenklichen Salze bilden kann, so umfaßt die Erfindung auch mehrfache Salze. Zu typischen mehrfachen Salzformen zählen zum Beispiel Bitartrat, Diacetat, Difumarat, Dimeglumin, Diphosphat, Dinatrium und Trihydrochlorid, was jedoch keine Einschränkung darstellen soll.

Im Hinblick auf das oben Gesagte sieht man, daß unter dem Ausdruck "pharmazeutisch unbedenkliches Salz" im vorliegenden Zusammenhang ein Wirkstoff zu verstehen ist, der eine Verbindung der Formel I A4 in der Form eines ihrer Salze enthält, insbesondere dann, wenn diese Salzform dem Wirkstoff im Vergleich zu der freien Form des Wirkstoffs oder irgendeiner anderen Salzform des Wirkstoffs, die früher verwendet wurde, verbesserte pharmakokinetische Eigenschaften verleiht. Die pharmazeutisch unbedenkliche Salzform des Wirkstoffs kann auch diesem Wirkstoff erst eine gewünschte pharmakokinetische Eigenschaft verleihen, über die er früher nicht verfügt hat, und kann sogar die Pharmakodynamik dieses Wirkstoffs in bezug auf seine therapeutische Wirksamkeit im Körper positiv beeinflussen.

Gegenstand der Erfindung sind ferner Arzneimittel, enthaltend mindestens eine Verbindung der Formel I A4 und/oder ihre Solvate und Stereoisomere, einschließlich deren Mischungen in allen Verhältnissen, sowie gegebenenfalls Träger- und/oder Hilfsstoffe.

Pharmazeutische Formulierungen können in Form von Dosiseinheiten, die eine vorbestimmte Menge an Wirkstoff pro Dosiseinheit enthalten, dargereicht werden. Eine solche Einheit kann beispielsweise 0,5 mg bis 1 g, vorzugsweise 1 mg bis 700 mg, besonders bevorzugt 5 mg bis 100 mg einer erfindungsgemäßen Verbindung enthalten, je nach dem behandelten Krankheitszustand, dem Verabreichungsweg und dem Alter, Gewicht und Zustand des Patienten, oder pharmazeutische Formulierungen können in Form von Dosiseinheiten, die eine vorbestimmte Menge an Wirkstoff pro Dosiseinheit enthalten, dargereicht werden. Bevorzugte Dosierungseinheitsformulierungen sind solche, die eine Tagesdosis oder Teildosis, wie oben angegeben, oder einen entsprechenden Bruchteil davon eines Wirkstoffs enthalten. Weiterhin lassen sich solche pharmazeutischen Formulierungen mit einem der im pharmazeutischen Fachgebiet allgemein bekannten Verfahren herstellen.

Pharmazeutische Formulierungen lassen sich zur Verabreichung über einen beliebigen geeigneten Weg, beispielsweise auf oralem (einschließlich buccalem bzw. sublingualem), rektalem, nasalem, topischem (einschließlich buccalem, sublingualem oder transdermalem), vaginalem oder parenteralem (einschließlich subkutanem, intramuskulärem, intravenösem oder intradermalem) Wege, anpassen. Solche Formulierungen können mit allen im pharmazeutischen Fachgebiet bekannten Verfahren hergestellt werden, indem beispielsweise der Wirkstoff mit dem bzw. den Trägerstoff(en) oder Hilfsstoff(en) zusammengebracht wird.

An die orale Verabreichung angepaßte pharmazeutische Formulierungen können als separate Einheiten, wie z.B. Kapseln oder Tabletten; Pulver oder Granulate; Lösungen oder Suspensionen in wäßrigen oder nichtwäßrigen Flüssigkeiten; eßbare Schäume oder Schaumspeisen; oder Öl-in-Wasser-Flüssigemulsionen oder Wasser-in-Öl-Flüssigemulsionen dargereicht werden.

So läßt sich beispielsweise bei der oralen Verabreichung in Form einer Tablette oder Kapsel die Wirkstoffkomponente mit einem oralen, nicht-toxischen und pharmazeutisch unbedenklichen inerten Trägerstoff, wie z.B. Ethanol, Glyzerin, Wasser u.ä. kombinieren. Pulver werden hergestellt, indem die Verbindung auf eine geeignete feine Größe zerkleinert und mit einem in ähnlicher Weise zerkleinerten pharmazeutischen Trägerstoff, wie z.B. einem eßbaren Kohlenhydrat wie beispielsweise Stärke oder Mannit vermischt wird. Ein Geschmacksstoff, Konservierungsmittel, Dispersionsmittel und Farbstoff können ebenfalls vorhanden sein.

Kapseln werden hergestellt, indem ein Pulvergemisch wie oben beschrieben hergestellt und geformte Gelatinehüllen damit gefüllt werden. Gleit- und Schmiermittel wie z.B. hochdisperse Kieselsäure, Talkum, Magnesiumstearat, Kalziumstearat oder Polyethylenglykol in Festform können dem Pulvergemisch vor dem Füllvorgang zugesetzt werden. Ein Sprengmittel oder Lösungsvermittler, wie z.B. Agar-Agar, Kalziumcarbonat oder Natriumcarbonat, kann ebenfalls zugesetzt werden, um die Verfügbarkeit des Medikaments nach Einnahme der Kapsel zu verbessern.

Außerdem können, falls gewünscht oder notwendig, geeignete Bindungs-, Schmier- und Sprengmittel sowie Farbstoffe ebenfalls in das Gemisch eingearbeitet werden. Zu den geeigneten Bindemitteln gehören Stärke, Gelatine, natürliche Zucker, wie z.B. Glukose oder Beta-Lactose, Süßstoffe aus Mais, natürliche und synthetische Gummi, wie z.B. Akazia, Traganth oder Natriumalginat, Carboxymethylzellulose, Polyethylenglykol, Wachse, u.ä. Zu den in diesen Dosierungsformen verwendeten Schmiermitteln gehören Natriumoleat, Natriumstearat, Magnesiumstearat, Natriumbenzoat, Natriumacetat, Natriumchlorid u.ä. Zu den Sprengmitteln gehören, ohne darauf beschränkt zu sein, Stärke, Methylzellulose, Agar, Bentonit, Xanthangummi u.ä. Die Tabletten werden formuliert, indem beispielsweise ein Pulvergemisch hergestellt, granuliert oder trockenverpreßt wird, ein Schmiermittel und ein Sprengmittel zugegeben werden und das Ganze zu Tabletten verpreßt wird. Ein Pulvergemisch wird hergestellt, indem die in geeigneter Weise zerkleinerte Verbindung mit einem Verdünnungsmittel oder einer Base, wie oben beschrieben, und gegebenenfalls mit einem Bindemittel, wie z.B. Carboxymethylzellulose, einem Alginat, Gelatine oder Polyvinylpyrrolidon, einem Lösungsverlangsamer, wie z.B. Paraffin, einem Resorptionsbeschleuniger, wie z.B. einem quaternären Salz und/oder einem Absorptionsmittel, wie z.B. Bentonit, Kaolin oder Dikalziumphosphat, vermischt wird. Das Pulvergemisch läßt sich granulieren, indem es mit einem Bindemittel, wie z.B. Sirup, Stärkepaste, Acadia-Schleim oder Lösungen aus Zellulose- oder Polymermaterialen benetzt und durch ein Sieb gepreßt wird. Als Alternative zur Granulierung kann man das Pulvergemisch durch eine Tablettiermaschine laufen lassen, wobei ungleichmäßig geformte Klumpen entstehen, die in Granulate aufgebrochen werden. Die Granulate können mittels Zugabe von Stearinsäure, einem Stearatsalz, Talkum oder Mineralöl gefettet werden, um ein Kleben an den Tablettengußformen zu verhindern. Das gefettete Gemisch wird dann zu Tabletten verpreßt. Die erfindungsgemäßen Verbindungen können auch mit einem freifließenden inerten Trägerstoff kombiniert und dann ohne Durchführung der Granulierungs- oder Trockenverpressungsschritte direkt zu Tabletten verpreßt werden. Eine durchsichtige oder undurchsichtige Schutzschicht, bestehend aus einer Versiegelung aus Schellack, einer Schicht aus Zucker oder Polymermaterial und einer Glanzschicht aus Wachs, kann vorhanden sein. Diesen Beschichtungen können Farbstoffe zugesetzt werden, um zwischen unterschiedlichen Dosierungseinheiten unterscheiden zu können.

Orale Flüssigkeiten, wie z.B. Lösung, Sirupe und Elixiere, können in Form von Dosierungseinheiten hergestellt werden, so daß eine gegebene Quantität eine vorgegebene Menge der Verbindung enthält. Sirupe lassen sich herstellen, indem die Verbindung in einer wäßrigen Lösung mit geeignetem Geschmack gelöst wird, während Elixiere unter Verwendung eines nichttoxischen alkoholischen Vehikels hergestellt werden. Suspensionen können durch Dispersion der Verbindung in einem nicht-toxischen Vehikel formuliert werden. Lösungsvermittler und Emulgiermittel, wie z.B. ethoxylierte Isostearylalkohole und Polyoxyethylensorbitolether, Konservierungsmittel, Geschmackszusätze, wie z.B. Pfefferminzöl oder natürliche Süßstoffe oder Saccharin oder andere künstliche Süßstoffe, u.ä. können ebenfalls zugegeben werden.

Die Dosierungseinheitsformulierungen für die orale Verabreichung können gegebenenfalls in Mikrokapseln eingeschlossen werden. Die Formulierung läßt sich auch so herstellen, daß die Freisetzung verlängert oder retardiert wird, wie beispielsweise durch Beschichtung oder Einbettung von partikulärem Material in Polymere, Wachs u.ä.

Die Verbindungen der Formel I A4 sowie Salze und, Solvate lassen sich auch in Form von Liposomenzuführsystemen, wie z.B. kleinen unilamellaren Vesikeln, großen unilamellaren Vesikeln und multilamellaren Vesikeln, verabreichen. Liposomen können aus verschiedenen Phospholipiden, wie z.B. Cholesterin, Stearylamin oder Phosphatidylcholinen, gebildet werden.

Die Verbindungen der Formel IA4 sowie die Salze, Solvate können auch unter Verwendung monoklonaler Antikörper als individuelle Träger, an die die Verbindungsmoleküle gekoppelt werden, zugeführt werden. Die Verbindungen können auch mit löslichen Polymeren als zielgerichtete Arzneistoffträger gekoppelt werden. Solche Polymere können Polyvinylpyrrolidon, Pyran-Copolymer, Polyhydroxypropylmethacrylamidphenol, Polyhydroxyethylaspartamidphenol oder Polyethylenoxidpolylysin, substituiert mit Palmitoylresten, umfassen. Weiterhin können die Verbindungen an eine Klasse von biologisch abbaubaren Polymeren, die zur Erzielung einer kontrollierten Freisetzung eines Arzneistoffs geeignet sind, z.B. Polymilchsäure, Polyepsilon-Caprolacton, Polyhydroxybuttersäure, Polyorthoester, Polyacetale, Polydihydroxypyrane, Polycyanoacrylate und quervernetzte oder amphipatische Blockcopolymere von Hydrogelen, gekoppelt sein.

An die transdermale Verabreichung angepaßte pharmazeutische Formulierungen können als eigenständige Pflaster für längeren, engen Kontakt mit der Epidermis des Empfängers dargereicht werden. So kann beispielsweise der Wirkstoff aus dem Pflaster mittels lontophorese zugeführt werden, wie in Pharmaceutical Research, 3(6), 318 (1986) allgemein beschrieben.

An die topische Verabreichung angepaßte pharmazeutische Verbindungen können als Salben, Cremes, Suspensionen, Lotionen, Pulver, Lösungen, Pasten, Gele, Sprays, Aerosole oder Öle formuliert sein.

Für Behandlungen des Auges oder anderer äußerer Gewebe, z.B. Mund und Haut, werden die Formulierungen vorzugsweise als topische Salbe oder Creme appliziert. Bei Formulierung zu einer Salbe kann der Wirkstoff entweder mit einer paraffinischen oder einer mit Wasser mischbaren Cremebasis eingesetzt werden. Alternativ kann der Wirkstoff zu einer Creme mit einer Öl-in-Wasser-Cremebasis oder einer Wasser-in-Öl-Basis formuliert werden.

Zu den an die topische Applikation am Auge angepaßten pharmazeutischen Formulierungen gehören Augentropfen, wobei der Wirkstoff in einem geeigneten Träger, insbesondere einem wäßrigen Lösungsmittel, gelöst oder suspendiert ist.

An die topische Applikation im Mund angepaßte pharmazeutische Formulierungen umfassen Lutschtabletten, Pastillen und Mundspülmittel.

An die rektale Verabreichung angepaßte pharmazeutische Formulierungen können in Form von Zäpfchen oder Einläufen dargereicht werden.

An die nasale Verabreichung angepaßte pharmazeutische Formulierungen, in denen die Trägersubstanz ein Feststoff ist, enthalten ein grobes Pulver mit einer Teilchengröße beispielsweise im Bereich von 20-500 Mikrometern, das in der Art und Weise, wie Schnupftabak aufgenommen wird, verabreicht wird, d.h. durch Schnellinhalation über die Nasenwege aus einem dicht an die Nase gehaltenen Behälter mit dem Pulver. Geeignete Formulierungen zur Verabreichung als Nasenspray oder Nasentropfen mit einer Flüssigkeit als Trägersubstanz umfassen Wirkstofflösungen in Wasser oder Öl.

An die Verabreichung durch Inhalation angepaßte pharmazeutische Formulierungen umfassen feinpartikuläre Stäube oder Nebel, die mittels verschiedener Arten von unter Druck stehenden Dosierspendern mit Aerosolen, Verneblern oder Insufflatoren erzeugt werden können.

An die vaginale Verabreichung angepaßte pharmazeutische Formulierungen können als Pessare, Tampons, Cremes, Gele, Pasten, Schäume oder Sprayformulierungen dargereicht werden.

Zu den an die parenterale Verabreichung angepaßten pharmazeutischen Formulierungen gehören wäßrige und nichtwäßrige sterile Injektionslösungen, die Antioxidantien, Puffer, Bakteriostatika und Solute, durch die die Formulierung isotonisch mit dem Blut des zu behandelnden Empfängers gemacht wird, enthalten; sowie wäßrige und nichtwäßrige sterile Suspensionen, die Suspensionsmittel und Verdicker enthalten können. Die Formulierungen können in Einzeldosis- oder Mehrfachdosisbehältern, z.B. versiegelten Ampullen und Fläschchen, dargereicht und in gefriergetrocknetem (lyophilisiertem) Zustand gelagert werden, so daß nur die Zugabe der sterilen Trägerflüssigkeit, z.B. Wasser für Injektionszwecke, unmittelbar vor Gebrauch erforderlich ist. Rezepturmäßig hergestellte Injektionslösungen und Suspensionen können aus sterilen Pulvern, Granulaten und Tabletten hergestellt werden.

Es versteht sich, daß die Formulierungen neben den obigen besonders erwähnten Bestandteilen andere im Fachgebiet übliche Mittel mit Bezug auf die jeweilige Art der Formulierung enthalten können; so können beispielsweise für die orale Verabreichung geeignete Formulierungen Geschmacksstoffe enthalten.

Eine therapeutisch wirksame Menge einer Verbindung der Formel I A4 hängt von einer Reihe von Faktoren ab, einschließlich z.B. dem Alter und Gewicht des Tiers, dem exakten Krankheitszustand, der der Behandlung bedarf, sowie seines Schweregrads, der Beschaffenheit der Formulierung sowie dem Verabreichungsweg, und wird letztendlich von dem behandelnden Arzt bzw. Tierarzt festgelegt. Jedoch liegt eine wirksame Menge einer erfindungsgemäßen Verbindung für die Behandlung von neoplastischem Wachstum, z.B. Dickdarm- oder Brustkarzinom, im allgemeinen im Bereich von 0,1 bis 100 mg/kg Körpergewicht des Empfängers (Säugers) pro Tag und besonders typisch im Bereich von 1 bis 10 mg/kg Körpergewicht pro Tag. Somit läge für einen 70 kg schweren erwachsenen Säuger die tatsächliche Menge pro Tag für gewöhnlich zwischen 70 und 700 mg, wobei diese Menge als Einzeldosis pro Tag oder üblicher in einer Reihe von Teildosen (wie z.B. zwei, drei, vier, fünf oder sechs) pro Tag gegeben werden kann, so daß die Gesamttagesdosis die gleiche ist. Eine wirksame Menge eines Salzes oder Solvats oder eines physiologisch funktionellen Derivats davon kann als Anteil der wirksamen Menge der erfindungsgemäßen Verbindung per se bestimmt werden. Es läßt sich annehmen, daß ähnliche Dosierungen für die Behandlung der anderen, obenerwähnten Krankheitszustände geeignet sind.

Gegenstand der Erfindung sind ferner Arzneimittel enthaltend mindestens eine Verbindung der Formel I A4 und/oder ihre Solvate und Stereoisomere, einschließlich deren Mischungen in allen Verhältnissen, und mindestens einen weiteren Arzneimittelwirkstoff.

Bevorzugt aber nicht ausschliesslich werden die Arzneimittel der Tabelle 1 mit den Verbindungen der Formel I A4 kombiniert. Eine Kombination der Formel I A4 und Arzneimitteln der Tabelle 1 kann auch mit Verbindungen der Formel V kombiniert werden.

| Tabelle 1. | | |
|---|---|---|
| Alkylierungsmittel | Cyclophosphamid | Lomustin |
| | Busulfan | Procarbazin |
| | lfosfamid | Altretamin |
| | Melphalan | Estramustinphosphat |
| | Hexamethylmelamin | Mechlorethamin |
| | Thiotepa | Streptozocin |
| | Chlorambucil | Temozolomid |
| | Dacarbazin | Semustin |
| | Carmustin | |
| | | |
| Platinmittel | Cisplatin | Carboplatin |
| | Oxaliplatin | ZD-0473 (AnorMED) |
| | Spiroplatin | Lobaplatin (Aetema) |
| | Carboxyphthalatoplatinum | Satraplatin (Johnson |
| | Tetraplatin | Matthey) |
| | Ormiplatin | BBR-3464 (Hoffrnann-La |
| | lproplatin | Roche) |
| | | SM-11355 (Sumitomo) |
| | | AP-5280 (Access) |
| | | |
| Antimetabolite | Azacytidin | Tomudex |
| | Gemcitabin | Trimetrexate |
| | Capecitabin | Deoxycoformycin |
| | 5-Fluoruracil | Fludarabin |
| | Floxuridin | Pentostatin |
| | 2-Chlordesoxyadenosin | Raltitrexed |
| | 6-Mercaptopurin | Hydroxyharnstoff |
| | 6-Thioguanin | Decitabin (SuperGen) |
| | Cytarabin | Clofarabin (Bioenvision) |
| | 2-Fluordesoxycytidin | Irofulven (MGI Pharma) |
| | Methotrexat | DMDC (Hoffmann-La |
| | Idatrexate | Roche) |
| | | Ethinylcytidin (Taiho) |
| | | |
| Topoisomerase- | Amsacrin | Rubitecan (SuperGen) |
| Inhibitoren | Epirubicin | Exatecanmesylat (Daiichi) |
| | Etoposid | Quinamed (ChemGenex) |
| | Teniposid oder | Gimatecan (Sigma- Tau) |
| | Mitoxantron | Diflomotecan (Beaufour- |
| | Irinotecan (CPT-11) | Ipsen) |
| | 7-Ethyl-10- | TAS-103 (Taiho) |
| | hydroxycamptothecin | Elsamitrucin (Spectrum) |
| | Topotecan | J-107088 (Merck & Co) |
| | Dexrazoxanet | BNP-1350 (BioNumerik) |
| | (TopoTarget) | CKD-602 (Chong Kun |
| | Pixantron (Novuspharma) | Dang) |
| | Rebeccamycin-Analogon | KW-2170 (Kyowa Hakko) |
| | (Exelixis) | |
| | BBR-3576 (Novuspharrna) | |
| | | |
| Antitumor- | Dactinomycin (Actinomycin | Amonafid |
| Antibiotika | D) | Azonafid |
| | Doxorubicin (Adriamycin) | Anthrapyrazol |
| | Deoxyrubicin | Oxantrazol |
| | Valrubicin | Losoxantron |
| | Daunorubicin | Bleomycinsulfat |
| | (Daunomycin) | (Blenoxan) |
| | Epirubicin | Bleomycinsäure |
| | Therarubicin | Bleomycin A |
| | Idarubicin | Bleomycin B |
| | Rubidazon | Mitomycin C |
| | Plicamycinp | MEN-10755 (Menarini) |
| | Porfiromycin | GPX-100 (Gem |
| | Cyanomorpholinodoxorubi | Pharmaceuticals) |
| | cin | |
| | Mitoxantron (Novantron) | |
| | | |
| Antimitotische | Paclitaxel | SB 408075 |
| Mittel | Docetaxel | (GlaxoSmithKline) |
| | Colchicin | E7010 (Abbott) |
| | Vinblastin | PG-TXL (Cell |
| | Vincristin | Therapeutics) |
| | Vinorelbin | IDN 5109 (Bayer) |
| | Vindesin | A 105972 (Abbott) |
| | Dolastatin 10 (NCI) | A 204197 (Abbott) |
| | Rhizoxin (Fujisawa) | LU 223651 (BASF) |
| | Mivobulin (Warner- | D24851 (ASTA Medica) |
| | Lambert) | ER-86526 (Eisai) |
| | Cemadotin (BASF) | Combretastatin A4 (BMS) |
| | RPR 109881A (Aventis) | Isohomohalichondrin-B |
| | TXD 258 (Aventis) | (PharmaMar) |
| | Epothilon B (Novartis) | ZD 6126 (AstraZeneca) |
| | T 900607 (Tularik) | PEG-Paclitaxel (Enzon) |
| | T 138067 (Tularik) | AZ10992 (Asahi) |
| | Cryptophycin 52 (Eli Lilly) | !DN-5109 (Indena) |
| | Vinflunin (Fabre) | AVLB (Prescient |
| | Auristatin PE (Teikoku | NeuroPharma) |
| | Hormone) | Azaepothilon B (BMS) |
| | BMS 247550 (BMS) | BNP- 7787 (BioNumerik) |
| | BMS 184476 (BMS) | CA-4-Prodrug (OXiGENE) |
| | BMS 188797 (BMS) | Dolastatin-10 (NrH) |
| | Taxoprexin (Protarga) | CA-4 (OXiGENE) |
| | | |
| Aromatase- | Aminoglutethimid | Exemestan |
| Inhibitoren | Letrozol | Atamestan (BioMedicines) |
| | Anastrazol | YM-511 (Yamanouchi) |
| | Formestan | |
| | | |
| Thymidylatsyntha | Pemetrexed (Eli Lilly) | Nolatrexed (Eximias) |
| se-Inhibitoren | ZD-9331 (BTG) | CoFactor™ (BioKeys) |
| | | |
| DNA- | Trabectedin (PharmaMar) | Mafosfamid (Baxter |
| Antagonisten | Glufosfamid (Baxter | International) |
| | International) | Apaziquon (Spectrum |
| | Albumin + 32P (Isotope | Pharmaceuticals) |
| | Solutions) | O6-Benzylguanin |
| | Thymectacin (NewBiotics) | (Paligent) |
| | Edotreotid (Novartis) | |
| | | |
| Farnesyltransfera | Arglabin (NuOncology | Tipifarnib (Johnson & |
| se-Inhibitoren | Labs) | Johnson) |
| | lonafarnib (Schering-Plough) | Perillylalkohol (DOR BioPharma) |
| | BAY-43-9006 (Bayer) | |
| | | |
| Pumpen- | CBT-1 (CBA Pharma) | Zosuquidar-Trihydrochlorid |
| Inhibitoren | Tariquidar (Xenova) | (Eli Lilly) |
| | MS-209 (Schering AG) | Biricodar-Dicitrat (Vertex) |
| Histonacetyltransf | Tacedinalin (Pfizer) | Pivaloyloxymethylbutyrat |
| erase- | SAHA (Aton Pharma) | (Titan) |
| Inhibitoren | MS-275 (Schering AG) | Depsipeptid (Fujisawa) |
| | | |
| Metalloproteinase- | Neovastat (Aeterna | CMT -3 (CollaGenex) |
| Inhibitoren | Laboratories) | BMS-275291 (Celltech) |
| Ribonucleosidred | Marimastat (British | Tezacitabin (Aventis) |
| uktase- | Biotech) | Didox (Molecules for |
| Inhibitoren | Galliummaltolat (Titan) | Health) |
| | Triapin (Vion) | |
| | | |
| TNF-alpha- | Virulizin (Lorus | Revimid (Celgene) |
| Agonisten/Antago | Therapeutics) | |
| nisten | CDC-394 (Celgene) | |
| | | |
| Endothelin-A- | Atrasentan (Abbot) | YM-598 (Yamanouchi) |
| Rezeptor- | ZD-4054 (AstraZeneca) | |
| Antagonisten | | |
| | | |
| Retinsäurerezepto | Fenretinid (Johnson & | Alitretinoin (Ligand) |
| r-Agonisten | Johnson) | |
| | LGD-1550 (Ligand) | |
| | | |
| lmmunmodulatoren | Interferon | Dexosom-Therapie |
| | Oncophage (Antigenics) | (Anosys) |
| | GMK (Progenics) | Pentrix (Australian Cancer |
| | Adenokarzinom-Impfstoff | Technology) |
| | (Biomira) | JSF-154 (Tragen) |
| | CTP-37 (AVI BioPharma) | Krebsimpfstoff (Intercell) |
| | JRX-2 (Immuno-Rx) | Norelin (Biostar) |
| | PEP-005 (Peplin Biotech) | BLP-25 (Biomira) |
| | Synchrovax-Impfstoffe | MGV (Progenics) |
| | (CTL Immuno) | !3-Alethin (Dovetail) |
| | Melanom-Impfstoff (CTL | CLL-Thera (Vasogen) |
| | Immuno) | |
| | p21-RAS-Impfstoff | |
| | (GemVax) | |
| | | |
| Hormonelle und | Östrogene | Prednison |
| antihormonelle | konjugierte Östrogene | Methylprednisolon |
| Mittel | Ethinylöstradiol | Prednisolon |
| | Chlortrianisen | Aminoglutethimid |
| | Idenestrol | Leuprolid |
| | Hydroxyprogesteroncaproa | Goserelin |
| | t | Leuporelin |
| | Medroxyprogesteron | Bicalutamid |
| | Testosteron | Flutamid |
| | Testosteronpropionat | Octreotid |
| | Fluoxymesteron | Nilutamid |
| | Methyltestosteron | Mitotan |
| | Diethylstilbestrol | P-04 (Novogen) |
| | Megestrol | 2-Methoxyöstradiol |
| | Tamoxifen | (EntreMed) |
| | Toremofin | Arzoxifen (Eli Lilly) |
| | Dexamethason | |
| | | |
| Photodynamische | Talaporfin (Light Sciences) | Pd-Bacteriopheophorbid |
| Mittel | Theralux | (Yeda) |
| | (Theratechnologies) | Lutetium-Texaphyrin |
| | Motexafin-Gadolinium | (Pharmacyclics) |
| | (Pharmacyclics) | Hypericin |
| Tyrosinkinase-Inhibitoren | Imatinib (Novartis) | Kahalid F (PharmaMar) |
| | Leflunomid | CEP- 701 (Cephalon) |
| | (Sugen/Pharmacia) | CEP-751 (Cephalon) |
| | ZDI839 (AstraZeneca) | MLN518 (Millenium) |
| | Erlotinib (Oncogene | PKC412 (Novartis) |
| | Science) | Phenoxodiol O |
| | Canertjnib (Pfizer) | Trastuzumab (Genentech) |
| | Squalamin (Genaera) | C225 (ImClone) |
| | SU5416 (Pharmacia) | rhu-Mab (Genentech) |
| | SU6668 (Pharmacia) | MDX-H210 (Medarex) |
| | ZD4190 (AstraZeneca) | 2C4 (Genentech) |
| | ZD6474 (AstraZeneca) | MDX-447 (Medarex) |
| | Vatalanib (Novartis) | ABX-EGF (Abgenix) |
| | PKI166 (Novartis) | IMC-1C11 (ImClone) |
| | GW2016 | |
| | (GlaxoSmithKline) | |
| | EKB-509 (Wyeth) | |
| | EKB-569 (Wyeth) | |
| Verschiedene | SR-27897 (CCK-A- | BCX-1777 (PNP-Inhibitor, |
| Mittel | Inhibitor, Sanofi- | BioCryst) |
| | Synthelabo) | Ranpirnase |
| | Tocladesin (cyclisches- | (Ribonuclease-Stimulans, |
| | AMP-Agonist, Ribapharm) | Alfacell) |
| | Alvocidib (CDK-Inhibitor, | Galarubicin (RNA- |
| | Aventis) | Synthese-Inhibitor, Dong- |
| | CV-247 (COX-2-Inhibitor, | A) |
| | Ivy Medical) | Tirapazamin |
| | P54 (COX-2-Inhibitor, | (Reduktionsmittel, SRI |
| | Phytopharm) | International) |
| | CapCell™ (CYP450- | N-Acetylcystein |
| | Stimulans, Bavarian | (Reduktionsmittel, |
| | Nordic) | Zambon) |
| | GCS-IOO (gal3-Antagonist, | R-Flurbiprofen (NF-kappaB-Inhibitor, Encore) |
| | GlycoGenesys) | 3CPA (NF-kappaB- |
| | G17DT-Immunogen | Inhibitor, Active Biotech) |
| | (Gastrin-Inhibitor, Aphton) | Seocalcitol (Vitamin-D- |
| | Efaproxiral (Oxygenator, | Rezeptor-Agonist, Leo) |
| | Allos Therapeutics) | 131-I-TM-601 (DNA- |
| | PI-88 (Heparanase- | Antagonist, |
| | Inhibitor, Progen) | TransMolecular) |
| | Tesmilifen (Histamin- | Eflornithin (ODC-Inhibitor, |
| | Antagonist, YM | ILEX Oncology) |
| | BioSciences) | Minodronsäure |
| | Histamin (Histamin-H2- | (Osteoclasten-Inhibitor, |
| | Rezeptor Agonist, Maxim) | Yamanouchi) |
| | Tiazofurin (IMPDH- | Indisulam (p53-Stimulans, |
| | Inhibitor, Ribapharm) | Eisai) |
| | Cilengitid (Integrin- | Aplidin (PPT-Inhibitor, |
| | Antagonist, Merck KGaA) | PharmaMar) |
| | SR-31747 (IL-1- | Rituximab (CD20- |
| | Antagonist, Sanofi- | Antikörper, Genentech) |
| | Synthelabo) | Gemtuzumab (CD33- |
| | CCI-779 (mTOR-Kinase- | Antikörper, Wyeth Ayerst) |
| | Inhibitor, Wyeth) | PG2 (Hämatopoese- |
| | Exisulind (PDE-V-Inhibitor, | Verstärker, |
| | Cell Pathways) | Pharmagenesis) |
| | CP-461 (PDE-V-Inhibitor, | immunol™ (Triclosan- |
| | Cell Pathways) | Oralspülung, Endo) |
| | AG-2037 (GART-Inhibitor, | Triacetyluridin (Uridin- |
| | Pfizer) | Prodrug, Wellstat) |
| | WX-UK1 | SN-4071 (Sarkom-Mittel, |
| | (Plasminogenaktivator- | Signature BioScience) |
| | Inhibitor, Wilex) | TransMID-107™ |
| | PBI-1402 (PMN-Stimulans, | (Immunotoxin, KS |
| | ProMetic LifeSciences) | Biomedix) |
| | Bortezomib (Proteasom- | PCK-3145 (Apoptose- |
| | Inhibitor, Millennium) | Förderer, Procyon) |
| | SRL-172 (T-Zell- | Doranidazol (Apoptose- |
| | Stimulans, SR Pharma) | Förderer, Pola) |
| | TLK-286 (Glutathion-S- | CHS-828 (cytotoxisches |
| | Transferase-Inhibitor, | Mittel, Leo) |
| | Telik) | trans-Retinsäure |
| | PT-100 (Wachstumsfaktor- | (Differentiator, NIH) |
| | Agonist, Point | MX6 (Apoptose-Förderer, |
| | Therapeutics) | MAXIA) |
| | Midostaurin (PKC-Inhibitor, | Apomin (Apoptose- |
| | Novartis) | Förderer, ILEX Oncology) |
| | Bryostatin-1 (PKC-Stimulans, GPC Biotech) | Urocidin (Apoptose-Förderer, Bioniche) |
| | CDA-II (Apoptose- | Ro-31-7453 (Apoptose- |
| | Förderer, Everlife) | Förderer, La Roche) |
| | SDX-101 (Apoptose- | Brostallicin (Apoptose- |
| | Förderer, Salmedix) | Förderer, Pharmacia) |
| | Ceflatonin (Apoptose- | |
| | Förderer, ChemGenex) | |
| | | |
| Alkylierungsmittel | Cyclophosphamid | Lomustin |
| | Busulfan | Procarbazin |
| | lfosfamid | Altretamin |
| | Melphalan | Estramustinphosphat |
| | Hexamethylmelamin | Mechlorethamin |
| | Thiotepa | Streptozocin |
| | Chlorambucil | Temozolomid |
| | Dacarbazin | Semustin |
| | Carmustin | |
| | | |
| Platinmittel | Cisplatin | Carboplatin |
| | Oxaliplatin | ZD-0473 (AnorMED) |
| | Spiroplatin | Lobaplatin (Aetema) |
| | Carboxyphthalatoplatinum | Satraplatin (Johnson |
| | Tetraplatin | Matthey) |
| | Ormiplatin | BBR-3464 (Hoffrnann-La |
| | lproplatin | Roche) |
| | | SM-11355 (Sumitomo) |
| | | AP-5280 (Access) |
| | | |
| Antimetabolite | Azacytidin | Tomudex |
| | Gemcitabin | Trimetrexate |
| | Capecitabin | Deoxycoformycin |
| | 5-Fluoruracil | Fludarabin |
| | Floxuridin | Pentostatin |
| | 2-Chlordesoxyadenosin | Raltitrexed |
| | 6-Mercaptopurin | Hydroxyharnstoff |
| | 6-Thioguanin | Decitabin (SuperGen) |
| | Cytarabin | Clofarabin (Bioenvision) |
| | 2-Fluordesoxycytidin | Irofulven (MGI Pharrna) |
| | Methotrexat | DMDC (Hoffmann-La |
| | Idatrexate | Roche) |
| | | Ethinylcytidin (Taiho) |
| | | |
| Topoisomerase- | Amsacrin | Rubitecan (SuperGen) |
| Inhibitoren | Epirubicin | Exatecanmesylat (Daiichi) |
| | Etoposid | Quinamed (ChemGenex) |
| | Teniposid oder | Gimatecan (Sigma- Tau) |
| | Mitoxantron | Diflomotecan (Beaufour- |
| | Irinotecan (CPT-11) | Ipsen) |
| | 7-Ethyl-10- | TAS-103 (Taiho) |
| | hydroxycamptothecin | Elsamitrucin (Spectrum) |
| | Topotecan | J-107088 (Merck & Co) |
| | Dexrazoxanet | BNP-1350 (BioNumerik) |
| | (TopoTarget) | CKD-602 (Chong Kun |
| | Pixantron (Novuspharrna) | Dang) |
| | Rebeccamycin-Analogon | KW-2170 (Kyowa Hakko) |
| | (Exelixis) | |
| | BBR-3576 (Novuspharma) | |
| | | |
| Antitumor- | Dactinomycin (Actinomycin | Amonafid |
| Antibiotika | D) | Azonafid |
| | Doxorubicin (Adriamycin) | Anthrapyrazol |
| | Deoxyrubicin | Oxantrazol |
| | Valrubicin | Losoxantron |
| | Daunorubicin | Bleomycinsulfat |
| | (Daunomycin) | (Blenoxan) |
| | Epirubicin | Bleomycinsäure |
| | Therarubicin | Bleomycin A |
| | Idarubicin | Bleomycin B |
| | Rubidazon | Mitomycin C |
| | Plicamycinp | MEN-10755 (Menarini) |
| | Porfiromycin | GPX-100 (Gem |
| | Cyanomorpholinodoxorubi | Pharmaceuticals) |
| | cin | |
| | Mitoxantron (Novantron) | |
| | | |
| Antimitotische | Paclitaxel | SB 408075 |
| Mittel | Docetaxel | (GlaxoSmithKline) |
| | Colchicin | E7010 (Abbott) |
| | Vinblastin | PG-TXL (Cell |
| | Vincristin | Therapeutics) |
| | Vinorelbin | IDN 5109 (Bayer) |
| | Vindesin | A 105972 (Abbott) |
| | Dolastatin 10 (NCl) | A 204197 (Abbott) |
| | Rhizoxin (Fujisawa) | LU 223651 (BASF) |
| | Mivobulin (Wamer- | D 24851 (ASTA Medica) |
| | Lambert) | ER-86526 (Eisai) |
| | Cemadotin (BASF) | Combretastatin A4 (BMS) |
| | RPR 109881A (Aventis) | Isohomohalichondrin-B |
| | TXD 258 (Aventis) | (PharmaMar) |
| | Epothilon B (Novartis) | ZD 6126 (AstraZeneca) |
| | T 900607 (Tularik) | PEG-Paclitaxel (Enzon) |
| | T 138067 (Tularik) | AZ10992 (Asahi) |
| | Cryptophycin 52 (Eli Lilly) | IDN-5109 (Indena) |
| | Vinflunin (Fabre) | AVLB (Prescient |
| | Auristatin PE (Teikoku | NeuroPharma) |
| | Hormone) | Azaepothilon B (BMS) |
| | BMS 247550 (BMS) | BNP- 7787 (BioNumerik) |
| | BMS 184476 (BMS) | CA-4-Prodrug (OXiGENE) |
| | BMS 188797 (BMS) | Dolastatin-10 (NrH) |
| | Taxoprexin (Protarga) | CA-4 (OXiGENE) |
| | | |
| Aromatase- | Aminoglutethimid | Exemestan |
| Inhibitoren | Letrozol | Atamestan (BioMedicines) |
| | Anastrazol | YM-511 (Yamanouchi) |
| | Formestan | |
| | | |
| Thymidylatsyntha | Pemetrexed (Eli Lilly) | Nolatrexed (Eximias) |
| se-Inhibitoren | ZD-9331 (BTG) | CoFactor™ (BioKeys) |
| DNA- | Trabectedin (PharmaMar) | Mafosfamid (Baxter |
| Antagonisten | Glufosfamid (Baxter | International) |
| | International) | Apaziquon (Spectrum |
| | Albumin + 32P (Isotope | Pharmaceuticals) |
| | Solutions) | O6-Benzylguanin |
| | Thymectacin (NewBiotics) | (Paligent) |
| | Edotreotid (Novartis) | |
| | | |
| Farnesyltransfera | Arglabin (NuOncology | Tipifarnib (Johnson & |
| se-Inhibitoren | Labs) | Johnson) |
| | lonafarnib (Schering- | Perillylalkohol (DOR |
| | Plough). | BioPharma) |
| | BAY-43-9006 (Bayer) | |
| | | |
| Pumpen- | CBT-1 (CBA Pharma) | Zosuquidar-Trihydrochlorid |
| Inhibitoren | Tariquidar (Xenova) | (Eli Lilly) |
| | MS-209 (Schering AG) | Biricodar-Dicitrat (Vertex) |
| Histonacetyltransf | Tacedinalin (Pfizer) | Pivaloyloxymethylbutyrat |
| erase- | SAHA (Aton Pharma) | (Titan) |
| Inhibitoren | MS-275 (Schering AG) | Depsipeptid (Fujisawa) |
| Metalloproteinase- | Neovastat (Aeterna | CMT -3 (CollaGenex) |
| Inhibitoren | Laboratories) | BMS-275291 (Celltech) |
| Ribonucleosidred | Marimastat (British | Tezacitabin (Aventis) |
| uktase- | Biotech) | Didox (Molecules for |
| Inhibitoren | Galliummaltolat (Titan) | Health) |
| | Triapin (Vion) | |
| | | |
| TNF-alpha- | Virulizin (Lorus | Revimid (Celgene) |
| Agonisten/Antago | Therapeutics) | |
| nisten | CDC-394 (Celgene) | |
| Endothelin-A- | Atrasentan (Abbot) | YM-598 (Yamanouchi) |
| Rezeptor- | ZD-4054 (AstraZeneca) | |
| Antagonisten | | |
| | | |
| Retinsäurerezepto | Fenretinid (Johnson & | Alitretinoin (Ligand) |
| r-Agonisten | Johnson) | |
| | LGD-1550 (Ligand) | |
| | | |
| Immunmodulatoren | Interferon | Dexosom-Therapie |
| | Oncophage (Antigenics) | (Anosys) |
| | GMK (Progenics) | Pentrix (Australian Cancer |
| | Adenokarzinom-Impfstoff | Technology) |
| | (Biomira) | JSF-154 (Tragen) |
| | CTP-37 (AVI BioPharma) | Krebsimpfstoff (Intercell) |
| | JRX-2 (Immuno-Rx) | Norelin (Biostar) |
| | PEP-005 (Peplin Biotech) | BLP-25 (Biomira) |
| | Synchrovax-Impfstoffe | MGV (Progenics) |
| | (CTL Immuno) | !3-Alethin (Dovetail) |
| | Melanom-Impfstoff (CTL | CLL-Thera (Vasogen) |
| | Immuno) | |
| | p21-RAS-Impfstoff | |
| | (GemVax) | |
| | | |
| Hormonelle und | Östrogene | Prednison |
| antihormonelle | konjugierte Östrogene | Methylprednisolon |
| Mittel | Ethinylöstradiol | Prednisolon |
| | Chlortrianisen | Aminoglutethimid |
| | Idenestrol | Leuprolid |
| | Hydroxyprogesteroncaproa | Goserelin |
| | t | Leuporelin |
| | Medroxyprogesteron | Bicalutamid |
| | Testosteron | Flutamid |
| | Testosteronpropionat | Octreotid |
| | Fluoxymesteron | Nilutamid |
| | Methyltestosteron | Mitotan |
| | Diethylstilbestrol | P-04 (Novogen) |
| | Megestrol | 2-Methoxyöstradiol |
| | Tamoxifen | (EntreMed) |
| | Toremofin | Arzoxifen (Eli Lilly) |
| | Dexamethason | |
| | | |
| Photodynamische | Talaporfin (Light Sciences) | Pd-Bacteriopheophorbid |
| Mittel | Theralux | (Yeda) |
| | (Theratechnologies) | Lutetium-Texaphyrin |
| | Motexafin-Gadolinium | (Pharmacyclics) |
| | (Pharmacyclics) | Hypericin |
| Tyrosinkinase- | Imatinib (Novartis) | Kahalid F (PharmaMar) |
| Inhibitoren | Leflunomid | CEP- 701 (Cephalon) |
| | (Sugen/Pharmacia) | CEP-751 (Cephalon) |
| | ZDI839 (AstraZeneca) | MLN518 (Millenium) |
| | Erlotinib (Oncogene | PKC412 (Novartis) |
| | Science) | Phenoxodiol O |
| | Canertjnib (Pfizer) | Trastuzumab (Genentech) |
| | Squalamin (Genaera) | C225 (ImClone) |
| | SU5416 (Pharmacia) | rhu-Mab (Genentech) |
| | SU6668 (Pharmacia) | MDX-H210 (Medarex) |
| | ZD4190 (AstraZeneca) | 2C4 (Genentech) |
| | ZD6474 (AstraZeneca) | MDX-447 (Medarex) |
| | Vatalanib (Novartis) | ABX-EGF (Abgenix) |
| | PKI166 (Novartis) | IMC-1C11 (ImClone) |
| | GW2016 | |
| | (GlaxoSmithKline) | |
| | EKB-509 (Wyeth) | |
| | EKB-569 (Wyeth) | |
| | | |
| Verschiedene | SR-27897 (CCK-A- | BCX-1777 (PNP-Inhibitor, |
| Mittel | Inhibitor, Sanofi- | BioCryst) |
| | Synthelabo) | Ranpirnase (Ribonuclease- |
| | Tocladesin (cyclisches- | Stimulans, Alfacell) |
| | AMP-Agonist, Ribapharm) | Galarubicin (RNA- |
| | Alvocidib (CDK-Inhibitor, | Synthese-Inhibitor, Dong-A) |
| | Aventis) | Tirapazamin |
| | CV-247 (COX-2-Inhibitor, | (Reduktionsmittel, SRI |
| | Ivy Medical) | International) |
| | P54 (COX-2-Inhibitor, | N-Acetylcystein |
| | Phytopharm) | (Reduktionsmittel, Zambon) |
| | CapCell™ (CYP450- | R-Flurbiprofen (NF- |
| | Stimulans, Bavarian | kappaB-Inhibitor, Encore) |
| | Nordic) | 3CPA (NF-kappaB- |
| | GCS-IOO (gal3- | Inhibitor, Active Biotech) |
| | Antagonist, | Seocalcitol (Vitamin-D- |
| | GlycoGenesys) | Rezeptor-Agonist, Leo) |
| | G17DT-Immunogen | 131-I-TM-601 (DNA- |
| | (Gastrin-Inhibitor, Aphton) | Antagonist, |
| | Efaproxiral (Oxygenator, | TransMolecular) |
| | Allos Therapeutics) | Eflornithin (ODC-Inhibitor, |
| | PI-88 (Heparanase- | ILEX Oncology) |
| | Inhibitor, Progen) | Minodronsäure |
| | Tesmilifen (Histamin- | (Osteoclasten-Inhibitor, |
| | Antagonist, YM | Yamanouchi) |
| | BioSciences) | Indisulam (p53-Stimulans, |
| | Histamin (Histamin-H2- | Eisai) |
| | Rezeptor- Agonist, | Aplidin (PPT-Inhibitor, |
| | Maxim) | PharmaMar) |
| | Tiazofurin (IMPDH- | Rituximab (CD20- |
| | Inhibitor, Ribapharm) | Antikörper, Genentech) |
| | Cilengitid (Integrin- | Gemtuzumab (CD33- |
| | Antagonist, Merck KGaA) | Antikörper, Wyeth Ayerst) |
| | SR-31747 (IL-1- | PG2 (Hämatopoese- |
| | Antagonist, Sanofi- | Verstärker, |
| | Synthelabo) | Pharmagenesis) |
| | CCI-779 (mTOR-Kinase- | Immunol™ (Triclosan- |
| | Inhibitor, Wyeth) | Oralspülung, Endo) |
| | Exisulind (PDE-V- | Triacetyluridin (Uridin- |
| | Inhibitor, Cell Pathways) | Prodrug, Wellstat) |
| | CP-461 (PDE-V-Inhibitor, | SN-4071 (Sarkom-Mittel, |
| | Cell Pathways) | Signature BioScience) |
| | AG-2037 (GART-Inhibitor, | TransMID-107™ |
| | Pfizer) | (Immunotoxin, KS |
| | WX-UK1 | Biomedix) |
| | (Plasminogenaktivator- | PCK-3145 (Apoptose- |
| | Inhibitor, Wilex) | Förderer, Procyon) |
| | PBI-1402 (PMN- | Doranidazol (Apoptose- |
| | Stimulans, ProMetic | Förderer, Pola) |
| | LifeSciences) | CHS-828 (cytotoxisches |
| | Bortezomib (Proteasom- | Mittel, Leo) |
| | Inhibitor, Millennium) | trans-Retinsäure |
| | SRL-172 (T-Zell- | (Differentiator, NIH) |
| | Stimulans, SR Pharma) | MX6 (Apoptose-Förderer, |
| | TLK-286 (Glutathion-S- | MAXIA) |
| | Transferase-Inhibitor, | Apomin (Apoptose- |
| | Telik) | Förderer, ILEX Oncology) |
| | PT-100 | Urocidin (Apoptose- |
| | (Wachstumsfaktor- | Förderer, Bioniche) |
| | Agonist, Point | Ro-31-7453 (Apoptose- |
| | Therapeutics) | Förderer, La Roche) |
| | Midostaurin (PKC- | Brostallicin (Apoptose- |
| | Inhibitor, Novartis) | Förderer, Pharmacia) |
| | Bryostatin-1 (PKC- | |
| | Stimulans, GPC Biotech) | |
| | CDA-II (Apoptose- | |
| | Förderer, Everlife) | |
| | SDX-101 (Apoptose- | |
| | Förderer, Salmedix) | |
| | Ceflatonin (Apoptose- | |
| | Förderer, ChemGenex) | |

Bevorzugt werden die Verbindungen der Formel I A4 mit den mit bekannten Antikrebsmitteln kombiniert:
Zu diesen bekannten Antikrebsmitteln zählen die folgenden: Ostrogenrezeptormodulatoren, Androgenrezeptormodulatoren, Retinoidrezeptormodulatoren, Zytotoxika, antiproliferative Mittel, Prenyl-Proteintransferasehemmer, HMG-CoA-Reduktase-Hemmer, HIV-Protease-Hemmer, Reverse-Transkriptase-Hemmer sowie weitere Angiogenese-hemmer. Die vorliegenden Verbindungen eignen sich insbesondere zur gemeinsamen Anwendung mit Radiotherapie. Die synergistischen Wirkungen der Hemmung des VEGF in Kombination mit Radiotherapie sind in der Fachwelt beschrieben worden (siehe WO 00/61186).
"Östrogenrezeptormodulatoren" bezieht sich auf Verbindungen, die die Bindung von Östrogen an den Rezeptor stören oder diese hemmen, und zwar unabhängig davon, wie dies geschieht. Zu den Östrogenrezeptormodulatoren zählen zum Beispiel Tamoxifen, Raloxifen, Idoxifen, LY353381, LY 117081, Toremifen, Fulvestrant, 4-[7-(2,2-Dimethyl-1-oxopropoxy-4-methyl-2-[4-[2-(1- piperidinyl)ethoxy]phenyl]-2H-1-benzopyran-3-yl]phenyl-2,2-dimethylpropanoat, 4,4'-Dihydroxybenzophenon-2,4-dinitrophenylhydrazon und SH646, was jedoch keine Einschränkung darstellen soll.
"Androgenrezeptormodulatoren" bezieht sich auf Verbindungen, die die Bindung von Androgenen an den Rezeptor stören oder diese hemmen, und zwar unabhängig davon, wie dies geschieht. Zu den Androgenrezeptormodulatoren zählen zum Beispiel Finasterid und andere 5α-Reduktase-Hemmer, Nilutamid, Flutamid, Bicalutamid, Liarozol und Abirateron-acetat.
"Retinoidrezeptormodulatoren" bezieht sich auf Verbindungen, die die Bindung von Retinoiden an den Rezeptor stören oder diese hemmen, und zwar unabhängig davon, wie dies geschieht. Zu solchen Retinoidrezeptormodulatoren zählen zum Beispiel Bexaroten, Tretinoin, 13-cis-Retinsäure, 9-cis-Retinsäure, α-Difluormethylomithin, ILX23-7553, trans-N-(4'-Hydroxyphenyl)retinamid und N-4-Carboxyphenylretinamid.
"Zytotoxika" bezieht sich auf Verbindungen, die in erster Linie durch direkte Einwirkung auf die Zellfunktion zum Zelltod führen oder die die Zellmyose hemmen oder diese stören, darunter Alkylierungsmittel, Tumornekrosefaktoren, interkaliernde Mittel, Mikrotubulin-Hemmer und Topoisomerase-Hemmer.
Zu den Zytotoxika zählen zum Beispiel Tirapazimin, Sertenef, Cachectin, Ifosfamid, Tasonermin, Lonidamin, Carboplatin, Altretamin, Prednimustin, Dibromdulcit, Ranimustin, Fotemustin, Nedaplatin, Oxaliplatin, Temozolomid, Heptaplatin, Estramustin, Improsulfan-tosylat, Trofosfamid, Nimustin, Dibrospidium-chlorid, Pumitepa, Lobaplatin, Satraplatin, Profiromycin, Cisplatin, lrofulven, Dexifosfamid, cis-Amindichlor(2-methylpyridin)platin, Benzylguanin, Glufosfamid, GPX100, (trans,trans,trans)-bis-mu-(hexan-1,6-diamin)-mu-[diamin-platin(ll)]bis-[diamin(chlor)platin(II)]-tetrachlorid, Diarizidinylspermin, Arsentrioxid, 1-(11-Dodecylamino-10-hydroxyundecyl)-3,7-dimethylxanthin, Zorubicin, Idarubicin, Daunorubicin, Bisantren, Mitoxantron, Pirarubicin, Pinafid, Valrubicin, Amrubicin, Antineoplaston, 3'-Desamino-3'-morpholino-13-desoxo-10-hydroxycarminomycin, Annamycin, Galarubicin, Elinafid, MEN10755 und 4-Desmethoxy-3-desamino-3-aziridinyl-4-methylsulfonyl-daunorubicin (siehe WO 00/50032), was jedoch keine Einschränkung darstellen soll.
Zu den Mikrotubulin-Hemmern zählen zum Beispiel Paclitaxel, Vindesinsulfat, 3',4'-Dideshydro-4'-desoxy-8'-norvincaleukoblastin, Docetaxol, Rhizoxin, Dolastatin, Mivobulin-isethionat, Auristatin, Cemadotin, RPR109881, BMS184476, Vinflunin, Cryptophycin, 2,3,4,5,6-pentafluor-N-(3-fluor-4-methoxyphenyl)benzolsulfonamid, Anhydrovinblastin, N,N-dimethyl-L-valyl-L-valyl-N-methyl-L-valyl-L-prolyl-L-prolin-t-butylamid, TDX258 und BMS188797.
Topoisomerase-Hemmer sind zum Beispiel Topotecan, Hycaptamin, Irinotecan, Rubitecan, 6-Ethoxypropionyl-3',4'-O-exo-benzyliden-chartreusin, 9-Methoxy-N,N-dimethyl-5-nitropyrazolo[3,4,5-kl]acridin-2-(6H)propanamin, 1-Amino-9-ethyl-5-fluor-2,3-dihydro-9-hydroxy-4-methyl-1H,12H-benzo[de]pyrano[3',4':b,7]indolizino[1,2b]chinolin-10,13(9H,15H)-dion, Lurtotecan, 7-[2-(N-Isopropylamino)ethyl]-(20S)camptothecin, BNP1350, BNPI1100, BN80915, BN80942, Etoposid-phosphat, Teniposid, Sobuzoxan, 2'-Dimethylamino-2'-desoxy-etoposid, GL331, N-[2-(Dimethylamino)ethyl]-9-hydroxy-5,6-dimethyl-6H-pyrido[4,3-b]carbazol-1-carboxamid, Asulacrin, (5a,5aB,8aa,9b)-9-[2-[N-[2-(Dimethylamino)ethyl]-N-methylamino]ethyl]-5-[4-hydroxy-3,5-dimethoxyphenyl]-5,5a,6,8,8a,9-hexohydrofuro(3',4':6,7)naphtho(2,3-d)-1,3-dioxol-6-on, 2,3-(Methylen-dioxy)-5-methyl-7-hydroxy-8-methoxybenzo[c]phenanthridinium, 6,9-Bis[(2-aminoethyl)amino]benzo[g]isochinolin-5,10-dion, 5-(3-Aminopropylamino)-7,10-dihydroxy-2-(2-hydroxyethylaminomethyl)-6H-pyrazolo[4,5,1-de]-acridin-6-on, N-[1-[2(Diethylamino)ethylamino]-7-methoxy-9-oxo-9H-thio-xanthen-4-ylmethyl]formamid, N-(2-(Dimethyl-amino)-ethyl)acridin-4-carboxamid, 6-[[2-(Dimethylamino)-ethyl]amino]-3-hydroxy-7H-indeno[2,1-c]chinolin-7-on und Dimesna.
Zu den "antiproliferativen Mitteln" zählen Antisense-RNA- und -DNA-Oligonucleotide wie G3139, ODN698, RVASKRAS, GEM231 und INX3001, sowie Antimetaboliten wie Enocitabin, Carmofur, Tegafur, Pentostatin, Doxifluridin, Trimetrexat, Fludarabin, Capecitabin, Galocitabin, Cytarabinocfosfat, Fosteabin-Natriumhydrat, Raltitrexed, Paltitrexid, Emitefur, Tiazofurin, Decitabin, Nolatrexed, Pemetrexed, Nelzarabin, 2'-Desoxy-2'-methylidencytidin, 2'-Fluormethylen-2'-desoxycytidin, N-[5-(2,3-Dihydrobenzofuryl)sulfonyl]-N'-(3,4-dichlorphenyl)harnstoff, N6-[4-Desoxy-4-[N2-[2(E),4(E)-tetradecadienoyl]glycylamino]-L-glycero-B-L-manno-hepto-pyranosyl]adenin, Aplidin, Ecteinascidin, Troxacitabine, 4-[2-Amino-4-oxo-4,6,7,8-tetrahydro-3H-pyrimidino[5,4-b][1,4]thiazin-6-yl-(S)-ethyl]-2,5-thienoyl-L-glutaminsäure, Aminopterin, 5-Flurouracil, Alanosin, 11-Acetyl-8-(carbamoyloxymethyl)-4-formyl-6-methoxy-14-oxa-1,11-diazatetracyclo-(7.4.1.0.0)-tetradeca-2,4,6-trien-9-ylessigsäureester, Swainsonin, Lometrexol, Dexrazoxan, Methioninase, 2'-cyan-2'-desoxy-N4-palmitoyl-1-B-D-Arabinofuranosylcytosin und 3-Aminopyridin-2-carboxaldehyd-thiosemicarbazon. Die "antiproliferativen Mittel" beinhalten auch andere monoklonale Antikörper gegen Wachstumsfaktoren als bereits unter den "Angiogenese-Hemmern" angeführt wurden, wie Trastuzumab, sowie Tumorsuppressorgene, wie p53, die über rekombinanten virusvermittelten Gentransfer abgegeben werden können (siehe z.B. US-Patent Nr. 6,069,134).
Insbesondere bevorzugt ist die Verwendung der erfindungsgemäßen Verbindung zur Behandlung und Prophylaxe von Tumorerkrankungen.

Der Tumor ist vorzugsweise ausgewählt aus der Gruppe der Tumoren des Plattenepithel, der Blasen, des Magens, der Nieren, von Kopf und Hals, des Ösophagus, des Gebärmutterhals, der Schilddrüse, des Darm, der Leber, des Gehirns, der Prostata, des Urogenitaltrakts, des lymphatischen Systems, des Magens, des Kehlkopft und/oder der Lunge.

Der Tumor ist weiterhin vorzugsweise ausgewählt aus der Gruppe Lungenadenokarzinom, kleinzellige Lungenkarzinome, Bauchspeicheldrüsenkrebs, Glioblastome, Kolonkarzinom und Brustkarzinom.

Weiterhin bevorzugt ist die Verwendung zur Behandlung eines Tumors des Blut- und Immunsystems, vorzugsweise zur Behandlung eines Tumors ausgewählt aus der Gruppe der akuten myelotischen Leukämie, der chronischen myelotischen Leukämie, akuten lymphatischen Leukämie und/oder chronischen lymphatischen Leukämie.

Die Erfindung umfasst auch ein Verfahren zur Behandlung eines Patienten, der ein Neoplasma, wie einen Krebs, hat, durch Verabreichung
a) einer oder mehrerer der Verbindungen der Formel IA4
b) und einer oder mehrerer der Verbindungen der Formel V oder deren Säure-Additionssalze, insbesondere Hydrochloride: 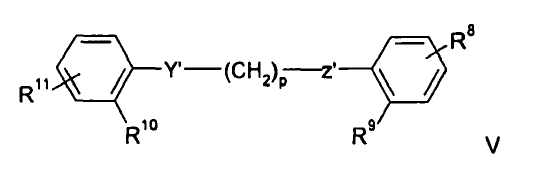 worin Y' und Z' jeweils unabhängig voneinander O oder N bedeuten, R⁶ und R⁷ jeweils unabhängig voneinander H, OH, Halogen, OC1-10-Alkyl, OCF₃, NO₂ oder NH₂ bedeuten, n eine ganze Zahl zwischen 2 und 6, jeweils einschließlich, bedeutet und R⁸ und R⁹ jeweils unabhängig voneinander vorzugsweise an der meta- oder para-Position stehen und aus der Gruppe: 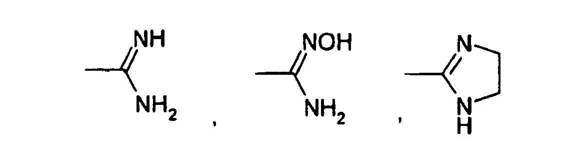 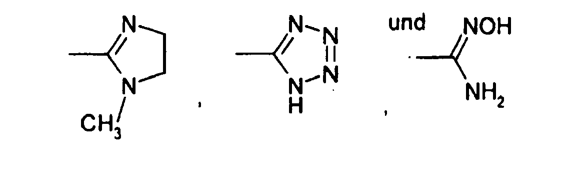 ausgewählt sind, wobei die erste und die zweite Verbindung gleichzeitig oder innerhalb von 14 Tagen voneinander in Mengen verabreicht werden, die ausreichen, um das Wachstum des Neoplasmas zu hemmen.

Die Kombination der Verbindungen der Formel IA4 mit den Verbindungen der Formels V und anderer Pentamedin-Analoga führt zu einer synergistischen Wirkung bei der Hemmung von Neoplasien. Kombinationen enthaltend die Verbindungen der Formel V sind z.B. in WO 02058684 erwähnt.

Der Wirkungsmechanismus von Pentamidin oder seiner Derivate ist derzeit nicht eindeutig geklärt: Pentamidin oder seine Derivate hat offenbar pleiotrope Wirkungen, da es zu einer Abnahme von DNA-, RNA- und Protein-Synthese führt. Vor kurzem wurde beschrieben, dass Pentamidin ein leistungsfähiger Hemmstoff von PRL1-, -2- und 3-Phosphatasen (Pathak et al., 2002) und Tyrosinphosphatasen ist, und ihre Überexpression geht mit neoplastischen bösartigen Tumoren beim Menschen einher. Andererseits wurde beschrieben, dass Pentamidin ein Arzneimittel ist, das an die kleine DNA-Furche bindet (Puckowska et al., 2004) und das seine Wirkung über die Störung der Genexpression und/oder DNA-Synthese ausüben kann.

Die beigefügten Experimente zeigen, dass:
- sowohl Pentamidin als auch die Verbindungen der Formel IA4 Zellen im G2/M-Zellzyklus aufhalten.
- die Kombination von Pentamidin und Verbindungen der Formel IA4 additive bis synergistische Wirkungen auf die Zellproliferation haben.

Andere geeignete Pentamidin-Analoga umfassen Stilbamidin (G-1) und Hydroxystilbamidin (G-2) und ihre Indolanaloga (z.B. G-3): 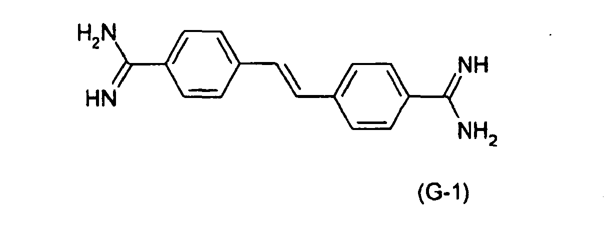 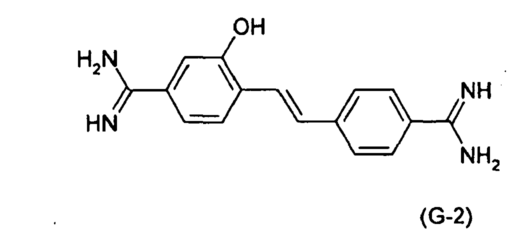 und

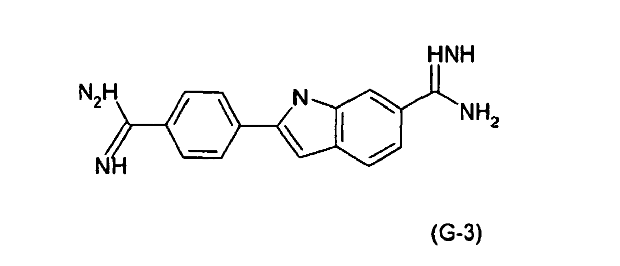

Jede Amidineinheit kann unabhängig voneinander durch eine der Einheiten ersetzt werden, die vorstehend für R⁸ und R¹¹ definiert sind. Wie im Fall der Benzimidazole und Pentamidine, eignen sich auch Salze von Stilbamidin, Hydroxystilbamidin und ihren Indolderivaten für das erfindungsgemäße Verfahren. Bevorzugte Salze umfassen zum Beispiel Dihydrochlorid- und Methansulfonatsalze.

Noch andere Analoga sind diejenigen, die unter eine Formel fallen, die in einem der U.S.-Patente Nr. 5,428,051, 5,521,189, 5,602,172, 5,643,935, 5,723,495, 5,843,980, 6,172,104 und 6,326,395 oder der U.S.-Patentanmeldung mit der Veröffentlichungsnr. US 2002/0019437 A1 bereitgestellt werden, die jeweils in ihrer Gesamtheit durch Bezugname aufgenommen sind. Beispielhafte Analoga umfassen 1,5-Bis-(4'-(N-hydroxyamidino)phenoxy)pentan, 1,3-Bis-(4'-(N-hydroxyamidino)-phenoxy)propan, 1,3-Bis-(2'-methoxy-4'-(N-hydroxyamidino)-phenoxy)-propan, 1,4-Bis-(4'-(N-hydroxyamidino)phenoxy)butan, 1,5-Bis-(4'-(N-hydroxyamidino)phenoxy)pentan, 1,4-Bis-(4'-(N-hydroxyamidino)-phenoxy)butan, 1,3-Bis-(4'-(4-hydroxyamidino)phenoxy)propan, 1,3-Bis-(2'-methoxy-4'-(N-hydroxyamidino)phenoxy)propan, 2,5-Bis-[4-amidinophenyl]furan, 2,5-Bis-[4-amidinophenyl]furan-bis-amidoxim, 2,5-Bis-[4-amidinophenyl]furan-bis-O-methylamidoxim, 2,5-Bis-[4-amidinophenyl]furan-bis-O-ethylamidoxim, 2,8-Diamidinodibenzothiophen, 2,8-Bis-(N-isopropylamidino)carbazol, 2,8-Bis-(N-hydroxyamidino)carbazol, 2,8-Bis-(2-imidazolinyl)dibenzothiophen, 2,8-Bis-(2-imidazolinyl)-5,5-dioxodibenzothiophen, 3,7-Diamidinodibenzothiophen, 3,7-Bis-(N-isopropylamidino)dibenzothiophen, 3,7-Bis-(N-hydroxyamidino)-dibenzothiophen, 3,7-Diaminodibenzothiophen, 3,7-Dibromdibenzothiophen, 3,7-Dicyanodibenzothiophen, 2,8-Diamidinodibenzofuran, 2,8-Di-(2-imidazolinyl)dibenzofuran, 2,8-Di-(N-isopropylamidino)dibenzofuran, 2,8-Di-(N-hydroxylamidino)dibenzofuran, 3,7-Di-(2-imidazolinyl)dibenzofuran, 3,7-Di-(isopropylamidino)dibenzofuran, 3,7-Di-(A-hydroxylamidino)dibenzofuran, 2,8-Dicyanodibenzofuran, 4,4'-Dibrom-2,2'-dinitrobiphenyl, 2-Methoxy-2'-nitro-4,4'-dibrombiphenyl, 2-Methoxy-2'-amino-4,4'-dibrombiphenyl, 3,7-Dibromdibenzofuran, 3,7-Dicyanodibenzofuran, 2,5-Bis-(5-amidino-2-benzimidazolyl)pyrrol, 2,5-Bis-[5-(2-imidazolinyl)-2-benzimidazolyl]pyrrol, 2,6-Bis-(5-(2-imidazolinyl)-2-benzimidazolyl]pyridin, 1-Methyl-2,5-bis-(5-amidino-2-benzimidazolyl)pyrrol, 1-Methyl-2,5-bis-[5-(2-imidazolyl)-2-benzimidazolyl]pyrrol, 1-Methyl-2,5-bis-[5-(1,4,5,6-tetrahydro-2-pyrimidinyl)-2-benzimidazolyl]pyrrol, 2,6-Bis-(5-amidino-2-benzimidazoyl)pyridin, 2,6-Bis-[5-(1,4,5,6-tetrahydro-2-pyrimidinyl)-2-benzimidazolyl]pyridin, 2,5-Bis-(5-amidino-2-benzirnidazolyl)furan, 2,5-Bis-[5-(2-imidazolinyl)-2-benzimidazolyl]furan, 2,5-Bis-(5-N-isopropylamidino-2-benzimidazolyl)furan, 2,5-Bis-(4-guanylphenyl)furan, 2,5-Bis(4-guanylphenyl)-3,4-dimethylfuran, 2,5-Di-p-[2-(3,4,5,6-tetrahydropyrimidyl)phenyl]furan, 2,5-Bis-[4-(2-imidazolinyl)phenyl]-furan, 2,5-[Bis-{4-(2-tetrahydropyrimidinyl)}phenyl]-p-(tolyloxy)furan, 2,5-[Bis-{4-(2-imidazolinyl)}-phenyl]-3-p-(tolyloxy)furan, 2,5-Bis-{4-[5-(N-2-aminoethylamido)benzimidazol-2-yl]phenyl}furan, 2,5-Bis-[4-(3a,4,5,6,7,7a-hexahydro-1 H-benzimidazol-2-yl)phenyl]furan, 2,5-Bis-[4-(4,5,6,7-tetrahydro-1H-1,3-diazepin-2-yl)phenyl]furan, 2,5-Bis-(4-N,N-dimethylcarboxhydrazidphenyl)furan, 2,5-Bis-{4-[2-(N-2-hydroxyethyl)imidazolinyl]phenyl}furan, 2,5-Bis-[4-(N-isopropyl-amidino)phenyl]furan, 2,5-Bis-{4-[3-(dimethylaminopropyl)amidino]phenyl}-furan, 2,5-Bis-{4-[N-(3-aminopropyl)amidino]phenyl}furan, 2,5-Bis-[2-(imidzaolinyl)phenyl]-3,4-bis-(methoxymethyl)furan, 2,5-Bis-[4-N-(dimethylaminoethyl)guanyl]-phenylfuran, 2,5-Bis-{4-[(N-2-hydroxyethyl)guanyl]-phenyl}furan, 2,5-Bis-[4-N-(cyclopropylguanyl)phenyl]furan, 2,5-Bis-[4-(N,N-diethylaminopropyl)-guanyl]phenylfuran, 2,5-Bis-(4-(2-(N-ethylimidazolinyl)]-phenyl}furan, 2,5-Bis-{4-[N-(3-pentylguanyl)])phenylfuran, 2,5-Bis-[4-(2-imidazolinyl)phenyl]-3-methoxyfuran, 2,5-Bis-[4-(N-isopropylamidino)phenyl]-3-methylfuran, Bis-[5-amidino-2-benzimidazolyl]methan, Bis-[5-(2-imidazolyl)-2-benzimidazolyl]methan, 1,2-Bis-[5-amidino-2-benzimidazolyl]ethan, 1,2-Bis-[5-(2-imidazolyl)-2-benzimidazolyl]ethan, 1,3-Bis-[5-amidino-2-benzimidazolyl]propan, 1,3-Bis-[5-(2-imidazolyl)-2-benzimidazolyl]propan, 1,4-Bis-[5-amidino-2-benzimidazolyl]propan, 1,4-Bis-[5-(2-imidazolyl)-2-benzimidazolyl]butan, 1,8-Bis-[5-amidino-2-benzimidazolyl]octan, trans-1,2-Bis-[5-amidino-2-benzimidazolyl]ethen, 1,4-Bis-[5-(2-imidazolyl)-2-benzimidazolyl]-1-buten, 1,4-Bis-[5-(2-imidazolyl)-2-benzimidazolyl]-2-buten, 1,4-Bis-[5-(2-imidazolyl)-2-benzimidazolyl]-1-methylbutan, 1,4-Bis-[5-(2-imidazolyl)-2-benzimidazolyl]-2-ethylbutan, 1,4-Bis-[5-(2-imidazolyl)-2-benzimidazolyl]-1-methyl-1-buten, 1,4-Bis-[5-(2-imidazolyl)-2-benzimidazolyl]-2,3-diethyl-2-buten, 1,4-Bis-[5-(2-imidazolyl)-2-benzimidazolyl]-1,3-butadien, 1,4-Bis-[5-(2-imidazolyl)-2-benzimidazolyl]-2-methyl-1,3-butadien, Bis-[5-(2-pyrimidyl)-2-benzimidazolyl]methan, 1,2-Bis-[5-(2-pyrimidyl)-2-benzimidazolyl]ethan, 1,3-Bis-[5-amidino-2-benzimidazolyl]propan, 1,3-Bis-[5-(2-pyrimidyl)-2-benzimidazolyl]propan, 1,4-Bis-[5-(2-pyrimidyl)-2-benzimidazolyl]butan, 1,4-Bis-[5-(2-pyrimidyl)-2-benzimidazolyl]-1-buten, 1,4-Bis-[5-(2-pyrimidyl)-2-benzimidazolyl]-2-buten, 1,4-Bis-[5-(2-pyrimidyl)-2-benzimidazolyl]-1-methylbutan, 1,4-Bis-[5-(2-pyrimidyl)-2-benzimidazolyl]-2-ethylbutan, 1,4-Bis-[5-(2-pyrimidyl)-2-benzimidazolyl]-1-methyl-1-buten, 1,4-Bis-[5-(2-pyrimidyl)-2-benzimidazolyl]-2,3-diethyl-2-buten, 1,4-Bis-[5-(2-pyrimidyl)-2-benzimidazotyl]-1,3-butadien und 1,4-Bis-[5-(2-pyrimidyl)-2-benzimidazolyl]-2-methyl-1,3-butadien, 2,4-Bis-(4-guanylphenyl)pyrimidin, 2,4-Bis-(4-imidazolin-2-yl)pyrimidin, 2,4-Bis-[(tetrahydropyrimidinyl-2-yl)phenyl]pyrimidin, 2-(4-[N-i-Propylguanyl]phenyl)-4-(2-methoxy-4-[N-i-propylguanyl]phenyl)pyrimidin, 4-(N-Cyclopentylamidino)-1,2-phenylendiamin, 2,5-Bis-[2-(5-amidino)benzimidazoyl]furan, 2,5-Bis-[2-{5-(2-imidazolino)}benzimidazoyl)furan, 2,5-Bis-[2-(5-N-isopropylamidino)-benzimidazoyl]furan, 2,5-Bis-[2-(5-N-cyclopentylamidino)-benzimidazoyl]furan, 2,5-Bis[2-(5-amidino)benzimidazoyl]pyrrol, 2,5-Bis-[2-{5-(2-imidazolino)}benzimidazoyl]pyrrol, 2,5-Bis-[2-(5-N-isopropylamidino)-benzimidazoyl]pyrrol, 2,5-Bis-[2-(5-N-cyclopentylamidino)-benzimidazoyl]pyrrol, 1-Methyl-2,5-bis-[2-(5-amidino)benzimidazoyl]pyrrol, 2,5-Bis-[2-{5-(2-imidazolino)}benzimidazoyl]-1-methylpyrrol, 2,5-Bis-[2-(5-N-cyclopentylamidino)benzimidazoyl]-1-methylpyrrol, 2,5-Bis-[2-(5-N-isopropylamidino)benzimidazoyl]thiophen, bis-[2-{5-(2-imidazolino)}benzimidazoyl]pyridin, 2,6-Bis-[2-(5-amidino)benzimidazoyl]-pyridin, 4,4'-Bis-[2-(5-N-isopropylamidino)benzimidazoyl]-1,2-diphenylethan, 4,4'-Bis-[2-(5-N-cyclopentylamidino)benzimidazoyl]-2,5-diphenylfuran, 2,5-Bis-[2-(5-amidino)benzimidazoyl]benzo-[b]-furan, 2,5-Bis-(2-(5-N-cyctopentylamidino)benzimidazoyl]benzo-[b]-furan, 2,7-Bis-[2-(5-N-isopropylamidino)benzimidazoyl]fluor, 2,5-Bis-[4-(3-(N-morpholinopropyl)-carbamoyl)phenyl]furan, 2,5-Bis-[4-(2-N,N-dimethylaminoethylcarbamoyl)phenyl]furan, 2,5-Bis-[4-(3-N,N-dimethylaminopropylcarbamoyl)phenyl]furan, 2,5-Bis-[4-(3-N-methyl-3-N-phenylaminopropylcarbamoyl)phenyl]furan, 2,5-Bis-[4-(3-N,N8,N11-trimethylaminopropylcarbamoyl)phenyl]furan, 2,5-Bis-[3-amidinophenyl]furan, 2,5-Bis-[3-(N-isopropylamidino)amidinophenylyuran, 2,5-Bis-[3-[(N-(2-dimethylaminoethyl)amidino]phenylfuran, 2,5-Bis-[4-(N-2,2,2-trichlorethoxycarbonyl)amidinophenyl]furan, 2,5-Bis-(4-(N-thioethylcarbonyl)amidinophenyl]furan, 2,5-Bis-[4-(N-benzyloxycarbonyl)amidinophenyl]furan, 2,5-Bis[4-(N-phenoxycarbonyl)-amidinophenyl]furan, 2,5-Bis-[4-(N-(4-fluor)-phenoxycarbonyl)amidinophenyl]furan, 2,5-Bis-[4-(N-(4-methoxy)phenoxycarbonyl)amidinophenyl]furan, 2,5-Bis-[4-(1-acetoxyethoxycarbonyl)amidinophenyl]furan und 2,5-Bis-[4-(N-(3-fluor)phenoxycarbonyl)amidinophenyl]furan. Verfahren zur Herstellung einer der vorstehenden Verbindungen sind in den U.S.-Patenten Nr. 5,428,051, 5,521,189, 5,602,172, 5,643,935, 5,723,495, 5,843,980, 6,172,104 und 6,326,395 oder der US-Patentanmeldung mit der Veröffentlichungsnr. US 200210019437 A1 beschrieben.

Pentamidin-Metabolite eignen sich ebenfalls in der erfindungsgemäßen antiprofiferativen Kombination. Pentamidin wird im Körper schnell zu mindestens sieben primären Metaboliten metabolisiert. Einige dieser Metabolite haben eine oder mehrere Wirkungen mit Pentamidin gemeinsam. Pentamidin-Metabolite weisen eine antiproliferative Wirkung auf, wenn sie mit einem Benzimidazol oder einem Analogon davon kombiniert werden.

Sieben Pentamidin-Analoga sind nachstehend gezeigt.

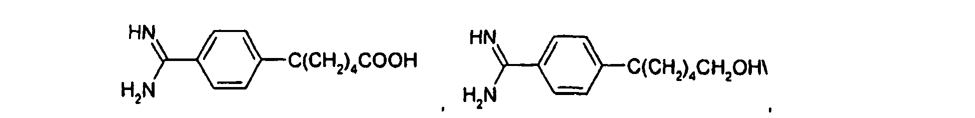

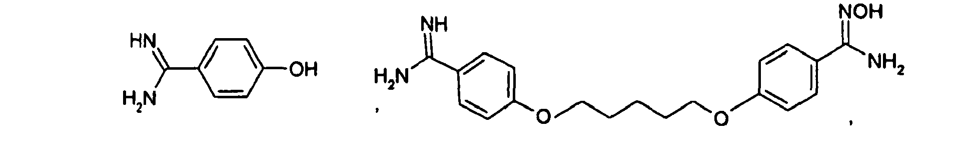

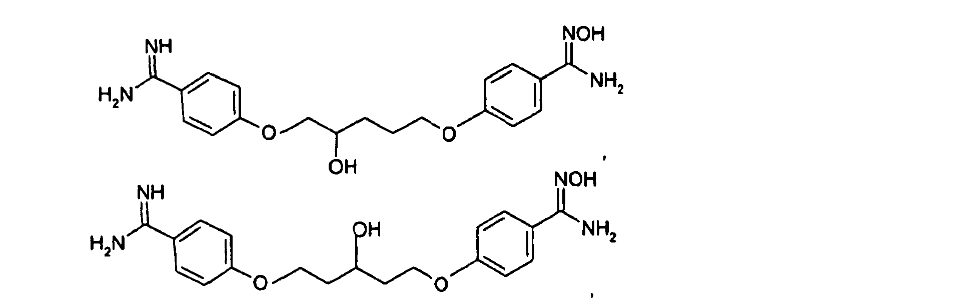

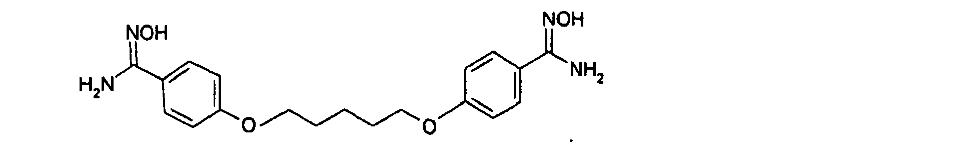

Die erfindungsgemäßen Kombinationen von Verbindungen der Formel IA4 und Formel V oder deren Analoga und seiner Metaboliten eignen sich zur Behandlung von Neoplasmen. Eine Kombinationstherapie kann allein oder in Verbindung mit einer anderen Therapie (z.B. Operation, Bestrahlung, Chemotherapie, biologische Therapie) durchgeführt werden. Zusätzlich kann eine Person, deren Risiko, ein Neoplasma zu entwickeln, größer ist, (z.B. jemand, der genetisch prädisponiert ist, oder jemand, der zuvor ein Neoplasma hatte) eine prophylaktische Behandlung erhalten, um die Neoplasmabildung zu hemmen oder zu verzögern.

Die Kombination der kinesin-ATPase Eg5/KSP mit den Verbindungen der Formel V, Pentamidin, seine Analoga und /der seiner Metaboliten ist ebenfalls Gegenstand der Erfindung.

Die Dosierung und Häufigkeit der Verabreichung jeder Verbindung der Kombination kann unabhängig gesteuert werden. Zum Beispiel kann eine Verbindung dreimal täglich oral verabreicht werden, während die zweite Verbindung einmal pro Tag intramuskulär verabreicht werden kann. Die Verbindungen können auch zusammen formuliert werden, so dass eine Verabreichung beiden Verbindungen zuführt.

Die erfindungsgemäßen antiproliferativen Kombinationen können auch als Komponenten eines pharmazeutischen Pakets bereitgestellt werden. Die zwei Arzneimittel können zusammen oder getrennt und in einzelnen Dosierungsmengen formuliert werden.

Unter einem anderen Aspekt umfasst die Erfindung ein Verfahren zur Behandlung eines Patienten, der ein Neoplasma, wie einen Krebs, hat, durch Verabreichung einer Verbindung der Formel (IA4) und (V) in Kombination mit einem antiproliferativen Mittel. Geeignete antiproliferative Mittel umfassen die in Tabelle 1 bereitgestellten.

Vor- und nachstehend sina alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetet oder Dichlormethan, trennt ab, trocknet die organische Phase über Natriumsulfat, dampft ein und reinigt duch Chromatographie an Kieselgel und/oder durch Kristallisation. Rf-Werte an Kieselgel; Laufmittel: Ethylacetat/Methanol 9:1.
Massenspektrometrie (MS): EI (Eletronenstoß-Ionisation) M⁺
FAB (Fast Atom Bombardment) (M+H)⁺
ESI (Electrospray Ionization) (M+H)⁺
APCI-MS (atmospheric pressure chemical ionization - mass spectrometry) (M+H)⁺

### Beispiel 1

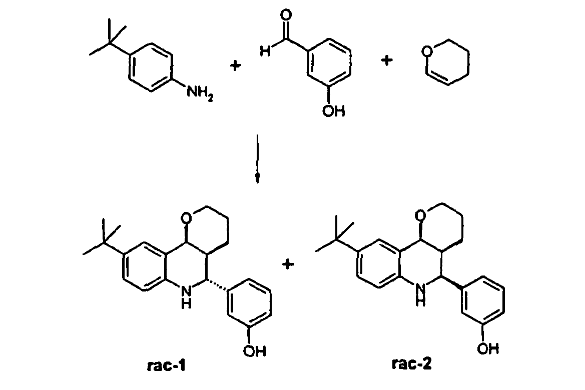

### a. Umsetzung in Gegenwart von Trifluoressigsäure (TFA)

Die Lösung des TFA-Salzes von 4-*tert*.-Butylanilin in Acetonitril (4-*tert*.-Butylanilin (0.50 g, 3.35 mmol) wurde in Acetonitril (4 mL) aufgenommen, auf 0°C abgekühlt und TFA (0.38 g, 3.35 mmol) langsam unter Rühren zugefügt) wurde schnell zu einer auf 0°C gekühltem Lösung aus 3-Hydroxybenzaldehyd (0.41 g, 3.35 mmol) und 3,4-Dihydro-2H-pyran (0.28 g, 3.35 mmol) in Acetonitril (2 mL) gegeben und weitere 60 min bei dieser Temperatur gerührt. Die Reaktionslösung wurde zur Trockne eingedampft und säulenchromatographisch aufgetrennt. Man erhielt einen farblosen Feststoff (620 mg, 1.84 mmol, 55 %), der sich als Isomerengemisch der jeweils racemischen *trans*/*cis*-Verbindung im Verhältnis 13:1 herausstellte.

Zur Auftrennung der *cis*/*trans*-Isomeren wurde der Feststoff in 0.1 N HCl in Isopropanol (20 mL) aufgenommen, jeweils 100 mL Diethylether und Cyclohexan zugegeben und über Nacht bei 4°C kristallisiert. Der farblose Feststoff wurde abfiltriert, mit wenig Diethylether gewaschen und getrocknet. 570 mg (1.52 mol) der farblosen Verbindung rac-1 Hydrochlorid konnten gewonnen werden. Die Mutterlauge wurde zur Trockne einrotiert und das *cis*-Isomer säulenchromatographisch aufgereinigt und ins Hydrochlorid überführt (70 mg, 0.19 mmol eines farblosen Feststoffes).

Kleinere Mengen an rac-1 und rac-2 wurden über chirale HPLC (2x Chiralpak AD 20µm, Eluent: Methanol) in die entsprechende Enantiomere aufgetrennt und erneut in die Hydrochloride überführt.

### b. Umsetzung in Gegenwart von Hexafluorisopropanol (HFIP)

Um höhere Ausbeuten des *cis*-Isomers zu erhalten, wurde anstelle von TFA Hexafluorisopropanol (HFIP) eingesetzt.

4-*tert.*-Butylanilin (0.50 g, 3:35 mmol), 3-Hydroxybenzaldehyd (0.41 g, 3.35 mmol) und 3,4-Dihydro-2H-pyran (0.28 g, 3.35 mmol) wurden in Acetonitril (1 mL) gelöst, bei RT HFIP (0.56 g, 3.35 mmol) zugetropft und weitere 18 h bei RT gerührt. Die Reaktionslösung wurde zur Trockne eingedampft und säulenchromatographisch aufgetrennt. Man erhielt einen farblosen Feststoff (485 mg, 1.44 mmol, 43 %), der sich als Isomerengemisch der jeweils racemischen *trans*/*cis*-Verbindung im Verhältnis 1:1.6 herausstellte. Eine Auftrennung der Isomere erfolgte wie unter a.) beschrieben.

### c. Umsetzung in Gegenwart von Bismut(III)chlorid

Zu einer Suspension von BiCl₃ (0.21 g, 0.67 mmol) und wasserfreiem Natriumsulfat (0.40 g) in Acetonitril (2 mL) wurden bei RT 3-Hydroxybenzaldehyd (0.41 g, 3.35 mmol) und 4-*tert*.-Butylanilin (0.50 g, 3.35 mmol) jeweils gelöst in Acetonitril (1 mL) zugegeben und 10 min bei RT gerührt. Dann wurde 3,4-Dihydro-2H-pyran (0.28 g, 3.35 mmol) bei RT zugetropft und weitere 30 min bei RT gerührt. Die Reaktionslösung wurde über Celite filtriert, das Filtrat zur Trockne eingedampft und säulenchromatographisch aufgereinigt. Man erhielt einen farblosen Feststoff (850 mg, 2.51 mmol, 75 %), der sich als Isomerengemisch der jeweils racemischen *trans*/*cis*-Verbindung im Verhältnis 1:1.1 herausstellte. Eine Auftrennung der Isomere erfolgte wie unter a.) beschrieben.

### d. Umsetzung in Gegenwart von Ytterbium(III)triflat

Zu einer Suspension von Yb(OTf)₃ (0.42 g, 0.67 mmol) und wasserfreiem Natriumsulfat (0.40 g) in Acetonitril (2 mL) wurden bei RT 3-Hydroxybenzaldehyd (0.41 g, 3.35 mmol) und 4-tert.-Butylanilin (0.50 g, 3.35 mmol) jeweils gelöst in Acetonitril (1 mL) zugegeben und 10 min bei RT gerührt. Dann wurde 3,4-Dihydro-2H-pyran (0.28 g, 3.35 mmol) bei RT zugetropft und weitere 30 min bei RT gerührt. Die Reaktionslösung wurde über Celite filtriert, das Filtrat zur Trockne eingedampft und säulenchromatographisch aufgereinigt. Man erhielt einen farblosen Feststoff (780 mg, 2.31 mmol, 69 %), der sich als Isomerengemisch der jeweils racemischen *trans*/*cis*-Verbindung im Verhältnis 1:1.3 herausstellte. Eine Auftrennung der Isomere erfolgte wie unter a.) beschrieben.

### e. Umsetzung in Gegenwart von Scandium(III)triflat

Zu einer Suspension von Sc(OTf)₃ (0.33 g, 0.67 mmol) und wasserfreiem Natriumsulfat (0.40 g) in Acetonitril (2 mL) wurden bei RT 3-Hydroxybenzaldehyd (0.41 g, 3.35 mmol) und 4-*tert*.-Butylanilin (0.50 g, 3.35 mmol) jeweils gelöst in Acetonitril (1 mL) zugegeben und 10 min bei RT gerührt. Dann wurde 3,4-Dihydro-2H-pyran (0.28 g, 3.35 mmol) bei RT zugetropft und weitere 30 min bei RT gerührt. Die Reaktionslösung wurde über Celite filtriert, das Filtrat zur Trockne eingedampft und säulenchromatographisch aufgereinigt. Man erhielt einen farblosen Feststoff (620 mg, 1.84 mmol, 55 %), der sich als Isomerengemisch der jeweils racemischen *trans*/*cis-*Verbindung herausstellte. Eine Auftrennung der Isomere erfolgte wie unter a.) beschrieben.

### f. Umsetzung in Gegenwart von Ammoniumcer(IV)nitrate (CAN)

Zu einer Lösung von 3-Hydroxybenzaldehyd (0.41 g, 3.35 mmol), 4-tert.-Butylanilin (0.50 g, 3.35 mmol) und 3,4-Dihydro-2H-pyran (0.28 g, 3.35 mmol) in Acetonitril (10 mL) wurde bei RT CAN (0.37 g, 0.67 mmol) zugefügt und weitere 30 min bei RT gerührt. Die Reaktionsmischung wurde zur Trockne eingedampft und säulenchromatographisch aufgereinigt. Man erhielt einen farblosen Feststoff (237 mg, 0.70 mmol, 21 %), der sich als Isomerengemisch der jeweils racemischen *trans*/*cis*-Verbindung im Verhältnis 5:1 herausstellte.

Eine Auftrennung der Isomere erfolgte wie unter a.) beschrieben.

### Beispiel 2

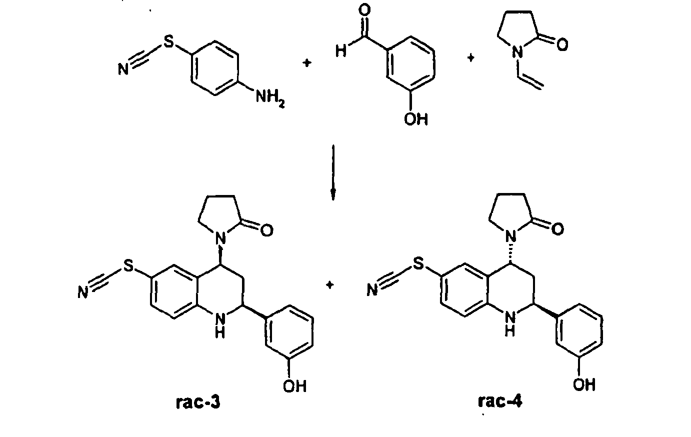

### a. Umsetzung in Gegenwart von Trifluoressigsäure (TFA)

Die Lösung des TFA-Salzes von 4-Thiocyanatoanilin in Acetonitril (Thiocyanatoanilin (0.46 g, 3.06 mmol) wurde in Acetonitril (4 mL) aufgenommen, auf 0°C abgekühlt und TFA (0.35 g, 3.06 mmol) langsam unter Rühren zugefügt) wurde schnell zu einer auf 0°C gekühltem Lösung aus 3-Hydroxybenzaldehyd (0.37 g, 3.06 mmol) und 1-Vinyl-2-pyrrolidinon (0.34 g, 3.06 mmol) in Acetonitril (2 mL) gegeben und weitere 60 min bei dieser Temperatur und 18 h bei RT gerührt. Die Reaktionslösung wurde eingeengt, mit wenig Ethylacetat aufgenommen und mit Diethylether kristallisiert. Man erhielt einen farblosen Feststoff (390 mg, 1.06 mmol, 35 %), der sich als *cis*-Isomer herausstellte.

Zur Auftrennung der *cis*/*trans*-Isomeren wurde das Filtrat zur Trockne eingeengt und säulenchromatographisch aufgereinigt. Man erhielt das *trans*-Isomer als farblosen Feststoff (160 mg, 0.44 mmol, 14 %),

Kleinere Mengen an rac-3 und rac-4 wurden über chirale HPLC (2x Chiralpak AD 20µm, Eluent: Methanol) in die entsprechende Enantiomere aufgetrennt und in die Hydrochloride überführt.

Analog werden unter Verwendung der entsprechenden Vorstufen die folgenden erfindungsgemäßen Verbindungen erhalten. Die angegebenen Retentionszeiten wurden dabei unter folgenden Bedingungen erhalten:

### Methode A:

Merck Hitachi LaChrom
Gradient 9 min
Flow rate : 1.5 mL/min von 80:20 zu 0:100 (innerhalb 6 min), 0:100 (1 min), zurück auf 80:20 (innerhalb 1 min), Wasser /Acetonitril Water + TFA (0. 1 % vol)
Acetonitril + TFA (0.1 % vol)
Column : LichroCART 125-4 RP- LiChrospher 60, RP-select-B (5 µm/60 mm)
Wavelength : 220 nm

### Methode B:

Merck Hitachi LaChrom
Gradient 9 min
Flow rate : 1.5 mL/min von 80:20 zu 0:100 (innerhalb 6 min), 0:100 (1 min), zurück auf 80:20 (innerhalb 0.8 min), Water /Acetonitril Water + TFA (0.1 % vol)
Acetonitril + TFA (0.1% vol)
Column : Lichrospher RP-select-B (5 µm/125 mm)
Wavelength : 220 nm

### Methode C:

Agilent 1100 Series
Gradient 3.5 min
Flow rate : 2 mL/min von 80:20 zu 0:100 (innerhalb 2.3 min), 0:100 (0.5 min),
zurück auf 80:20, Water /Acetonitril
Water + TFA (0. 1 % vol)
Acetonitril + TFA (0.1% vol)
Column : β-Test research sample, Chromolith Performance RP-18e
(3 µm/100 mm)

### Beispiele60-649

| | | **[M+1]⁺** | **Retentions-zeiten [min]** | **Methode** |
|---|---|---|---|---|
| (60) | 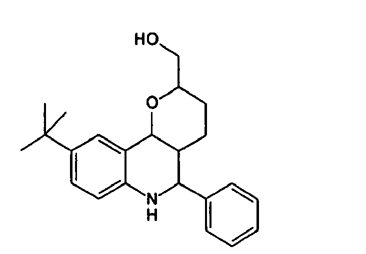 | 352 | 5.17/ 5.33 | A |
| (89) | 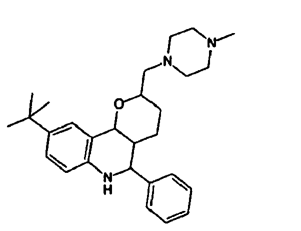 | 435 | 3,95 | A |
| (96) | 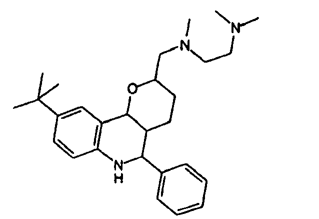 | 437 | 4.03/ 4.13 | A |
| (120) | 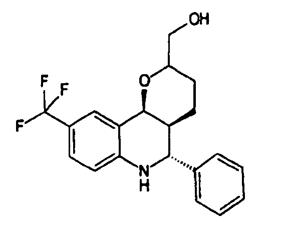 | 364 | 5.20/ 5.39 | A |
| (121) | 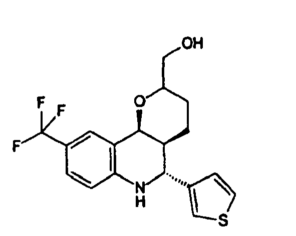 | 370 | 5,25 | A |
| (122) | 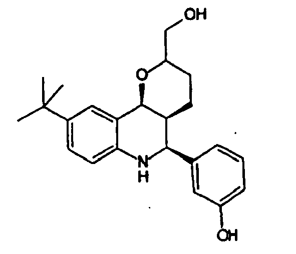 | 368 | 4,69 | A |
| (126) | 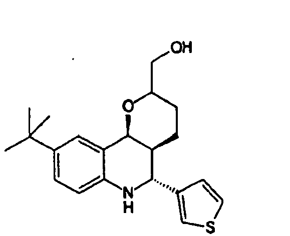 | 359 | 4,88 | A |
| (127) | 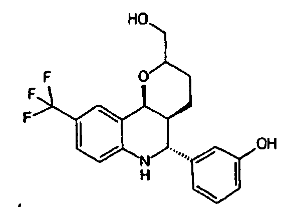 | 380 | 4,72 | A |
| (133) | 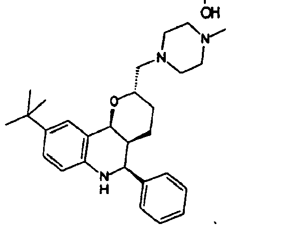 | 435 | 3,97 | A |
| (137) | 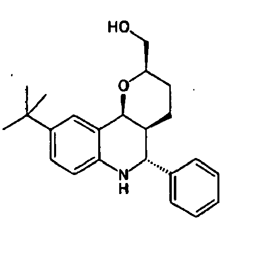 | 352 | 5,15 | A |
| (138) | 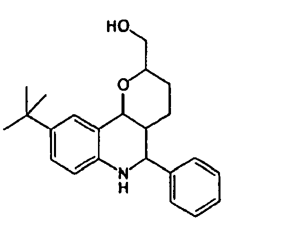 | 352 | 5.15/ 5.33 | A |
| (139) | 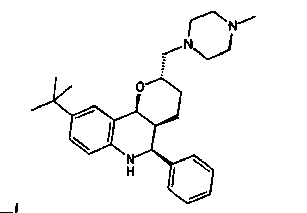 | 435 | 3,97 | A |
| (145) | 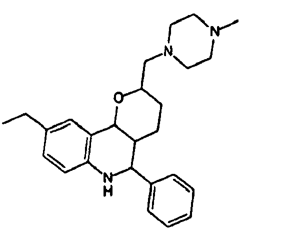 | 407 | 3,65 | A |
| (146) | 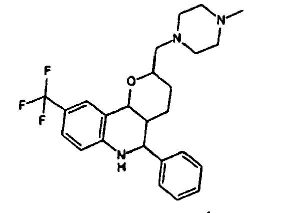 | 447 | 3.76/ 4.03 | A |
| (147) | 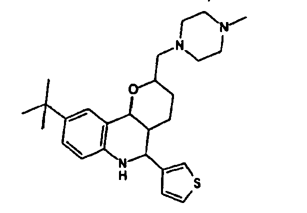 | 441 | 3.73/ 3.79 | A |
| (148) | 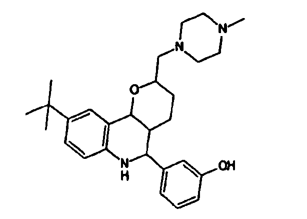 | 451 | 3,49 | A |
| (150) | 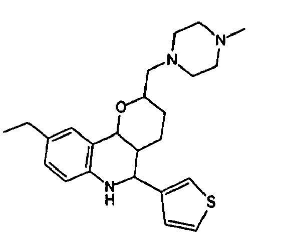 | 413 | 3,52 | A |
| (151) | 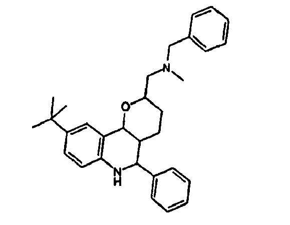 | 456 | 1.836/ 1.930 | C |
| (153) | 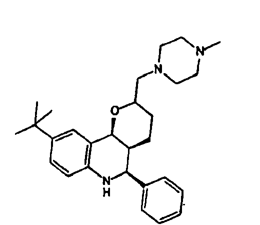 | 435 | 3,95 | A |
| (154) | 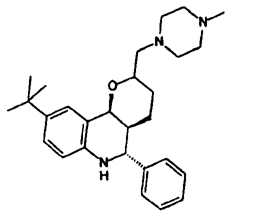 | 435 | 3,97 | A |
| (159) | 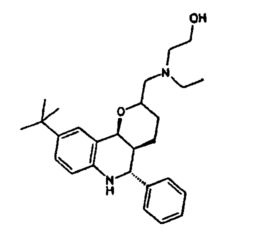 | 424 | 4,88 | A |
| (167) | 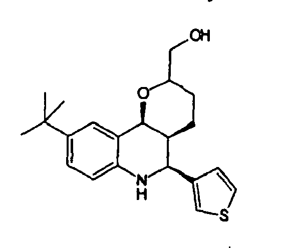 | 359 | 5,15 | A |
| (193) | 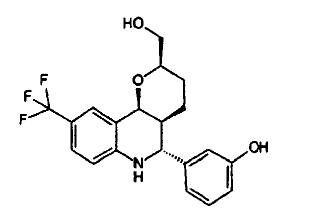 | 380 | 4,77 | A |
| (199) | 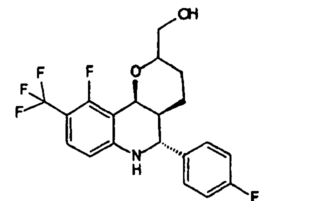 | 400 | 5,49 | A |
| (200) | 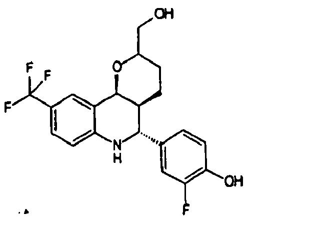 | 398 | 4.69/ 4.85 | A |
| (209) | 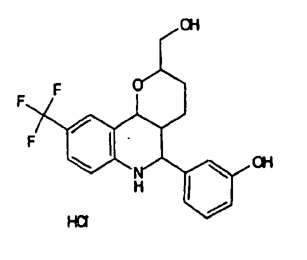 | 417 | 5,02 | B |
| (225) | 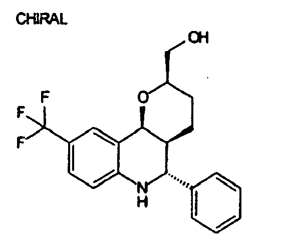 | 364 | 5,6 | A |
| (226) | 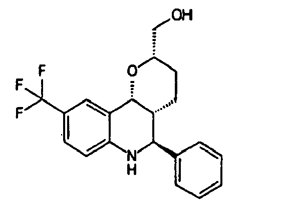 | 364 | 5,6 | A |
| (227) | 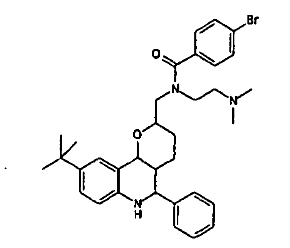 | 605/607 | 5,47 | A |
| (228) | 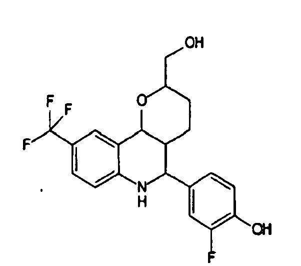 | 398 | 4,59 | A |
| (237) | 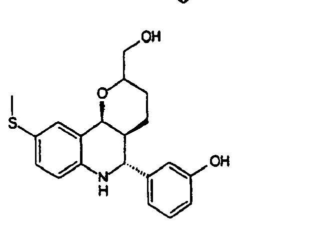 | 358 | 4,21 | A |
| (238) | 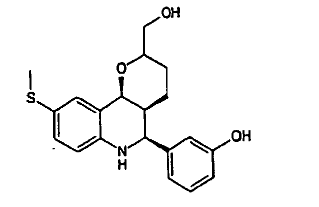 | 358 | 4,16 | A |
| (239) | 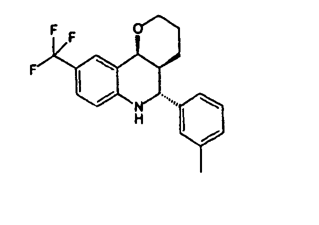 | 348 | 6,27 | A |
| (243) | 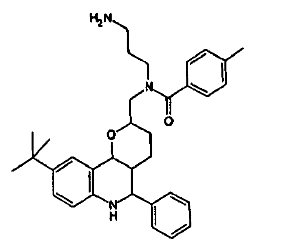 | 527 | 1.777/1.902 | C |
| (244) | 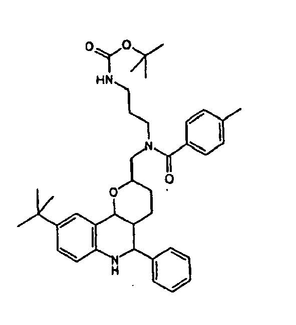 | 627 | 2.770/2871 | C |
| (248) | 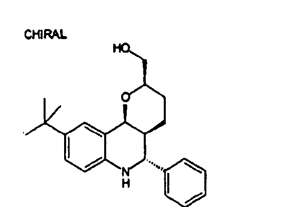 | 352 | 5,44 | A |
| (249) | 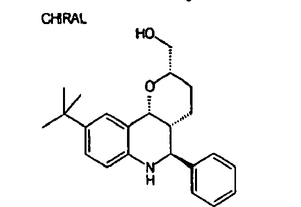 | 352 | 5,44 | A |
| (250) | 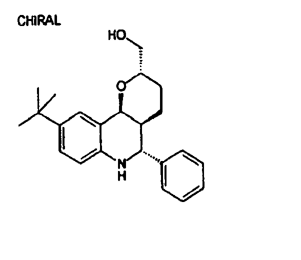 | 352 | 5,7 | A |
| (251) | 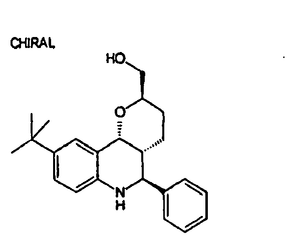 | 352 | 5,7 | A |
| (252) | 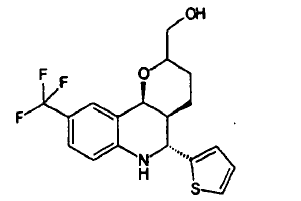 | 370 | 5,36 | A |
| (265) | 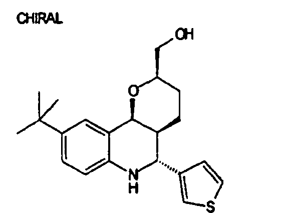 | 359 | 5,15 | B |
| (266) | 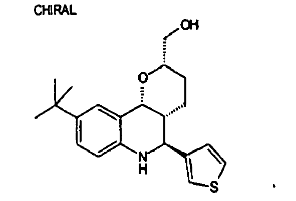 | 359 | 5,15 | B |
| (267) | 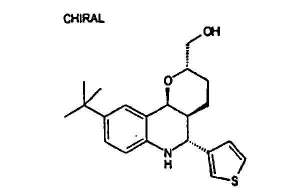 | 359 | 5,46 | B |
| (268) | 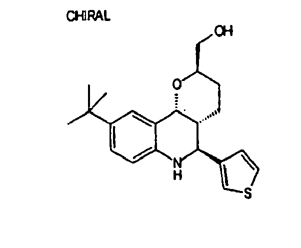 | 359 | 5,47 | B |
| (271) | 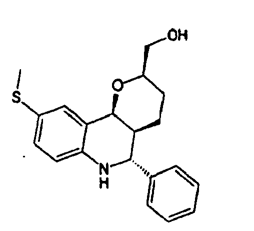 | 342 | 5,04 | A |
| (272) | 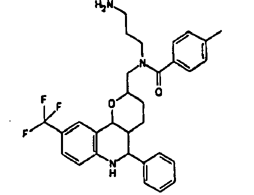 | 539 | 4.88/ 5.09 | A |
| (275) | 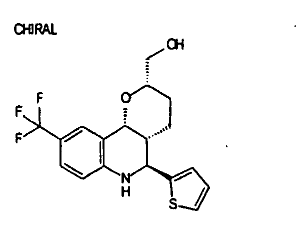 | 370 | 5,36 | A |
| (276) | 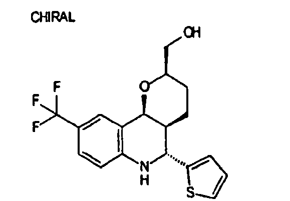 | 370 | 5,36 | A |
| (277) | 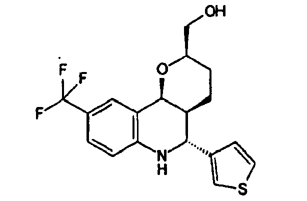 | 370 | 5,52 | A |
| (278) | 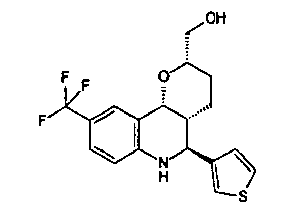 | 370 | 5,52 | A |
| (283) | 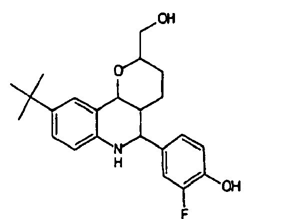 | 386 | 4.59/ 4.72 | A |
| (287) | 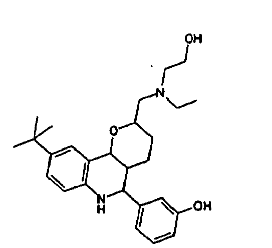 | 440 | 1,378 | A |
| (288) | 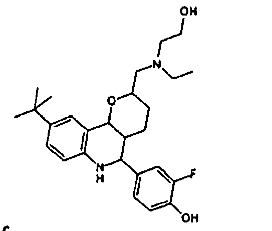 | 458 | 4,27 | A |
| (292) | 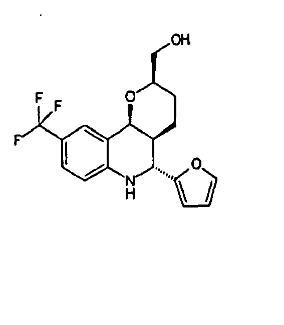 | 354 | 1,903 | C |
| (293) | 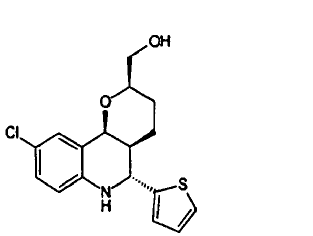 | 337 | 5,12 | A |
| (304) | 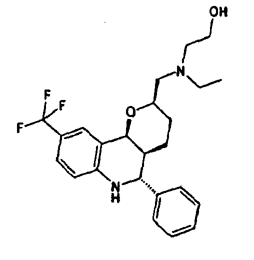 | 436 | 4,95 | B |
| (305) | 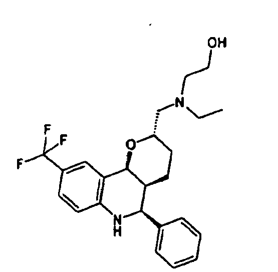 | 436 | 4,78 | B |
| (309) | 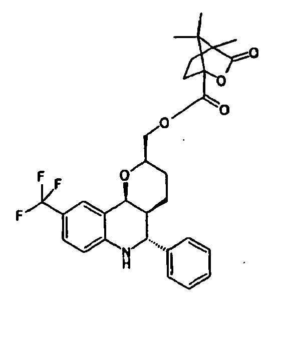 | 545 | 6,32 | A |
| (310) | 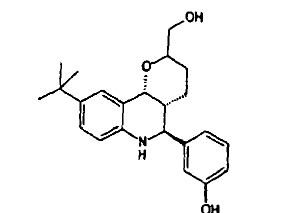 | 368 | 4,66 | B |
| (315) | 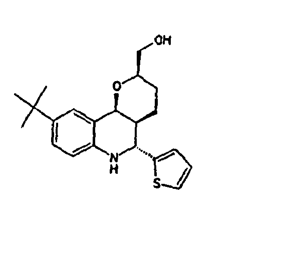 | 359 | 5,52 | A |
| (319) | 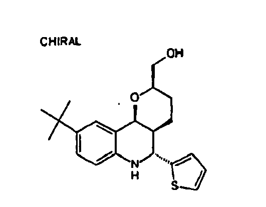 | 359 | 5.5 | A |
| (320) | 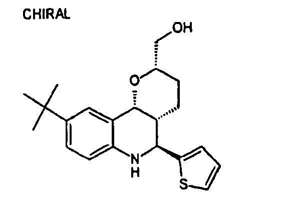 | 359 | 5.49 | A |
| (323) | 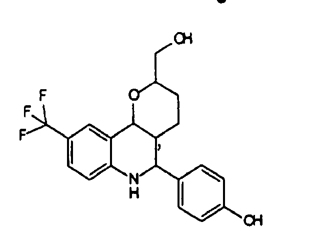 | 380 | 4.56/ 4.72 | A |
| (324) | 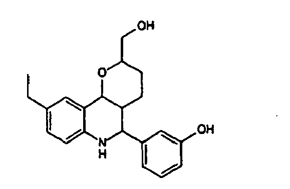 | 340 | 3.84/ 4.10 | A |
| (327) | 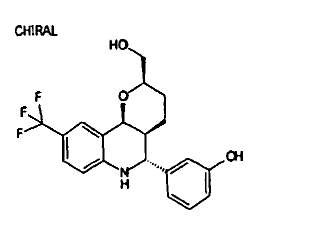 | 380 | 4.72 | A |
| (328) | 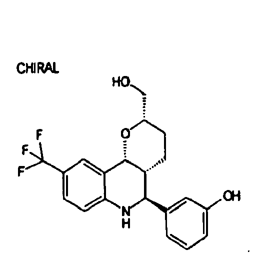 | 380 | 4.72 | A |
| (329) | 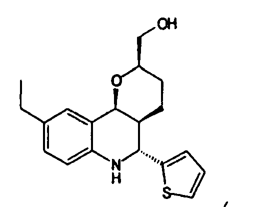 | 330 | 5,12 | A |
| (330) | 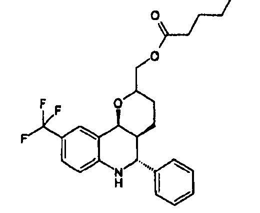 | 449 | 6.69 | A |
| (331) | 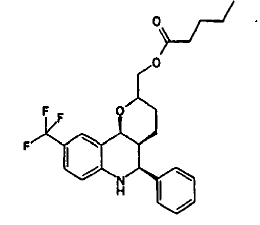 | 449 | 6.59 | A |
| (334) | 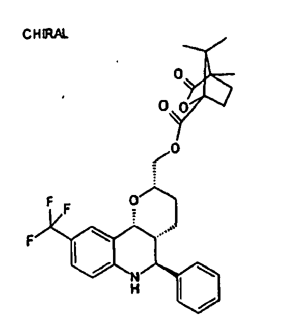 | 545 | 6.32 | A |
| (335) | 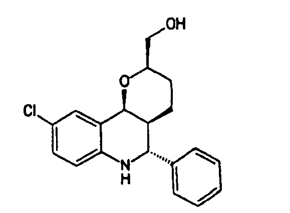 | 331 | 5,25 | A |
| (336) | 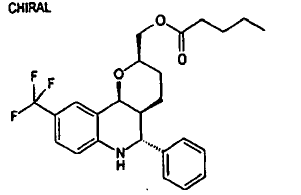 | 449 | 6.64 | A |
| (337) | 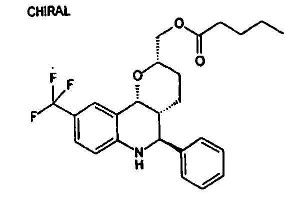 | 449 | 6.64 | A |
| (338) | 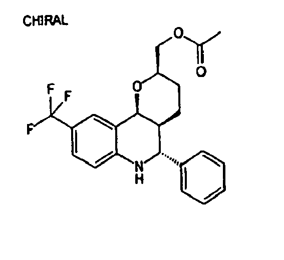 | 406 | 6.03 A | A |
| (339) | 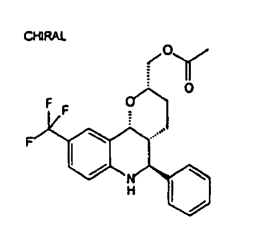 | 406 | 6.03 | A |
| (340) | 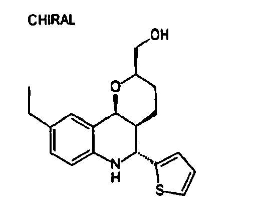 | 330 | 5.14 | A |
| (341) | 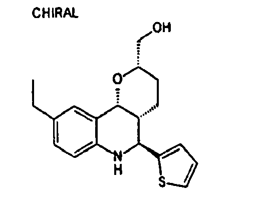 | 330 | 5.13 | A |
| (342) | 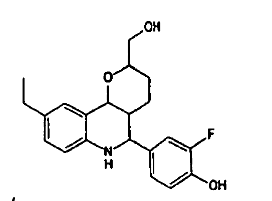 | 358 | 4.16/ 4.37 | A |
| (348) | 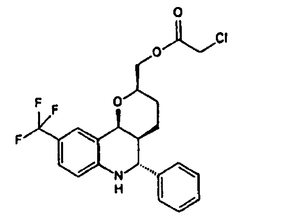 | 441 | 6,21 | A |
| (349) | 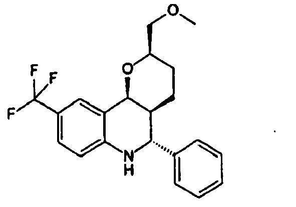 | 378 | 6,08 | A |
| (350) | 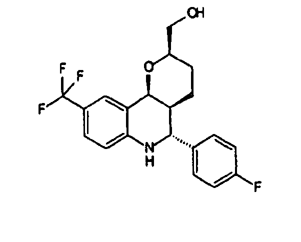 | 382 | 5,49 | A |
| (351) | 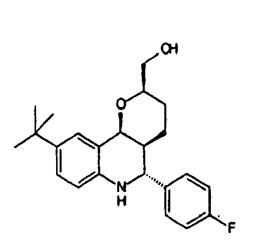 | 370 | 5,49 | A |
| (352) | 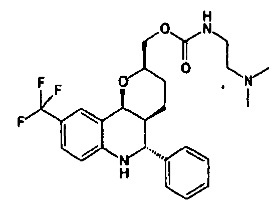 | 479 | 4,77 | A |
| (353) | 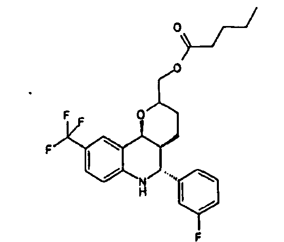 | 466 | 6,93 | B |
| (356) | 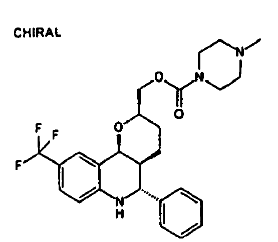 | 491 | 4,83 | A |
| (357) | 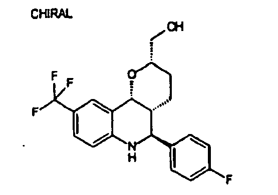 | 382 | 5.5 | A |
| (358) | 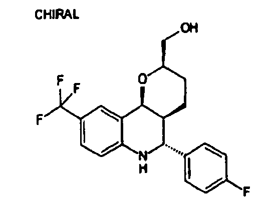 | 382 | 5.51 | A |
| (359) | 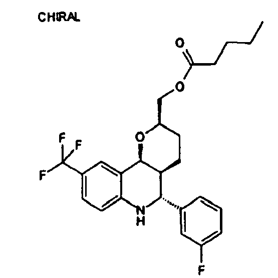 | 466 | 6,92 | B |
| (360) | 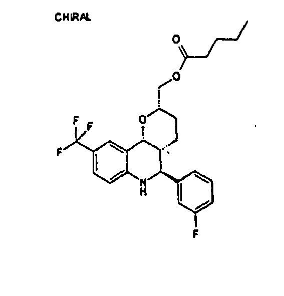 | 466 | 6,93 | B |
| (361) | 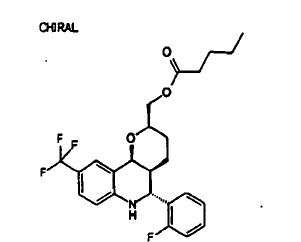 | 466 | 6,72 | A |
| (362) | 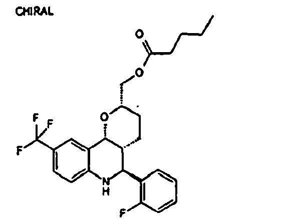 | 466 | 6,72 | A |
| (363) | 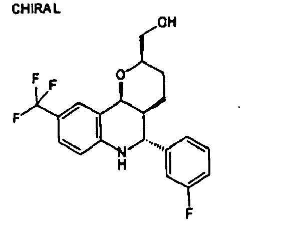 | 382 | 5,64 | B |
| (364) | 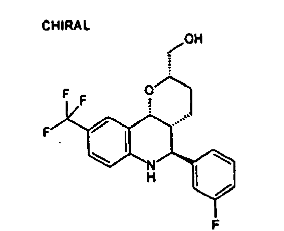 | 382 | 5,64 | B |
| (365) | 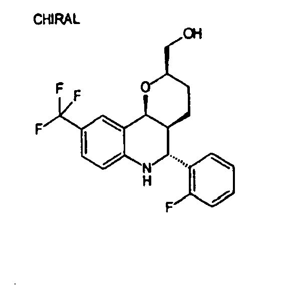 | 382 | 5,6 | A |
| (366) | 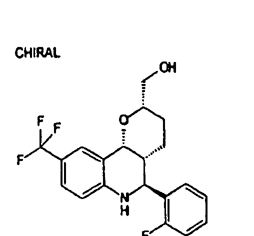 | 382 | 5,6 | A |
| (368) | 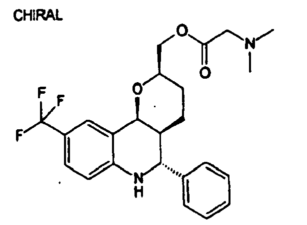 | 449 | 5,01 | A |
| (562) | 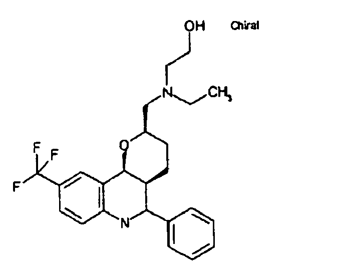 | | | |
| (563) | 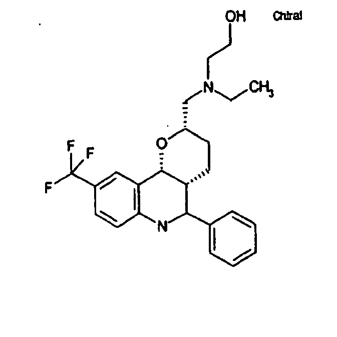 | | | |
| (564) | 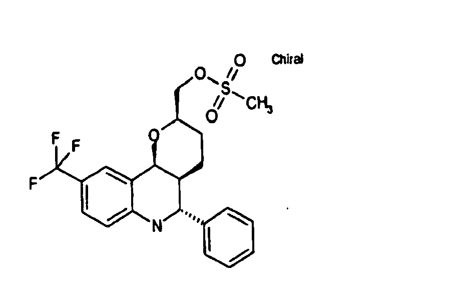 | | | |
| (565) | 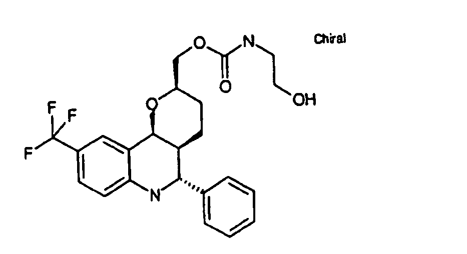 | | | |
| (566) | 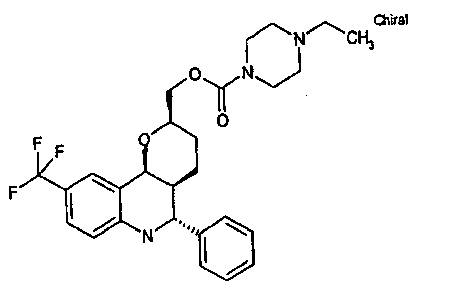 | | | |
| (567) | 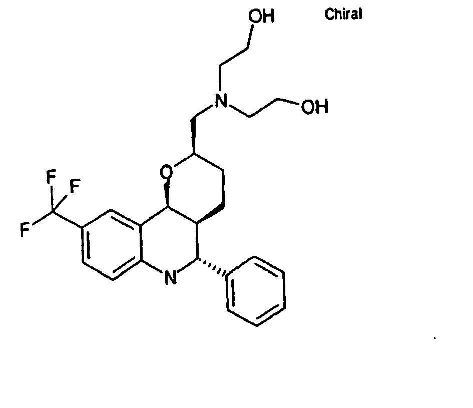 | | | |
| (568) | 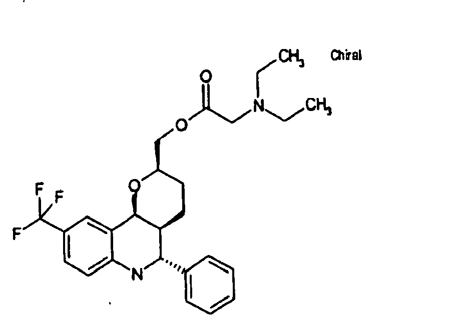 | | | |
| (569) | 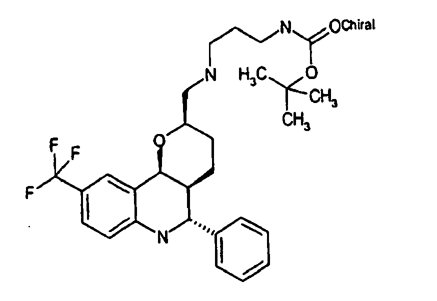 | | | |
| (570) | 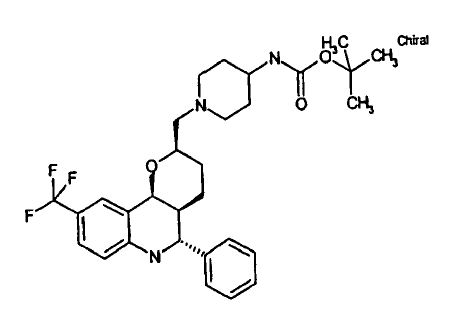 | | | |
| (571) | 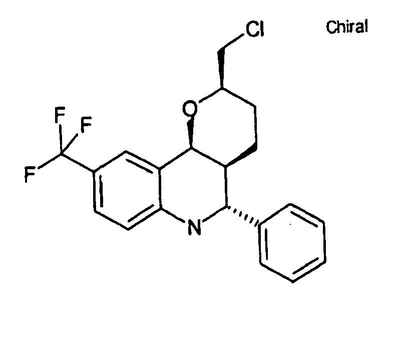 | | | |
| (572) | 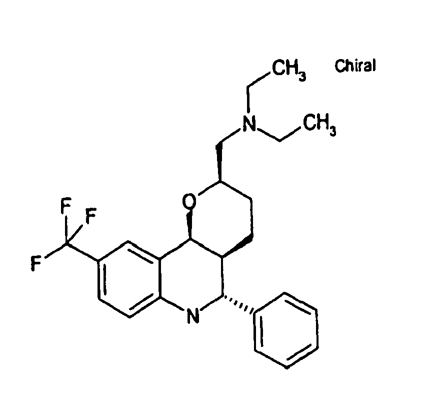 | | | |
| (573) | 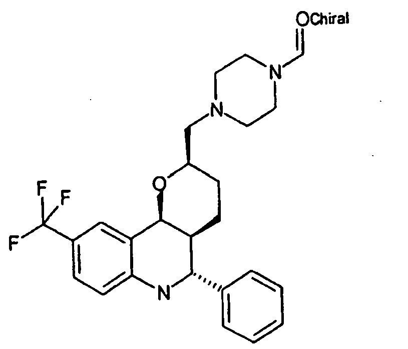 | | | |
| (574) | 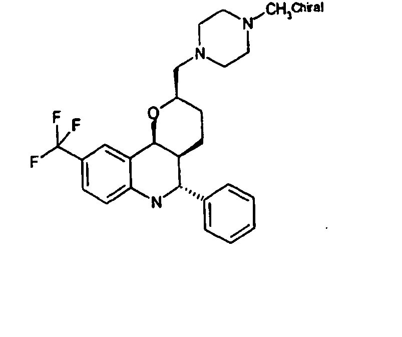 | | | |
| (575) | 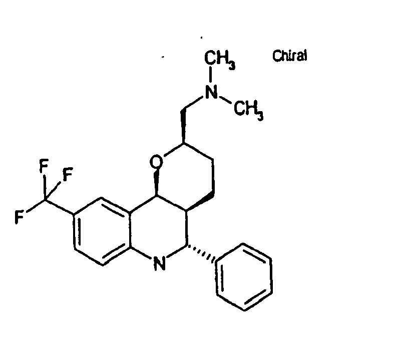 | | | |
| (576) | 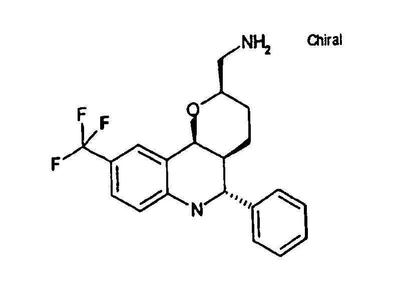 | | | |
| (577) | 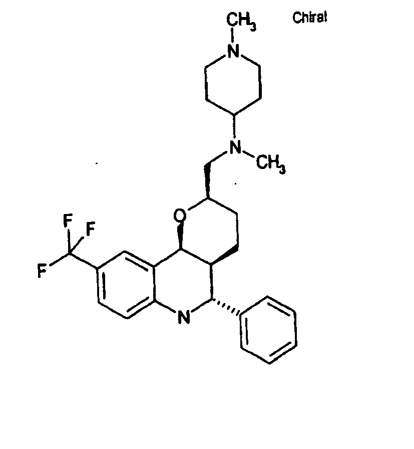 | | | |
| (578) | 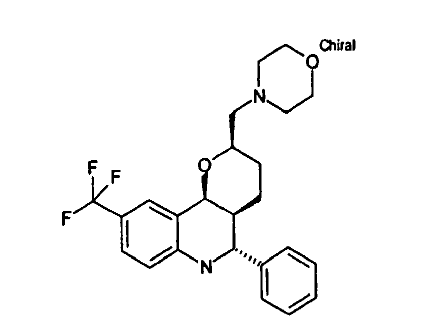 | | | |
| (579) | 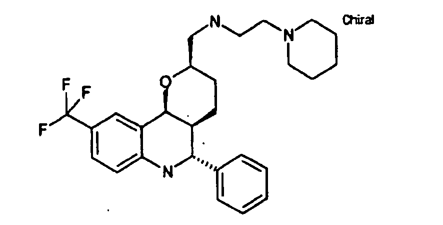 | | | |
| (580) | 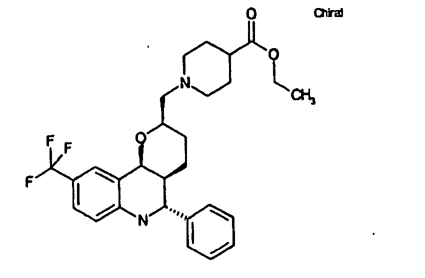 | | | |
| (581) | 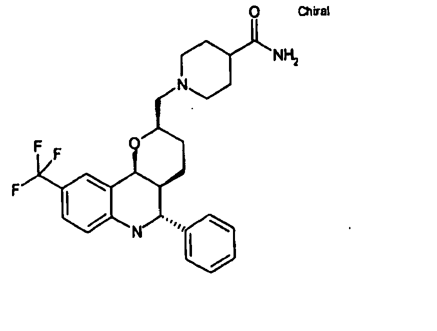 | | | |
| (582) | 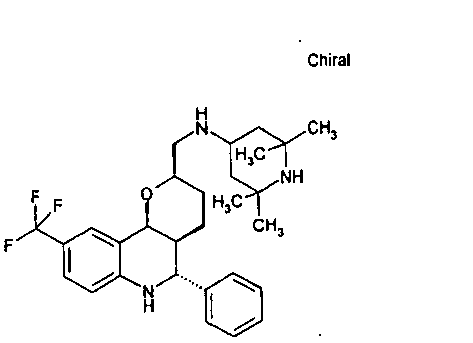 | | | |
| (583) | 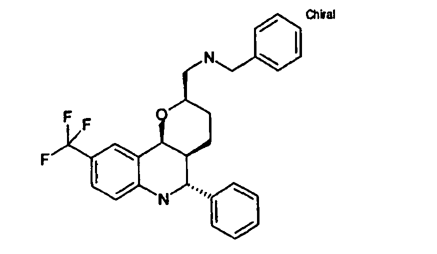 | | | |
| (584) | 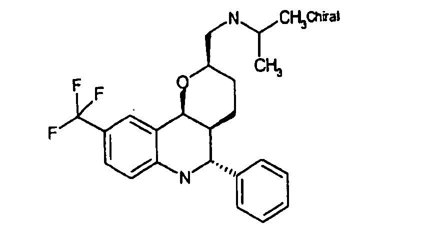 | | | |
| (585) | 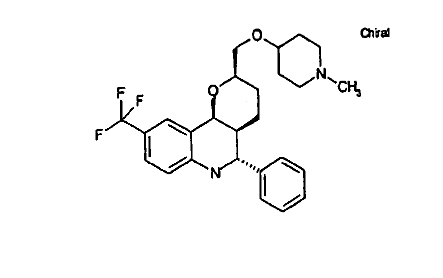 | | | |
| (586) | 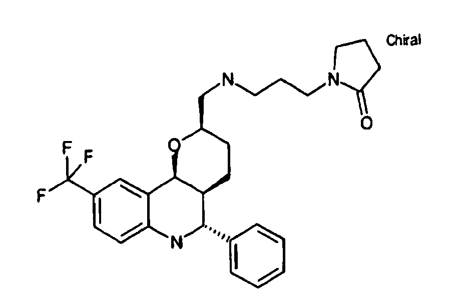 | | | |
| (587) | 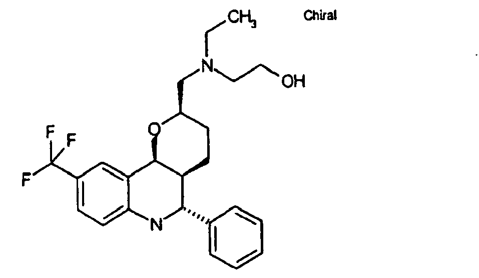 | | | |
| (588) | 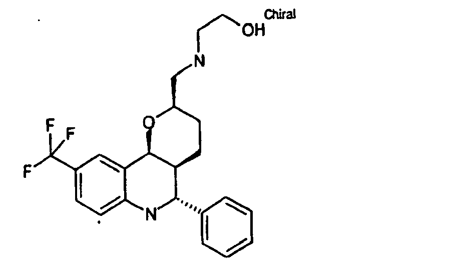 | | | |
| (589) | 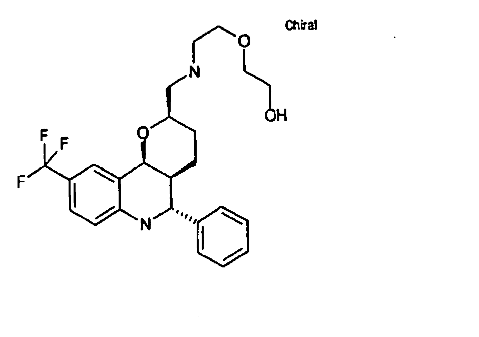 | | | |
| (590) | 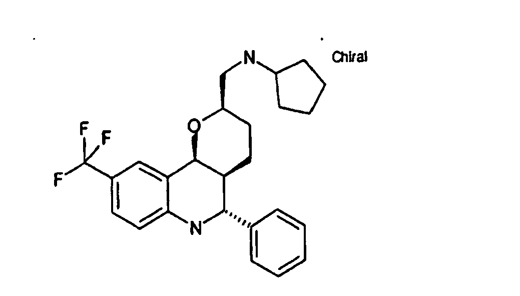 | | | |
| (591) | 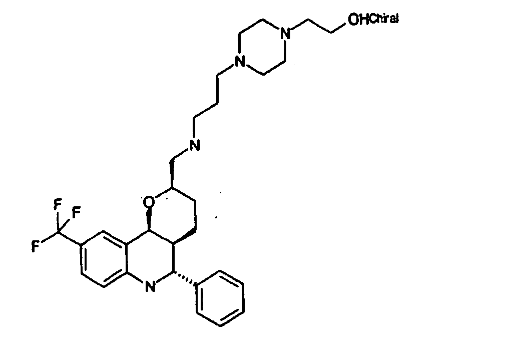 | | | |
| (592) | 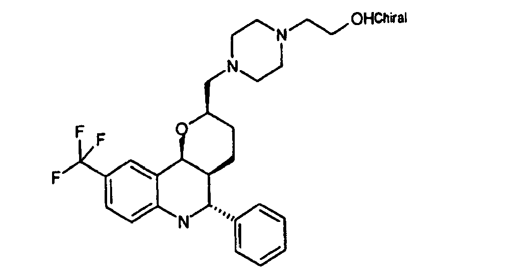 | | | |
| (593) | 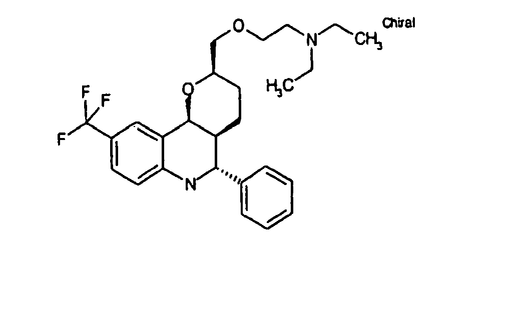 | | | |
| (594) | 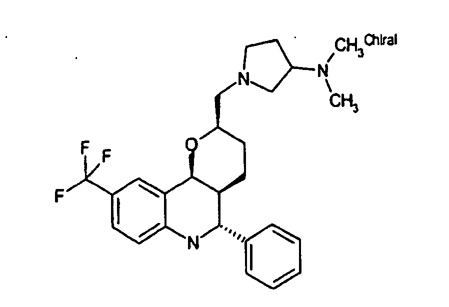 | | | |
| (595) | 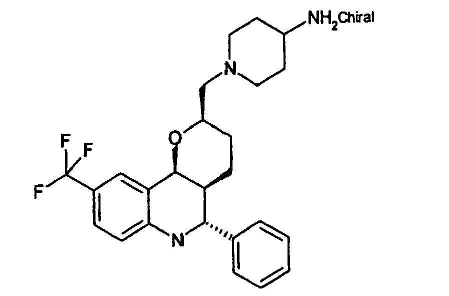 | | | |
| (596) | 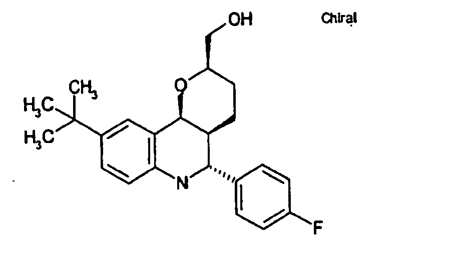 | | | |
| (597) | 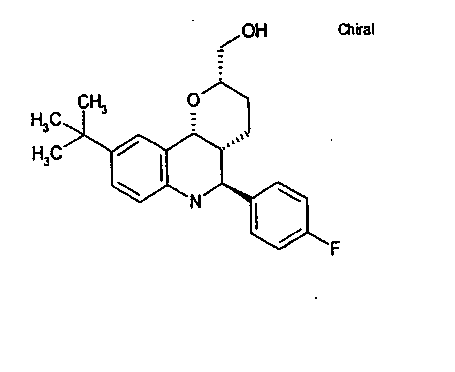 | | | |
| (598) | 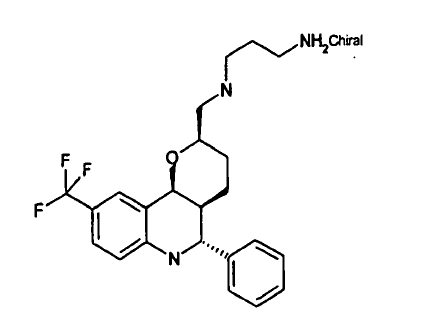 | | | |
| (599) | 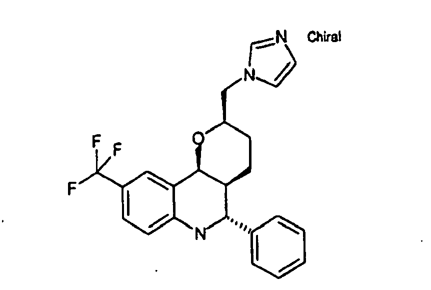 | | | |
| (600) | 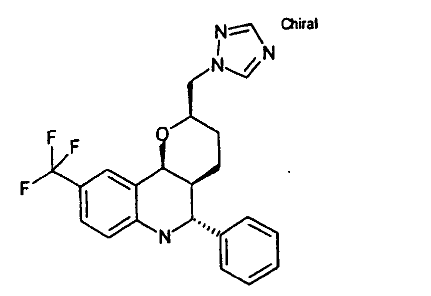 | | | |
| (601) | 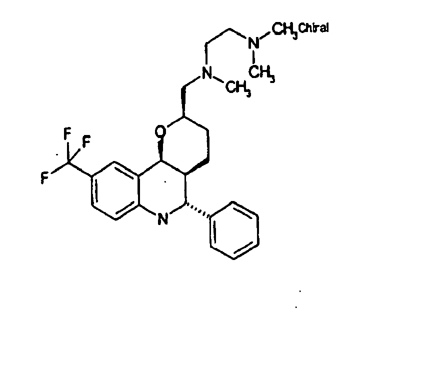 | | | |
| (602) | 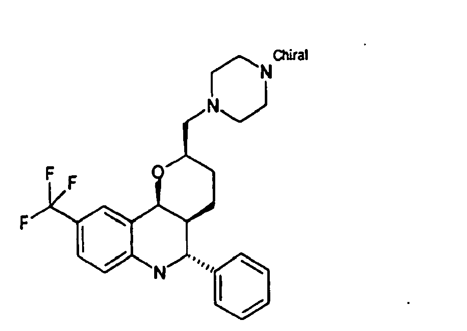 | | | |
| (603) | 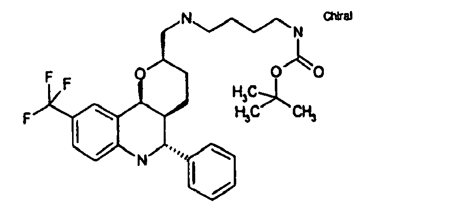 | | | |
| (604) | 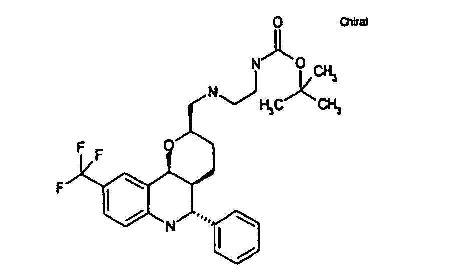 | | | |
| (605) | 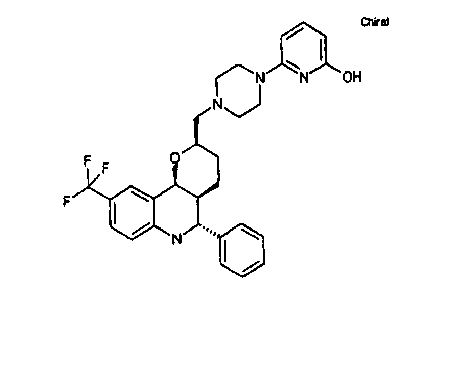 | | | |
| (606) | 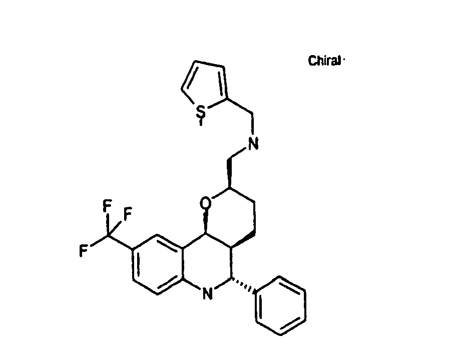 | | | |
| (607) | 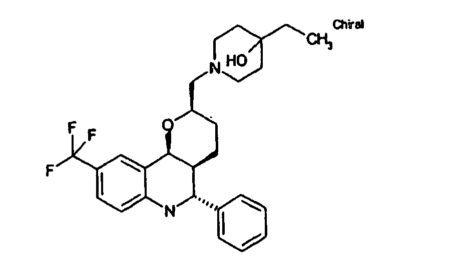 | | | |
| (608) | 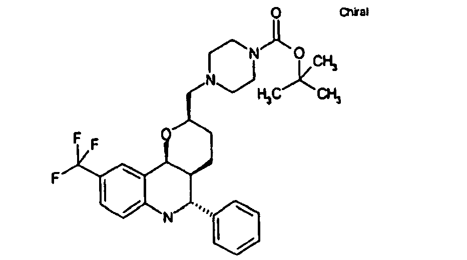 | | | |
| (609) | 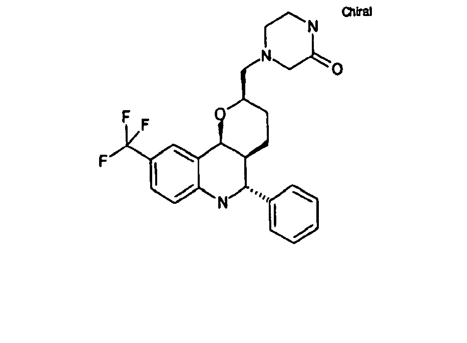 | | | |
| (610) | 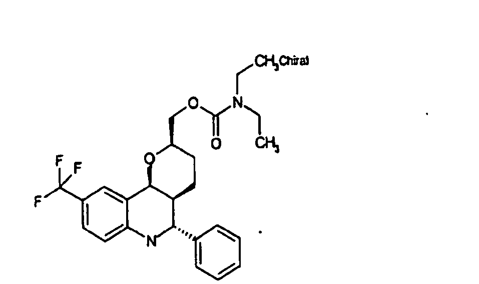 | | | |
| (611) | 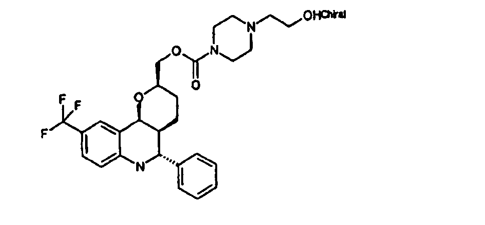 | | | |
| (612) | 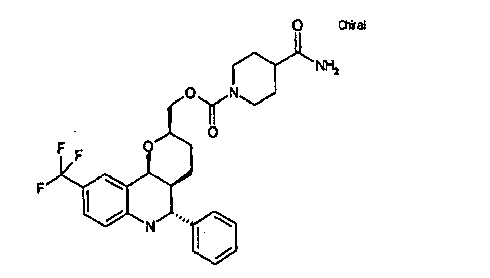 | | | |
| (613) | 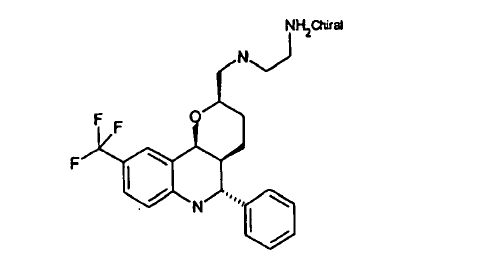 | | | |
| (614) | 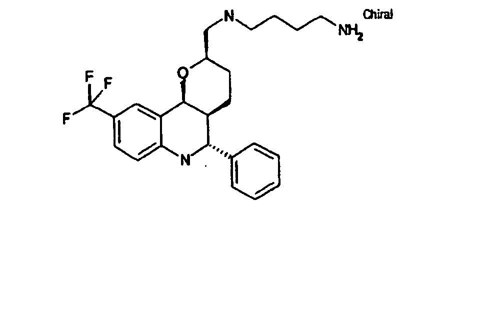 | | | |
| (615) | 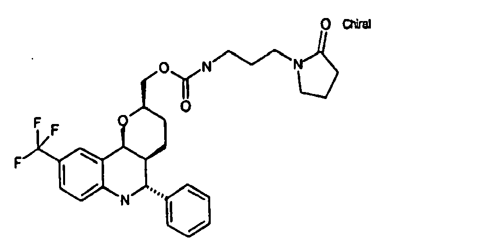 | | | |
| (616) | 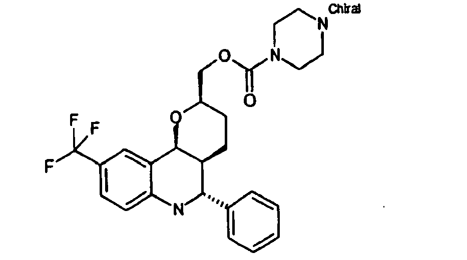 | | | |
| (617) | 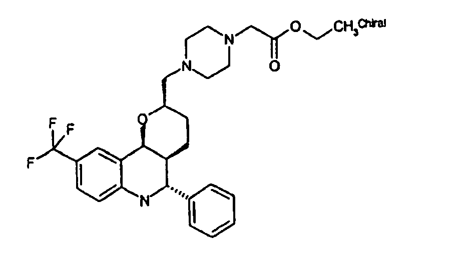 | | | |
| (618) | 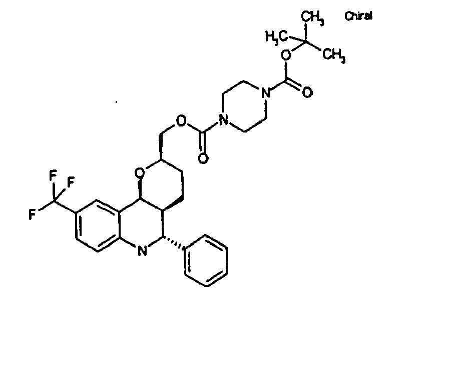 | | | |
| (619) | 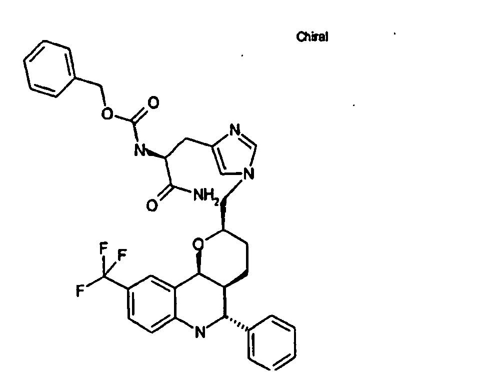 | | | |
| (620) | 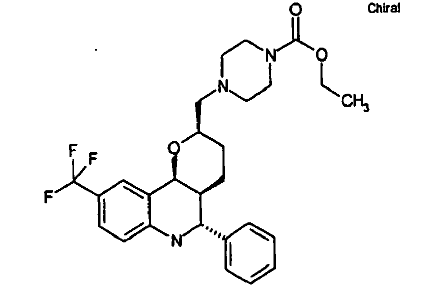 | | | |
| (621) | 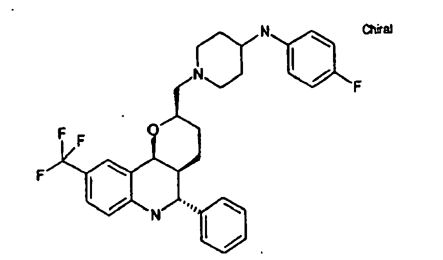 | | | |
| (622) | 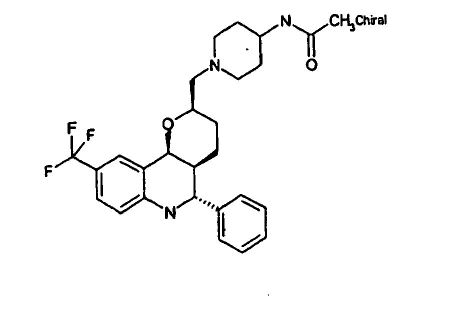 | | | |
| (623) | 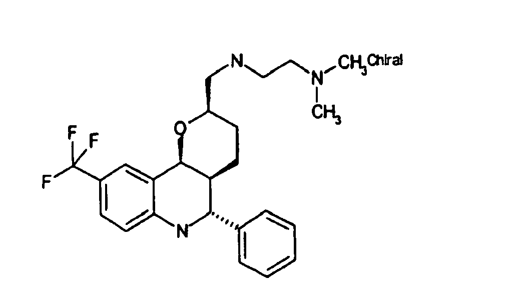 | | | |
| (624) | 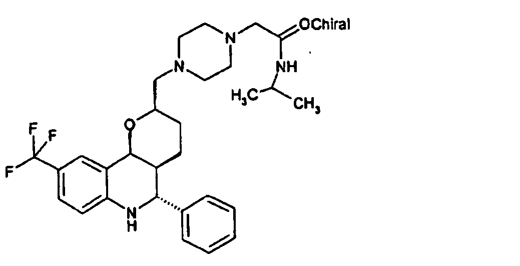 | | | |
| (625) | 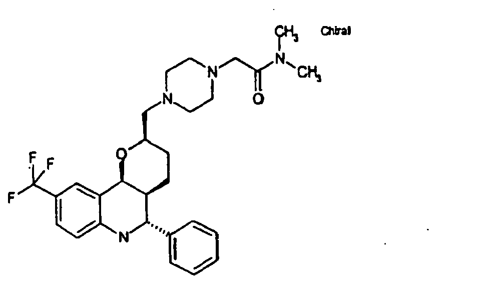 | | | |
| (626) | 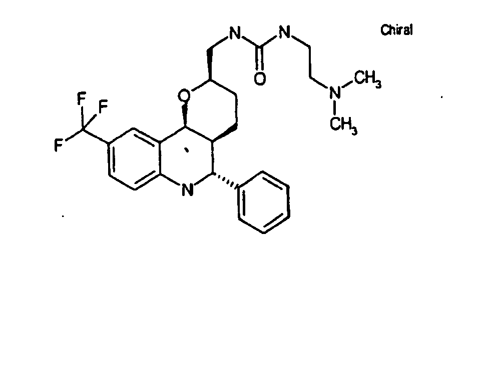 | | | |
| (627) | 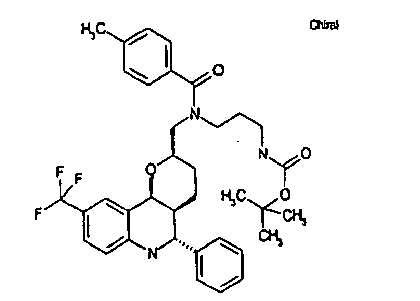 | | | |
| (628) | 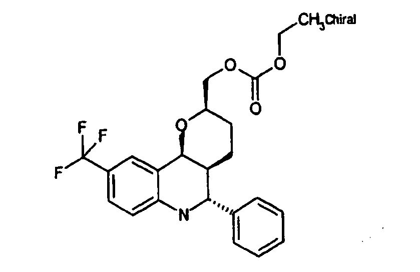 | | | |
| (629) | 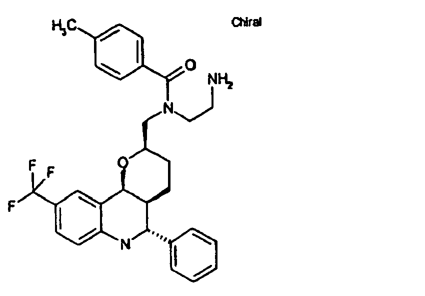 | | | |
| (630) | 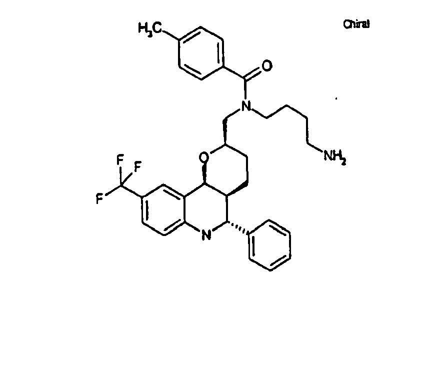 | | | |
| (631) | 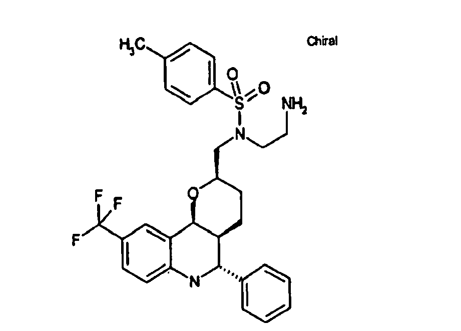 | | | |
| (632) | 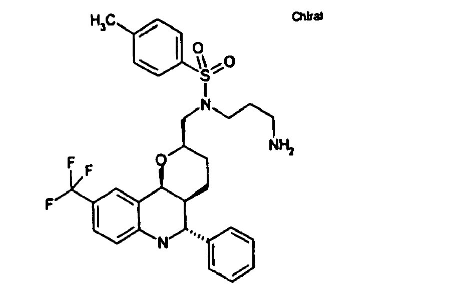 | | | |
| (633) | 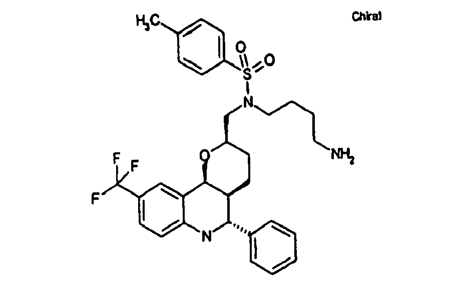 | | | |
| (634) | 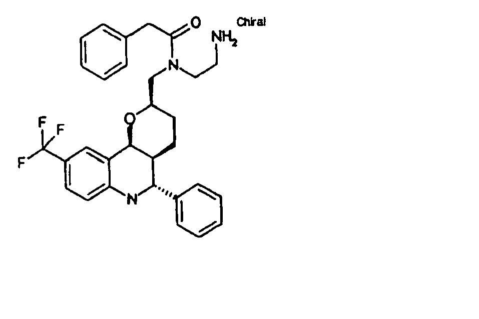 | | | |
| (635) | 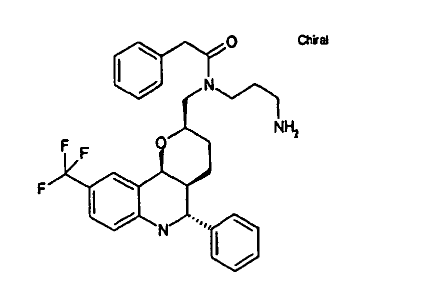 | | | |
| (636) | 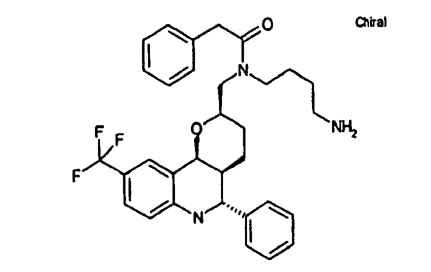 | | | |
| (637) | 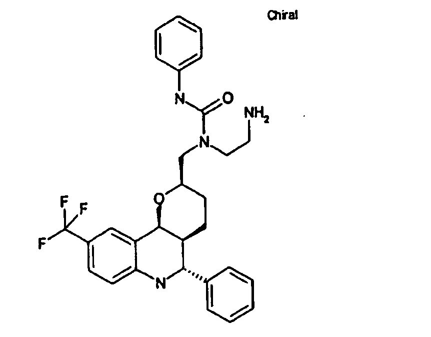 | | | |
| (638) | 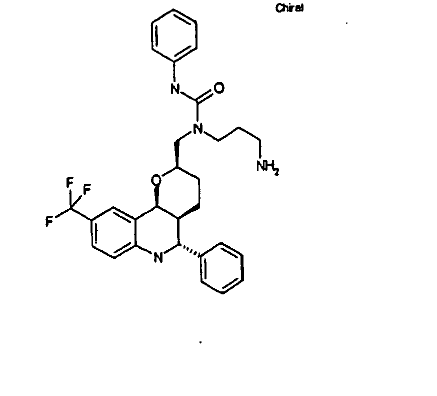 | | | |
| (639) | 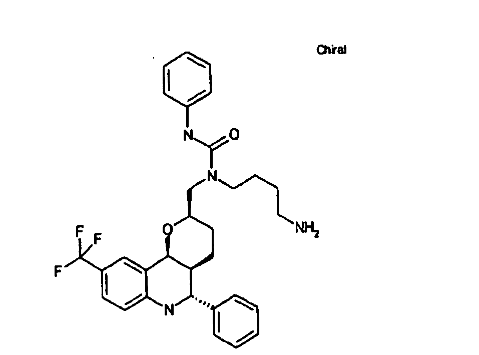 | | | |
| (640) | 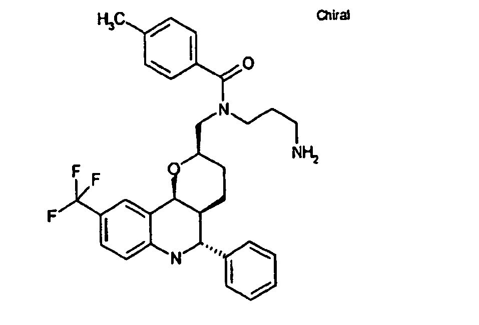 | | | |
| (641) | 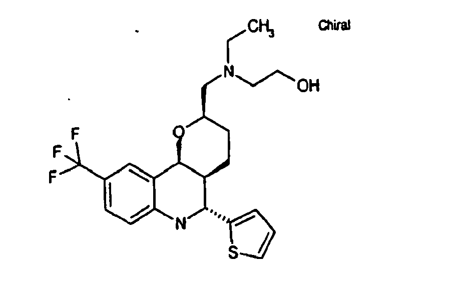 | | | |
| (642) | 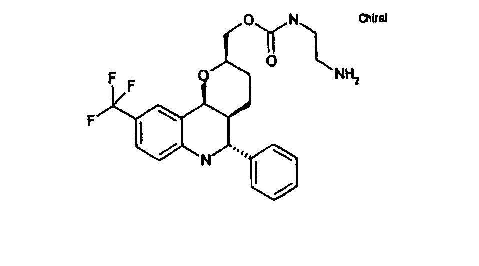 | | | |
| (643) | 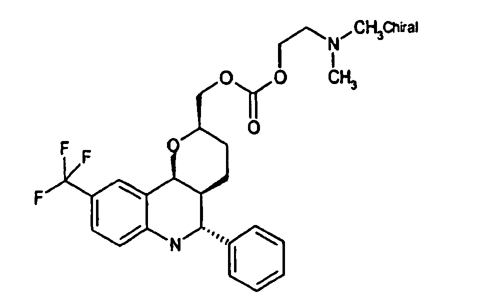 | | | |
| (644) | 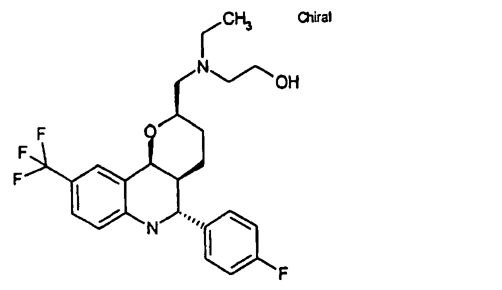 | | | |
| (645) | 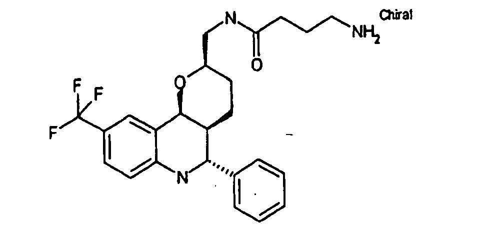 | | | |
| (646) | 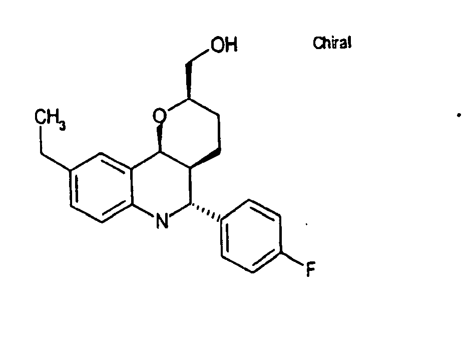 | | | |
| (647) | 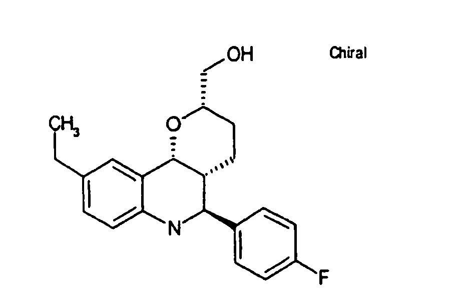 | | | |
| (648) | 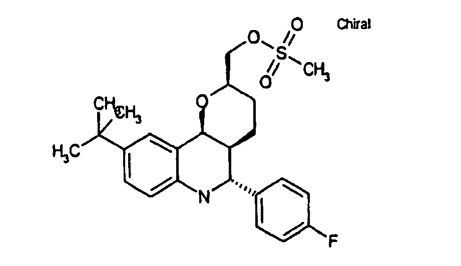 | | | |
| (649) | 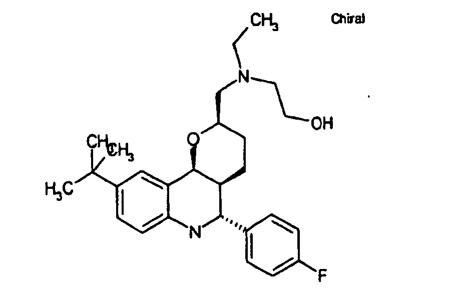 | | | |

Dabei sind die Verbindungen der Formel IA4 aus den Beispielen 562 bis 649 besonders bevorzugt da sie eine erhöhte erfindungsgemäße Wirkung zeigen.

### Beispeil A: Assay I

Die Bestimmung der Wirksamkeit der erfindungsgemäßen Verbindungen kann z. B. über die Eg5-ATPase Aktivität, die über eine enzymatische Regeneration des Produkts ADP zur ATP mittels Pyruvatkinase (PK) und anschließender Kopplung an eine NADH-abhängige Laktat-Dehydrogenase (LDH) Reaktion gemessen wird, erfolgen. Durch die Kopplung an die NADH-abhängige LDH kann die Reaktion über die Änderung der Extinktion bei 340 nm verfolgt werden. Die Regeneration des ATP gewhärleistet gleichzeitig, dass die Substratkonzentration konstant bleibt. Die Extinktionsänderung pro Zeiteinheit werden graphisch analysiert und eine lineare Regression im vusuell linearen Bereich der Reaktion durchgeführt.

### Beispiel B: Assay II

Die Kombination aus dem Antiprotozoikum Pentamidin und den Inhibitoren der Kinesin-ATPase Eg5/KSP führt zu verstärkter hemmender Wirkungen bei Zellproliferationstests mit der Kolon-Karzinom-Zelllinie HCT116. Eg5-Inhibitoren stören die ATPase-Aktivität und hemmen den Verlauf des Zellzyklus aufgrund eines Fehlers bei der Trennung der Spindelpole.

Die Bestimmung der Wirksamkeit der erfindungsgemäßen Verbindungen der Formel IA4 in kombination mit Verbindungen der Formel V und/oder Arzneimitteln der Tabelle I kann in Kombinations-Assays wie folgt gezeigt werden:
10³ bis 10⁴ Zellen einer definierten Zell-Linie (HCT116, Colo 205, MDA-MB 231, etc.) werden pro Vertiefung in einer 96-well Mikrotiterplatte ausgesät und über Nacht unter Standardbedingungen kultiviert. Für die Substanzen der zu testenden Kombination wurden 10-50 mM Stocklösungen in DMSO vorbereitet. Verdünnungsreihen (i.d.R. 3-fach Verdünnungsschritte) der einzelnen Substanzen wurden in Form einer Pipettierschemas (s. Schema unten), unter Konstanthaltung einer DMSO Endkonzentration von 0,5 % (v/v) miteinander kombiniert. Die Zellen wurden am nächsten Morgen mit den Substanzgemischen versetzt und für weitere 48 Stunden unter Kulturbedingungen inkubiert. Am Ende der Kultivierung erfolgte eine Kristallviolett-Färbung der Zellen. Nach Extraktion des Kristallviolett aus den fixierten Zellen wurde die Absorption bei 550 nm spektralphotometrisch gemessen. Sie kann als quantitatives Maß für die vorhandenen adhärenten Zellen herangezogen werden.

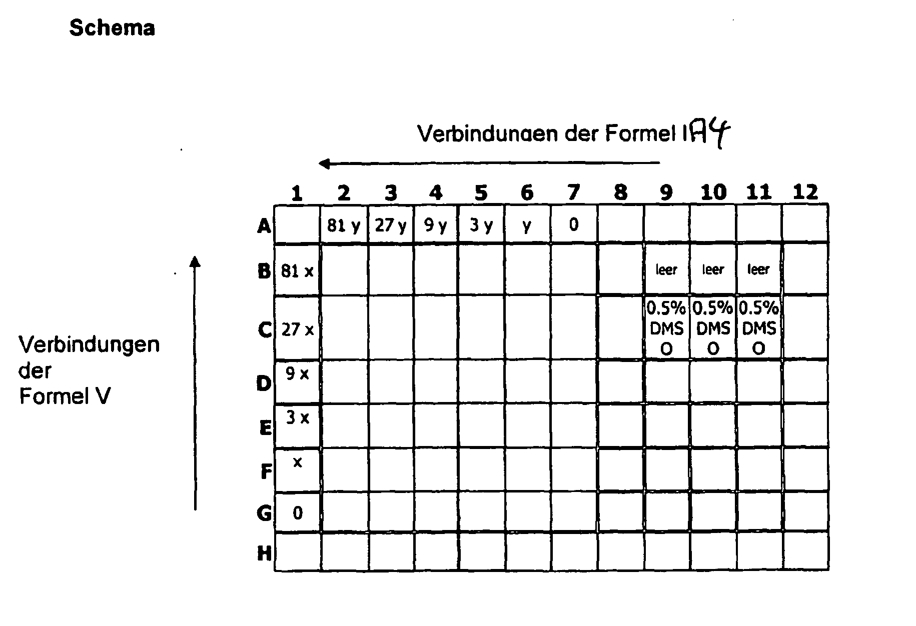

Die nachfolgenden Ergebnisse geben beispeilhaft Hinweise zur Hemmung des Zellzyklus durch die erfindungsgemässe Verbindung 112,

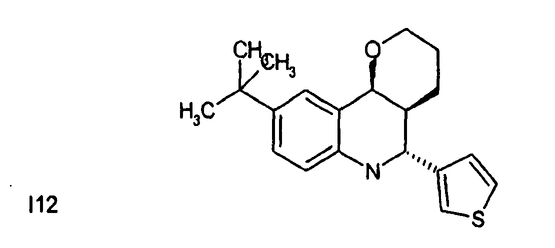

Monasterol und Pentamidin in Einzelexperimenten:
- HCT116-Zellen wurden 24 Std. mit den gegebenen Konzentrationen der Verbindungen behandelt, für die Propidiumiodid-Analyse mit einem BD-FACS-Scan verarbeitet, um den DNA-Gehalt der behandelten Zellpopulationen zu bestimmen. Der Prozentsatz an Zellen in der G0/G1-Phase (unreplizierte DNA), S-Phase (DNA-Replikationsphase) und G2/M-Phase (duplizierte DNA vor der Mitose) ist gezeigt. 10000 Ereignisse wurden für jede Konzentration gezählt. Auffallende Änderungen der G2/M-Anreicherung wurden hervorgehoben.

### I12 (erfindungsgemäßer Eg5-Inhibitor):

| µM | G1 | S | G2/M |
|---|---|---|---|
| 0 | 36 | 43 | 21 |
| 0.0032 | 36 | 44 | 21 |
| 0.016 | 36 | 43 | 21 |
| 0.08 | 35 | 42 | 23 |
| 0.4 | 7 | 7 | 86 |
| 2 | 0 | 15 | 85 |

### Monastrol (Eg5-Inhibitor aus dem Stand der Technik)

| µM | G1 | S | G2/M |
|---|---|---|---|
| 0 | 41 | 38 | 21 |
| 0.08 | 35 | 39 | 25 |
| 0.4 | 34 | 38 | 28 |
| 2 | 33 | 42 | 25 |
| 10 | 34 | 39 | 27 |
| 50 | 14 | 33 | 52 |

### Pentamidin-Isothionat

| µM | G1 | S | G2/M |
|---|---|---|---|
| 0 | 36 | 42 | 22 |
| 0.016 | 36 | 42 | 22 |
| 0.08 | 35 | 44 | 21 |
| 0.4 | 33 | 45 | 22 |
| 2 | 32 | 44 | 24 |
| 10 | 32 | 32 | 32 |

Die nachfolgenden Ergebnisse geben beispielhaft Hinweise zur Hemmung des Zellzyklus durch die erfindungsgemässe Verbindung I12 oder Monasterol und Pentamidin in Kombinationsexperimenten:

### Kombinationstherapie: 112 plus Pentamidin

HCT116-Zellen wurden in Platten mit 96 Vertiefungen bei einer Dichte von 2500 oder 5000 Zellen/Vertiefung über Nacht bei 37°C inkubiert. Am folgenden Tag wurde eine Konzentrationsmatrix von Pentamidin und den angegebenen Verbindungen zugegeben, und die Zellen wurden weitere 48 Std. inkubiert. Die Zellen wurden dann mit Kristallviolett gefärbt, und die Absorption wurde nach der Farbstoffextraktion gemessen. Der Absorptionswert spiegelt die Anzahl der Zellen in der Vertiefung wider.

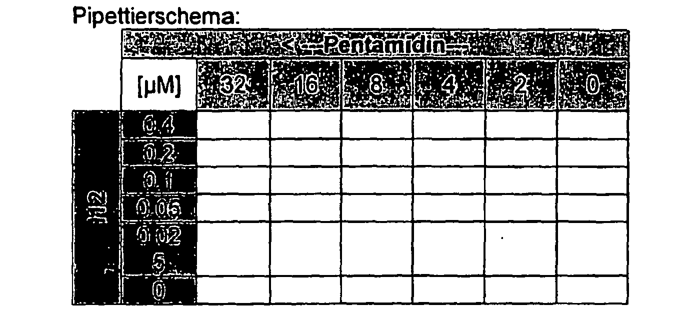

### Ergebnisse:

Obere Matrix: OD550 nm Hintergrund korrigiert
Mittlere Matrix: % der Kontrolle (der 100%-Wert steht für mit Vehikel behandelte Zellen minus Hintergrund-Signal)
Unteres Feld: -fache Hemmwerte (d.h. der 100%/x%-Wert steht für eine - fache Hemmung der Proliferation).

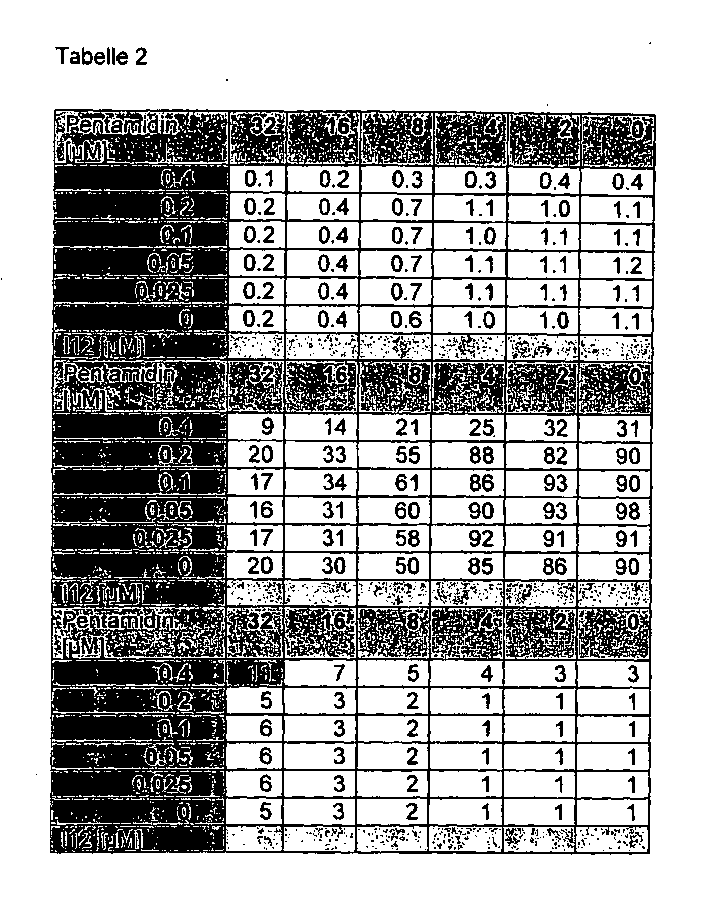

### Graphische Darstellung der Daten:

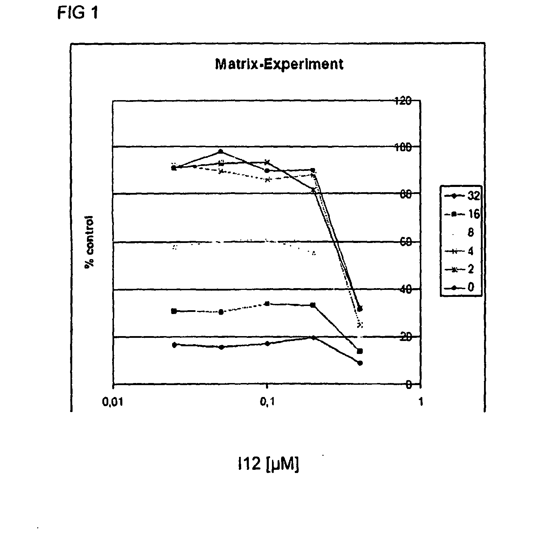

Die Daten wurden in FIG 1 als Konzentrationskurven dargestellt (jede Linie veranschaulicht eine Kurve bei einer gegebenen Pentamidin-Konzentration)

### Kombinationstherapie: Monastrol plus Pentamidin

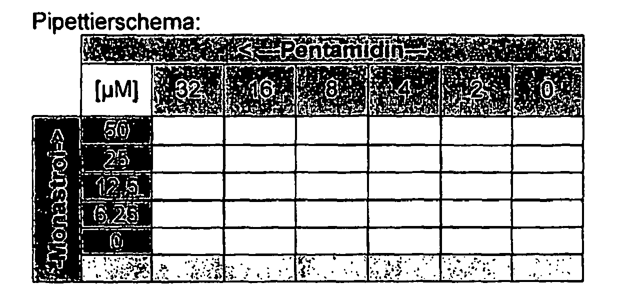

### Ergebnisse:

Obere Matrix: OD550 nm Hintergrund korrigiert
Mittlere Matrix: % der Kontrolle (der 100%-Wert steht für mit Vehikel behandelte Zellen minus Hintergrund-Signal)
Unteres Feld: -fache Hemmwerte (d.h. der 100%/x%-Wert steht für eine - fache Hemmung der Proliferation).

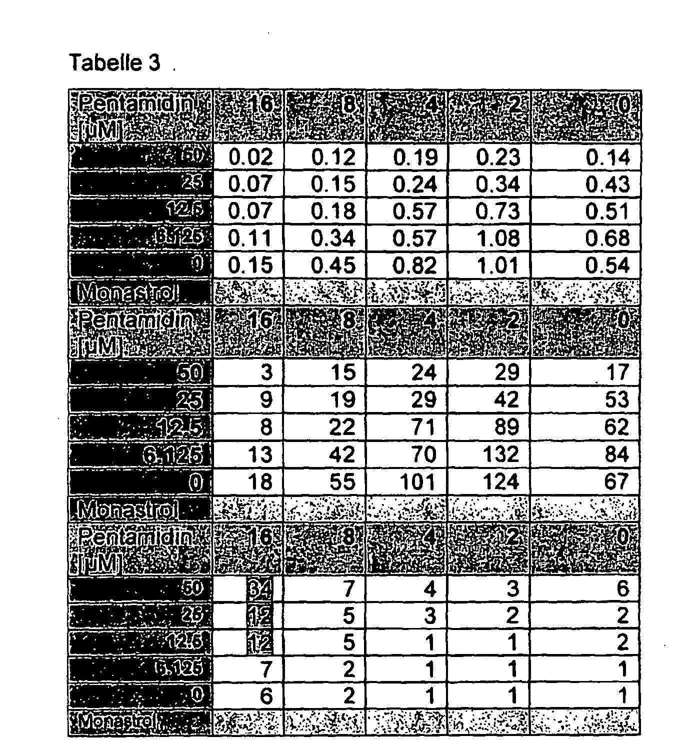

### Graphische Darstellung der Daten:

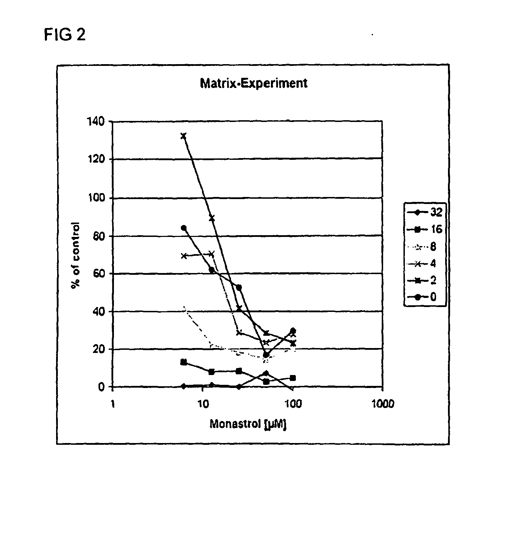

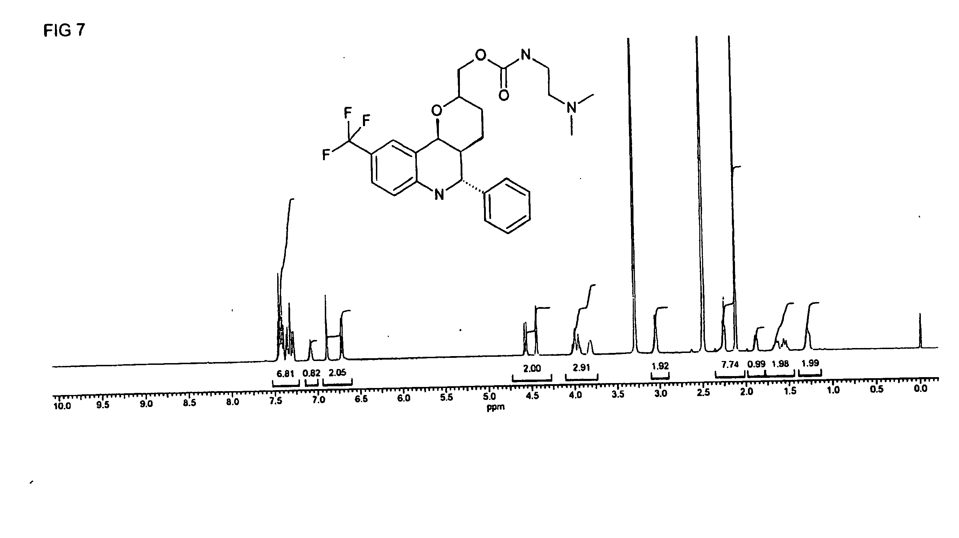

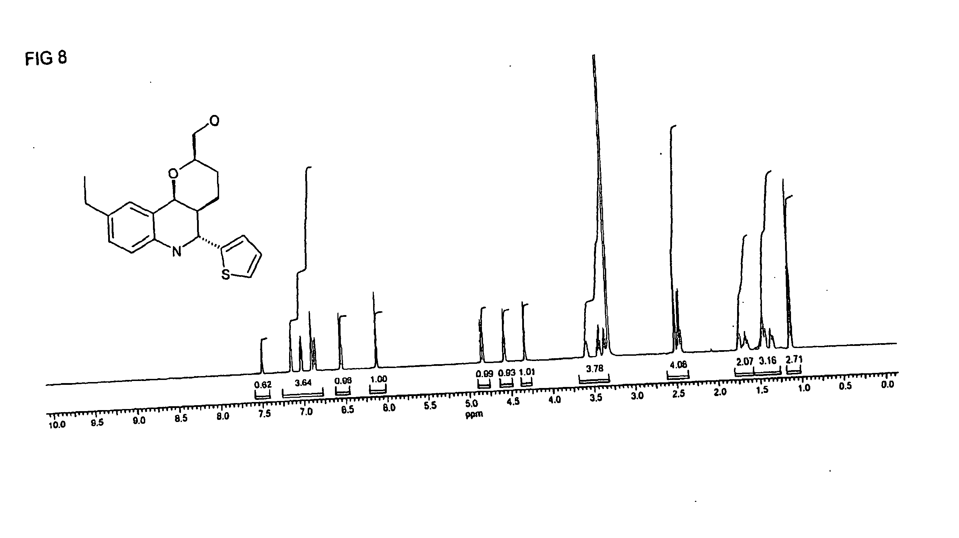

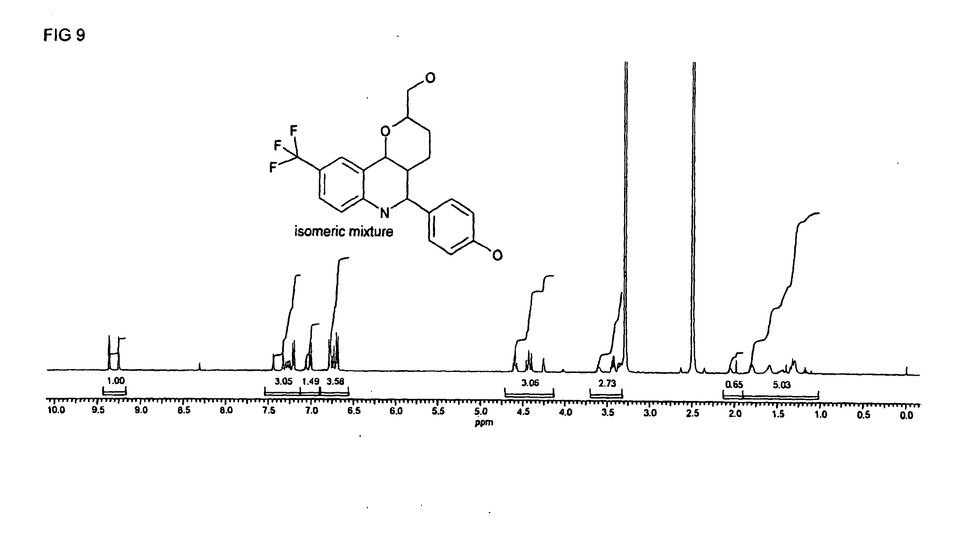

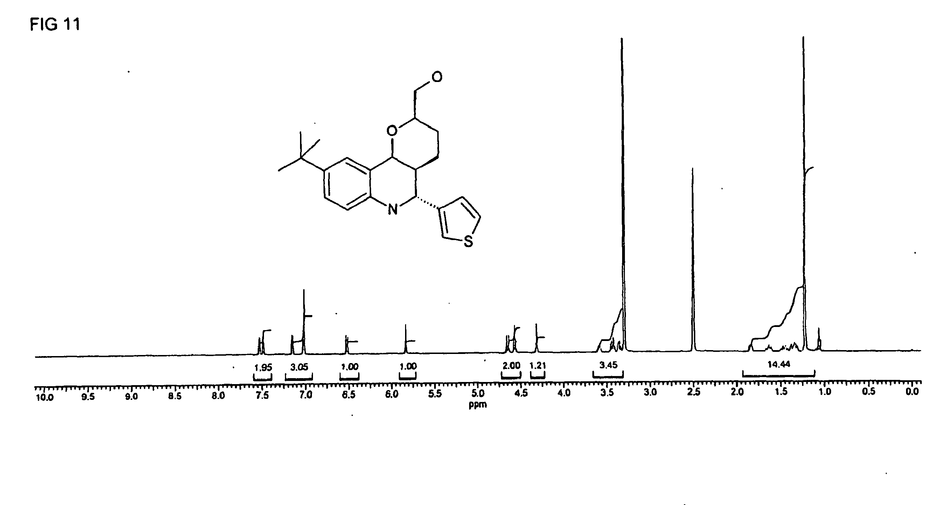

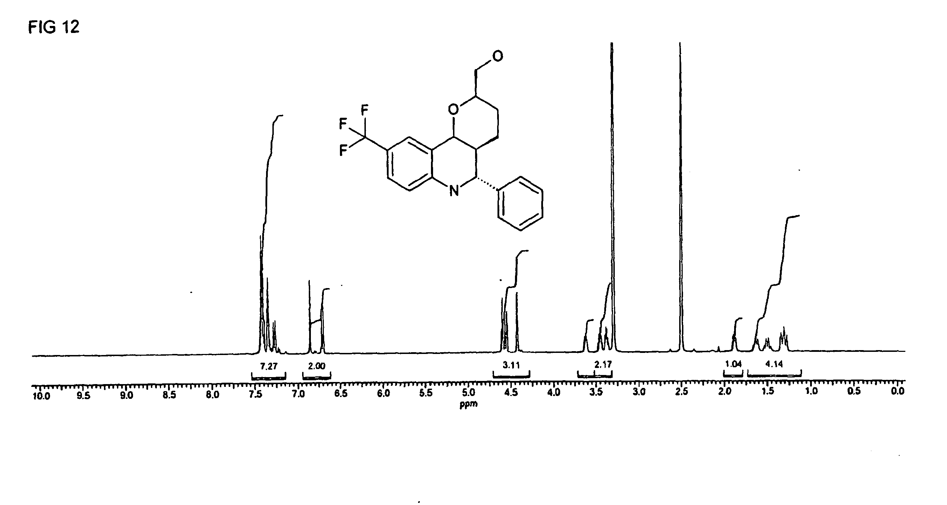

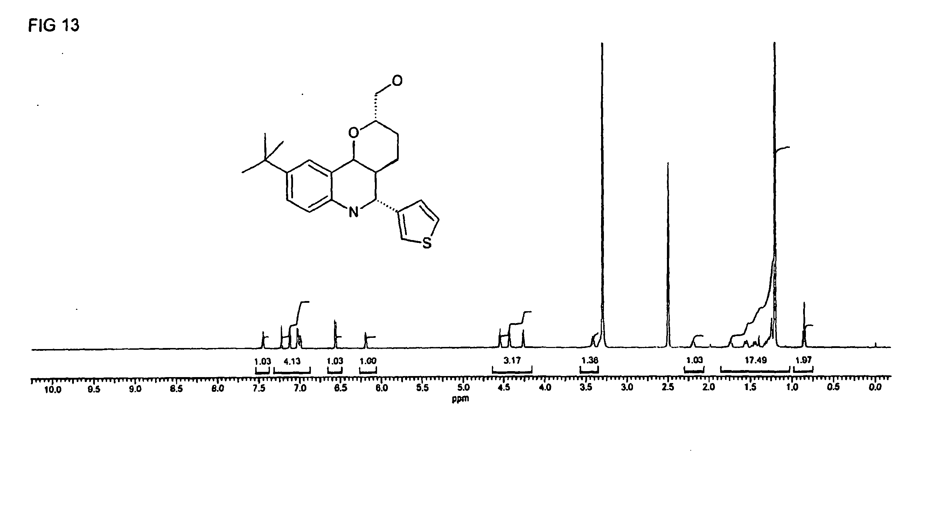

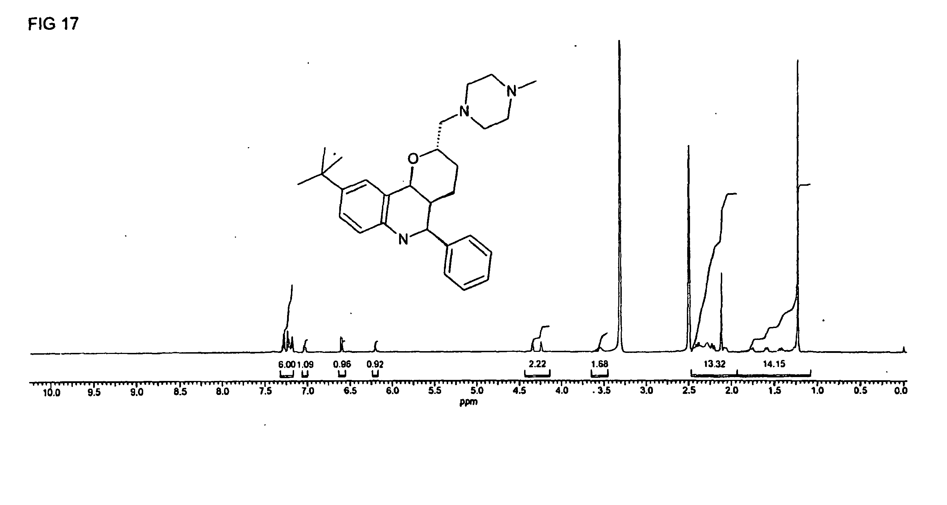

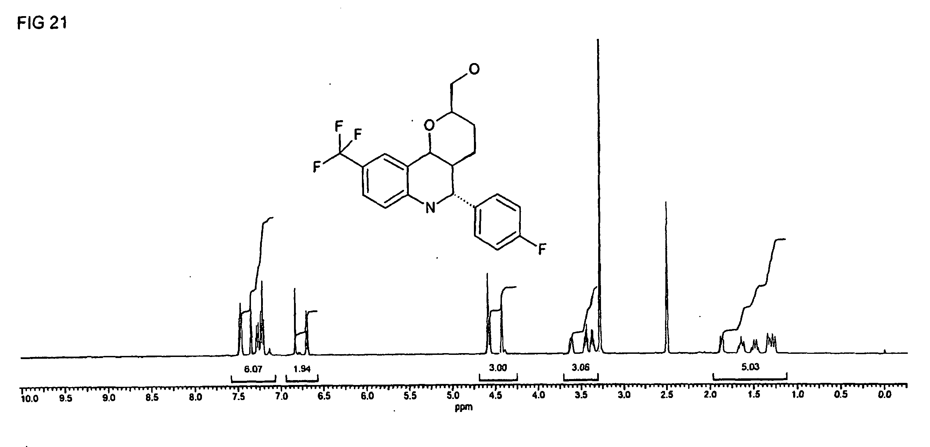

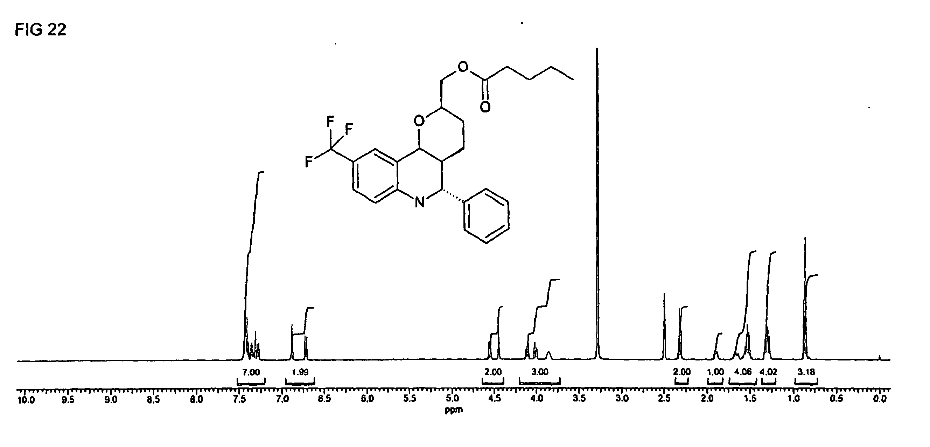

Die Daten wurden in FIG 2 als Konzentrationskurven dargestellt (jede Linie veranschaulicht eine Kurve bei einer gegebenen Pentamidin-Konzentration)

Die nachfolgenden Beispiele betreffen Arzneimittel:

### Beispiel C: Injektionsgläser

Eine Lösung von 100 g eines Wirkstoffes der Formel IA4 und 5 g Dinatrium-hydrogenphosphat wird in 3 I zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel D: Suppositorien

Man schmilzt ein Gemisch von 20 g eines Wirkstoffes der Formel IA4 mit 100 g Sojalecithin und 1400 g Kakaobutter, gießt in Formen und läßt erkalten. Jedes Suppositorium enthält 20 mg Wirkstoff.

### Beispiel E: Lösung

Man bereitet eine Lösung aus 1 g eines Wirkstoffes der Formel IA4, 9,38 g NaH₂PO₄ 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 I auf und sterilisiert durch Bestrahlung. Diese Lösung kann in Form von Augentropfen verwendet werden.

### Beispiel F: Salbe

Man mischt 500 mg eines Wirkstoffes der Formel IA4 mit 99,5 g Vaseline unter aseptischen Bedingungen.

### Beispiel G: Tabletten

Ein Gemisch von 1 kg Wirkstoff der Formel IA4, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel H: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel I: Kapseln

2 kg Wirkstoff der Formel IA4 werden in üblicher Weise in Hartgelatinekapseln gefüllt, so daß jede Kapsel 20 mg des Wirkstoffs enthält.

### Beispiel J: Ampullen

Eine Lösung von 1 kg Wirkstoff der Formel IA4 in 60 l zweifach destilliertem Wasser wird steril filtriert, in Ampullen abgefüllt, unter sterilen Bedingungen lyophilisiert und steril verschlossen. Jede Ampulle enthält 10 mg Wirkstoff.

### Bildbeschreibungen

**FIG 1** Graphische Darstellung der Daten aus Tabelle 2
   Die Daten wurden als Konzentrationskurven der Substanz der Formel I12 dargestellt. Jede Linie veranschaulicht eine Kurve bei einer gegebenen Pentamidin-Konzentration.
**FIG 2**
   Graphische Darstellung der Daten aus Tabelle 3
   Die Daten wurden als Konzentrationskurven der Substanz Monastrol dargestellt. Jede Linie veranschaulicht eine Kurve bei einer gegebenen Pentamidin-Konzentration.
FIG 7-9,11-13,17,21,22
   NMR-Spektren repräsentativer Verbindungen der Formel IA4

## Patentansprüche

1. Verbindungen der Formel IA4 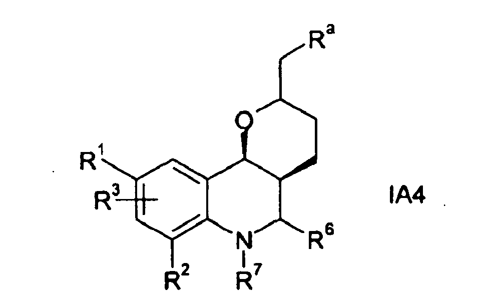 worin
R¹ A, CF₃, OCF₃, SA, SCN, CH₂CN, -OCOA, Hal oder SCF₃,
R² F oder H,
R³ F oder H,
R^{a} 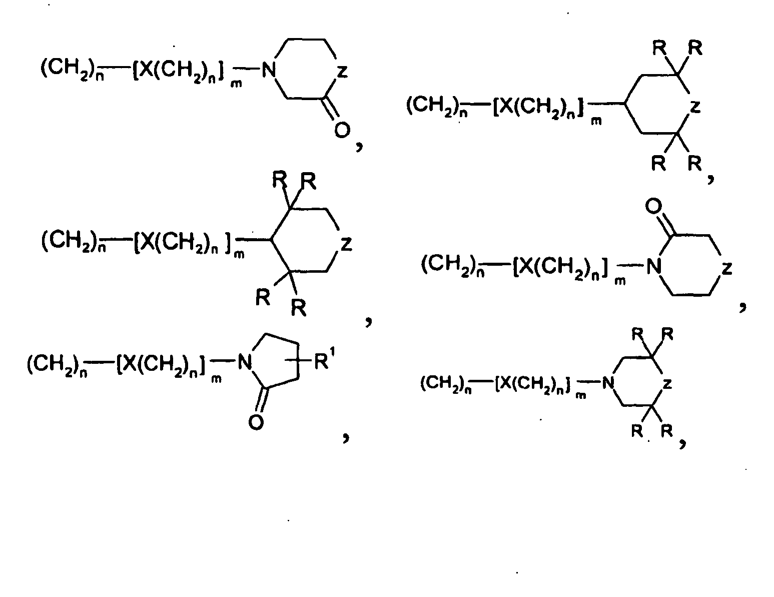 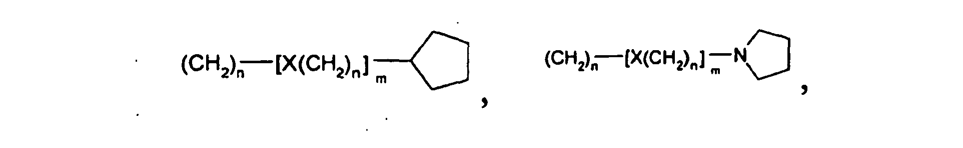 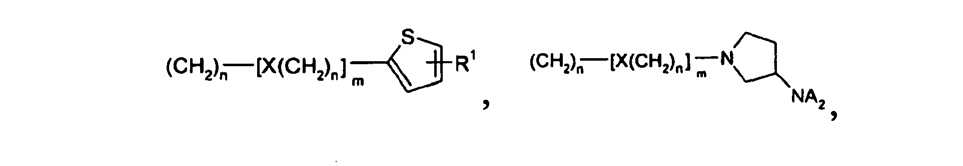 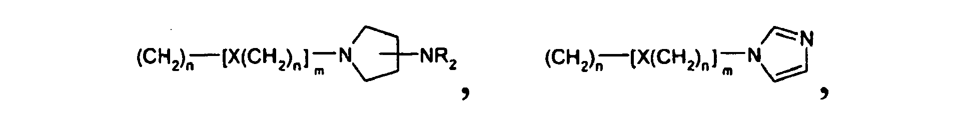 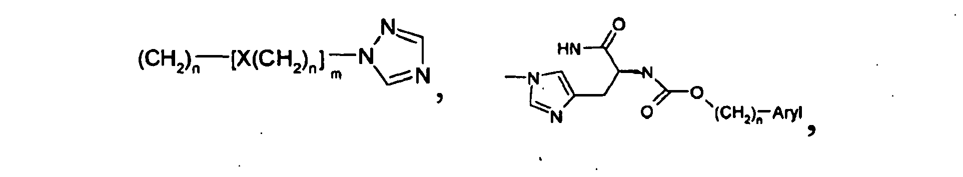 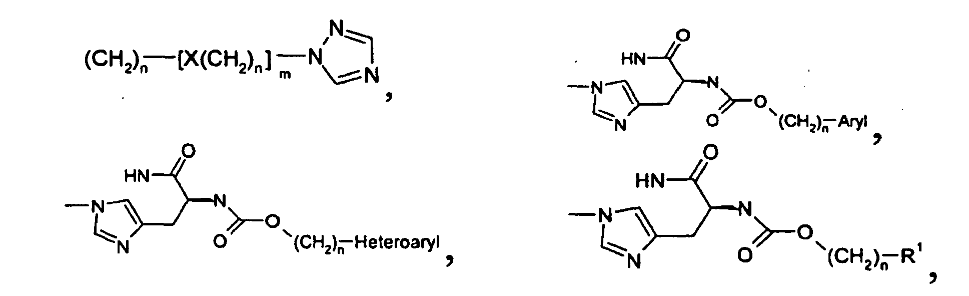 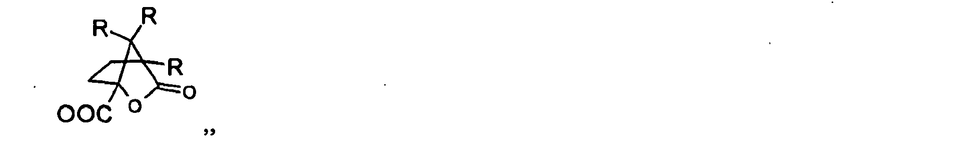 OR, NHR₂, NR₂, OR, NHR₂, NR₂, NR(CH₂)ₙAryl, NR(CH₂)ₙOR, COOR, N-Pyrrolidon-Rest, OCOR, NR(CH₂)ₙNR₂, N[(CH₂)ₙNR₂]CO(CH₂)ₙAryl, N[(CH₂)ₙNHCOOR]COAryl, R¹, N[CH₂(CH₂)ₙOR]₂, NR(CH₂)ₙNCOOR, X(CH₂)ₙX(CH₂)ₙXR, NR(CH₂)ₙX(CH₂)ₙOH, NR(CH₂)ₙO(CH₂)ₙOH, (CH₂)ₙCOOR, O(CO)NR(CH₂)ₙOR, O(CO)(CH₂)ₙNR₂, NR(CH₂)ₙNR₂, N[(CH₂)ₙNR₂]CO(CH₂)ₙAryl, N[(CH₂)ₙXR]CO(CH₂)ₙAryl, N[(CH₂)ₙXR]CO(CH₂)ₙHeteroaryl, N[(CH₂)ₙNR₂]CO(CH₂)ₙHeteroaryl, N[(CH₂)ₙNR₂]CO(CH₂)ₙR¹, N(R)(CH₂)ₙN(R)COOR, XCOO(CH₂)ₙNR₂, OSO₂A, OSO₂CF₃, OSO₂Ar, OCONR₂, OCH₂(CH₂)ₙNR₂
R H oder A, bei geminalen Resten R zusammen auch - (CH₂)₅-, -(CH₂)₄-, -(CH₂)ₙ-X-(CH₂)ₙ oder -(CH₂)ₙ-Z-(CH₂)ₙ,
A Alkyl oder Cycloalkyl, worin eines oder mehrere H-Atome durch Hal ersetzt sein können,
Hal F oder Cl,
X O, S oder NR,
Z CH₂, X, CHCONH₂, CH(CH₂)ₙNRCOOR, CHNRCOOR, NCO, CH(CH₂)ₙCOOR, NCOOR, CH(CH₂)ₙOH, N(CH₂)ₙOH, CHNH₂, CH(CH₂)ₙNR₂, CH(CH₂)ₙNR₂, C(OH)R, CHNCOR, CH(CH₂)ₙ-Aryl, CH(CH₂)ₙ-Heteroaryl, CH(CH₂)ₙR¹, N(CH₂)ₙCOOR, CH(CH₂)ₙX(CH₂)ₙ-Aryl, CH(CH₂)ₙX(CH₂)ₙ-Heteroaryl, N(CH₂)ₙCONR₂, XCONR(CH₂)ₙNR₂, N[(CH₂)ₙXCOOR]CO(CH₂)ₙ-Aryl, N[(CH₂)ₙXR]CO(CH₂)ₙ-Aryl, N[(CH₂)ₙXR]CO(CH₂)ₙX-Aryl, N[(CH₂)ₙXR]SO₂(CH₂)ₙ-Aryl, N[(CH₂)ₙNRCOOR]CO(CH₂)ₙ-Aryl, N[(CH₂)ₙNR₂]CO(CH₂)ₙ-Aryl, N[(CH₂)ₙNR₂]CO(CH₂)ₙNR- Aryl, N[(CH₂)ₙNR₂]SO₂(CH₂)ₙ-Aryl, N[(CH₂)ₙXR]CO(CH₂)ₙ- Heteroaryl, N[(CH₂)ₙXR]CO(CH₂)ₙX-Heteroaryl, N[(CH₂)ₙXR]SO₂(CH₂)ₙ-Heteroaryl, N[(CH₂)ₙNRCOOR]CO(CH₂)ₙ-Heteroaryl, N[(CH₂)ₙNR₂]CO(CH₂)ₙ-Heteroaryl, N[(CH₂)ₙNR₂]CO(CH₂)ₙNR-Heteroary). N[(CH₂)ₙNR₂]SO₂(CH₂)ₙ-Heteroaryl, O(CH₂)ₙNR₂, X(CH₂)ₙNR₂, NCO(CH₂)ₙNR₂,
R⁶ unsubstituiertes oder einfach oder mehrfach durch Hal, NO₂, CN, OH, CF₃, OCH(CF₃)₂, OCOCH₃ oder A substituiertes Phenyl, 2-, 3- oder 4-Pyridyl, Pyrimidyl, Furyl oder Thienyl,
R⁷ H,
Aryl unsubstituiertes oder ein-, zwei- oder dreifach durch Hal, A, OH, OA, NH₂, NO₂, CN, COOH, COOA, CONH₂, NHCOA, NHCONH₂, NHSO₂A, CHO, COA, SO₂NH₂, SO₂A, -CH₂-COOH oder -OCH₂-COOH substituiertes Phenyl, Naphthyl oder Biphenyl,
Heteroaryl einen ein- oder zweikernigen unsubstituierten oder ein-, zwei- oder dreifach durch Hal, A, NO₂, NHA, NA₂, OA, COOA oder CN substituierten aromatischen Heterocyclus mit einem oder mehreren N-, O- und/oder S-Atomen,
m 0, 1 oder 2 und
n 0, 1, 2, 3, 4, 5, 6 oder 7
bedeuten, sowie ihre Solvate, Tautomere, Salze and Stereoisomere, einschließlich deren Mischungen in allen Verhältnissen.

2. Verbindungen nach Anspruch 1, worin Alkyl Methyl bedeutet.

3. Arzneimittel, umfassend mindestens eine Verbindung der Formel IA4 nach Anspruch 1 oder 2 und/oder ihre Salze, Solvate, Tautomere und Stereoisomere, einschließlich deren Mischungen in allen Verhältnissen, sowie gegebenenfalls Träger- und/oder Hilfsstoffe.

4. Mischung umfassend eine oder mehrere Verbindungen der Formel IA4 nach Anspruch 1 oder 2 sowie eine Menge einer oder mehrerer Verbindungen der Formel V, deren Analoga und/oder Metaboliten, 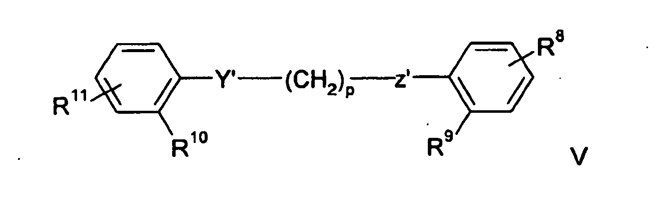 worin
Y' und Z' jeweils unabhängig voneinander O oder N bedeuten,
R⁹ und R¹⁰ jeweils unabhängig voneinander H, OH, Halogen, OC₁₋₁₀-Alkyl, OCF₃, NO₂ oder NH₂ bedeuten,
n eine ganze Zahl zwischen 2 und 6, jeweils einschließlich, bedeutet und
R⁸ und R¹¹ jeweils unabhängig voneinander an der meta- oder para-Position stehen und aus der Gruppe: 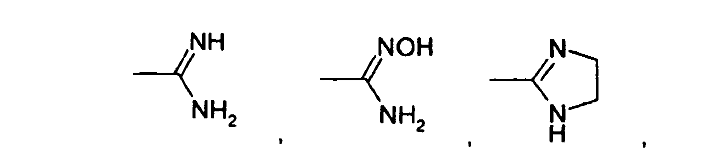 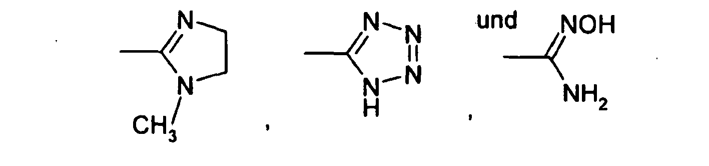 ausgewählt sind.

5. Mischung nach Anspruch 4, wobei als Verbindung der Formel V Pentamidin oder seine Salze ausgewählt sind.

6. Verwendung von Verbindungen der Formel IA4 nach Anspruch 1 oder 2 sowie ihrer Salze, Solvate, Tautomere und Stereoisomere, einschließlich deren Mischungen in allen Verhältnissen, oder der Mischung nach Anspruch 4 zur Herstellung eines Arzneimittels zur Behandlung von Krankheiten, die durch die Hemmung, Regulierung und/oder Modulation des mitotischen Motor-Proteins Eg5 beeinflusst werden können.

7. Verwendung von Verbindungen der Formel IA4 nach Anspruch 1 oder 2 oder der Mischung nach Anspruch 4 zur Herstellung eines Arzneimittels zur Behandlung und Prophylaxe von Krebskrankheiten.

8. Verwendung nach Anspruch 7, wobei die Krebskrankheiten mit einem Tumor aus der Gruppe der Tumoren des Plattenepithels, der Blase, des Magens, der Nieren, von Kopf und Hals, des Ösophagus, des Gebärmutterhalses, der Schilddrüse, des Darms, der Leber, des Gehirns, der Prostata, des Urogenitaltrakts, des lymphatischen Systems, des Magens, des Kehlkopf und/oder der Lunge einhergehen.

9. Verwendung nach Anspruch 8, wobei der Tumor aus der Gruppe von Monozytenleukämie, Lungenadenokarzinom, kleinzellige Lungenkarzinome, Bauchspeicheldrüsenkrebs, Glioblastome und Brustkarzinom und Kolokarzinom stammt.

10. Verwendung nach Anspruch 7, wobei die zu behandelnde Krebskrankheit ein Tumor des Blut- und Immunsystems ist.

11. Verwendung nach Anspruch 10, wobei der Tumor aus der Gruppe der akuten myelotischen Leukämie, chronischen myelotischen Leukämie, akuten lymphatischen Leukämie und/oder chronischen lymphatischen Leukämie stammt.

12. Verwendung von Verbindungen der Formel IA4 nach Anspruch 1 oder 2 und/oder ihrer physiologisch unbedenklichen Salze und Solvate zur Herstellung eines Arzneimittels zur Behandlung von Tumoren in Kombination mit einer therapeutisch wirksamen Menge einer oder mehrerer Verbindungen der Formel V, deren Analoga und/oder Metaboliten, 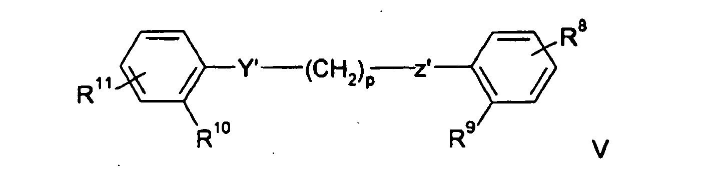 worin
Y' und Z' jeweils unabhängig voneinander O oder N bedeuten,
R⁹ und R¹⁰ jeweils unabhängig voneinander H, OH, Halogen, OC₁₋₁₀-Alkyl, OCF₃, NO₂ oder NH₂ bedeuten,
n eine ganze Zahl zwischen 2 und 6, jeweils einschließlich, bedeutet und
R⁸ und R¹¹ jeweils unabhängig voneinander an der meta- oder para-Position stehen und aus der Gruppe: 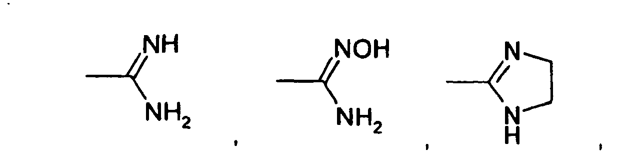 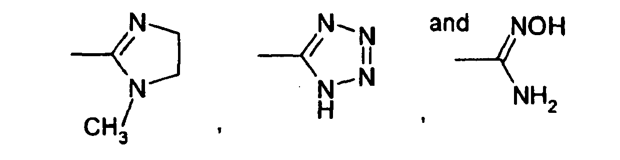 ausgewählt sind, wobei
die Verbindungen der Formel IA4 und die Verbindungen der Formel V, ihre Analoga und/oder ihre Metaboliten gleichzeitig oder innerhalb von 14 Tagen voneinander in Mengen verabreicht werden, die ausreichen, um das Wachstum eines Tumors oder von anderen hyperproliferativen Zellen zu hemmen.

13. Verwendung nach Anspruch 12, wobei als Verbindung der Formel V Pentamidin oder seine Salze verwendet werden.

14. Verwendung von Verbindungen der Formel IA4 nach Anspruch 1 oder 2 und/oder ihrer physiologisch unbedenklichen Salze und Solvate zur Herstellung eines Arzneimittels zur Behandlung von Tumoren, wobei eine therapeutisch wirksame Menge einer Verbindung der Formel IA4 in Kombination mit Radiotherapie und einer Verbindung aus der Gruppe: 1) Östrogenrezeptormodulator, 2) Androgenrezeptormodulator, 3) Retinoidrezeptormodulator, 4) Zytotoxikum, 5) antiproliferatives Mittel, 6) Prenyl-Proteintransferasehemmer, 7) HMG-CoA-Reduktase-Hemmer, 8) HIV-Protease-Hemmer, 9) Reverse-Transkriptase-Hemmer und 10) weiterer Angiogenese-Hemmer verabreicht wird.

## Claims

1. Compounds of the formula IA4 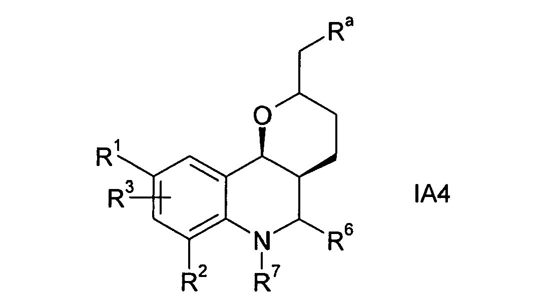 in which
R¹ denotes A, CF₃, OCF₃, SA, SCN, CH₂CN, OCOA, Hal or SCF₃,
R² denotes F or H,
R³ denotes F or H,
R^{a} denotes 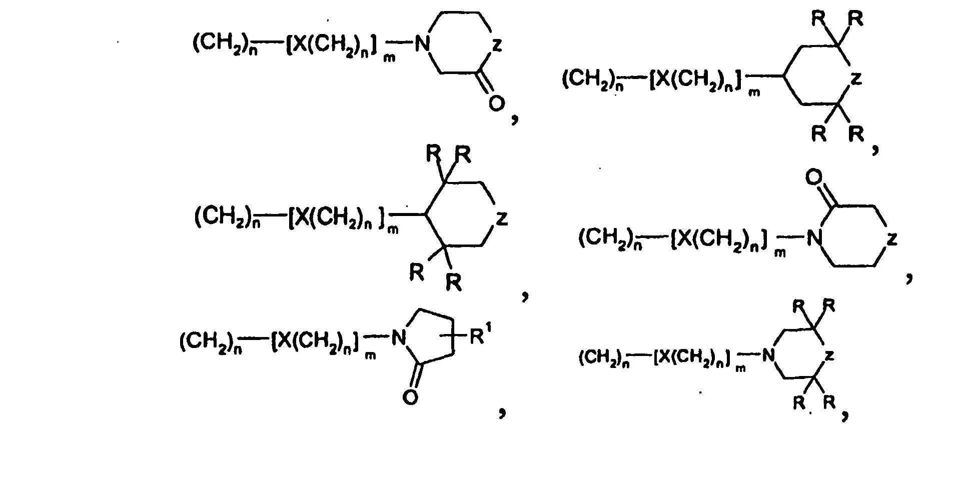 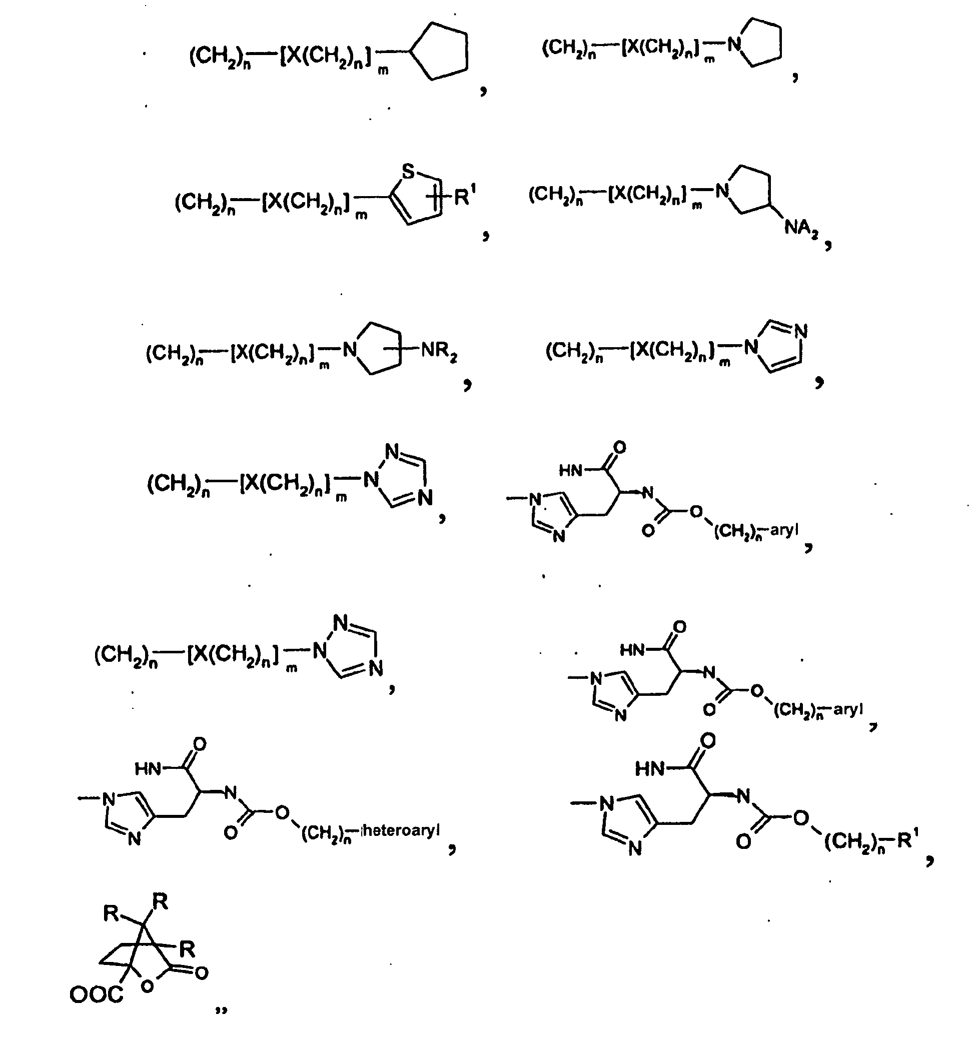 OR, NHR₂, NR₂, OR, NHR₂, NR₂, NR(CH₂)ₙ-aryl, NR(CH₂)ₙOR, COOR, N-pyrrolidone radical, OCOR, NR(CH₂)ₙNR₂, N[(CH₂)ₙNR₂]CO(CH₂)ₙ-aryl, N[(CH₂)ₙNHCOOR]CO-aryl, R¹, N[CH₂(CH₂)ₙOR]₂, NR(CH₂)ₙNCOOR, X(CH₂)ₙX(CH₂)ₙXR, NR(CH₂)ₙX(CH₂)ₙOH, NR(CH₂)ₙO(CH₂)ₙOH, (CH₂)ₙCOOR, O(CO)NR(CH₂)ₙOR, O(CO)(CH₂)ₙNR₂, NR(CH₂)ₙNR₂, N[(CH₂)ₙNR₂]CO(CH₂)ₙ-aryl, N[(CH₂)ₙXR]CO(CH₂)ₙ-aryl, N[(CH₂)ₙXR]CO(CH₂)ₙ-heteroaryl, N[(CH₂)ₙNR₂]CO(CH₂)ₙ-heteroaryl, N[(CH₂)ₙNR₂]CO(CH₂)ₙR¹, N(R)(CH₂)ₙN(R)COOR, XCOO(CH₂)ₙNR₂, OSO₂A, OSO₂CF₃, OSO₂Ar, OCONR₂, OCH₂(CH₂)ₙNR₂,
R denotes H or A, in the case of geminal radicals R together also denote -(CH₂)₅-, -(CH₂)₄-, -(CH₂)ₙ-X-(CH₂)ₙ or -(CH₂)ₙ-Z-(CH₂)ₙ,
A denotes alkyl or cycloalkyl, in which one or more H atoms may be replaced by Hal,
Hal denotes F or Cl,
X denotes O, S or NR,
Z denotes CH₂, X, CHCONH₂, CH(CH₂)ₙNRCOOR, CHNRCOOR, NCO, CH(CH₂)ₙCOOR, NCOOR, CH(CH₂)ₙOH, N(CH₂)ₙOH, CHNH₂, CH(CH₂)ₙNR₂, CH(CH₂)ₙNR₂, C(OH)R, CHNCOR, CH(CH₂)ₙ-aryl, CH(CH₂)ₙ-heteroaryl, CH(CH₂)ₙR¹, N(CH₂)ₙCOOR, CH(CH₂)ₙX(CH₂)ₙ-aryl, CH(CH₂)ₙX(CH₂)ₙ-heteroaryl, N(CH₂)ₙCONR₂, XCONR(CH₂)ₙNR₂, N[(CH₂)ₙXCOOR]CO(CH₂)ₙ-aryl, N[(CH₂)ₙXR]CO(CH₂)ₙ-aryl, N[(CH₂)ₙXR]CO(CH₂)ₙX-aryl, N[(CH₂)ₙXR]SO₂(CH₂)ₙ-aryl, N[(CH₂)ₙNRCOOR]CO(CH₂)ₙ-aryl, N[(CH₂)ₙNR₂]CO(CH₂)ₙ-aryl, N[(CH₂)ₙNR₂]CO(CH₂)ₙNR-aryl, N[(CH₂)ₙNR₂]SO₂(CH₂)ₙ-aryl, N[(CH₂)ₙXR]CO(CH₂)ₙ-heteroaryl, N[(CH₂)ₙXR]CO(CH₂)ₙX-heteroaryl, N[(CH₂)ₙXR]SO₂(CH₂)ₙ-heteroaryl, N[(CH₂)ₙNRCOOR]CO(CH₂)ₙ-heteroaryl, N[(CH₂)ₙNR₂]CO(CH₂)ₙ-heteroaryl, N[(CH₂)ₙNR₂]CO(CH₂)ₙNR-heteroaryl, N[(CH₂)ₙNR₂]SO₂(CH₂)ₙ-heteroaryl, O(CH₂)ₙNR₂, X(CH₂)ₙNR₂, NCO(CH₂)ₙNR₂,
R⁶ denotes phenyl, 2-, 3- or 4-pyridyl, pyrimidyl, furyl or thienyl, each of which is unsubstituted or mono- or poly- substituted by Hal, NO₂, CN, OH, CF₃, OCH(CF₃)₂, OCOCH₃ or A,
R⁷ denotes H,
aryl denotes phenyl, naphthyl or biphenyl, each of which is unsubstituted or mono-, di- or trisubstituted by Hal, A, OH, OA, NH₂, NO₂, CN, COOH, COOA, CONH₂, NHCOA, NHCONH₂, NHSO₂A, CHO, COA, SO₂NH₂, SO₂A, -CH₂-COOH or -OCH₂-COOH,
heteroaryl denotes mono- or bicyclic aromatic heterocycle having one or more N, O and/or S atoms which is unsubstituted or mono-, di- or trisubstituted by Hal, A, NO₂, NHA, NA₂, OA, COOA or CN,
m denotes 0, 1 or 2, and
n denotes 0, 1, 2, 3, 4, 5, 6 or 7,
and solvates, tautomers, salts and stereoisomers thereof, including mixtures thereof in all ratios.

2. Compounds according to Claim 1 in which alkyl denotes methyl.

3. Medicaments comprising at least one compound of the formula IA4 according to Claim 1 or 2 and/or salts, solvates, tautomers and stereoisomers thereof, including mixtures thereof in all ratios, and optionally excipients and/or adjuvants.

4. Mixture comprising one or more compounds of the formula IA4 according to Claim 1 or 2 and an amount of one or more compounds of the formula V, analogues and/or metabolites thereof, 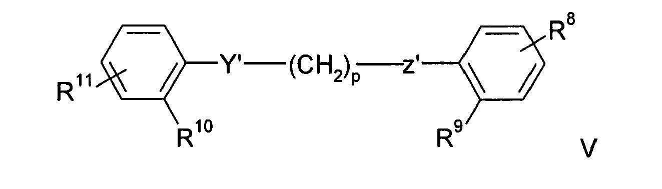 in which
Y' and Z' each, independently of one another, denote O or N,
R⁹ and R¹⁰ each, independently of one another, denote H, OH, halogen, OC₁₋₁₀-alkyl, OCF₃, NO₂ or NH₂,
n denotes an integer between 2 and 6 inclusive,
and
R⁸ and R¹¹ are each, independently of one another, in the meta- or para-position and are selected from the group:

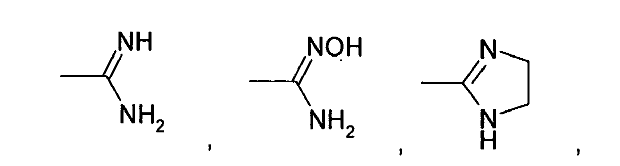

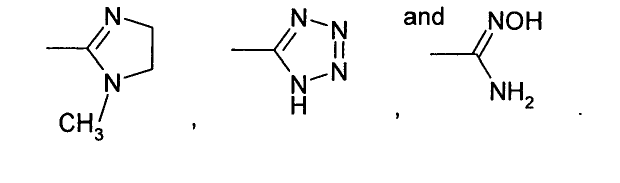

5. Mixture according to Claim 4, where the compound of the formula V selected is pentamidine or salts thereof.

6. Use of compounds of the formula IA4 according to Claim 1 or 2 and salts, solvates, tautomers and stereoisomers thereof, including mixtures thereof in all ratios, or the mixture according to Claim 4 for the preparation of a medicament for the treatment of diseases which can be influenced by the inhibition, regulation and/or modulation of the mitotic motor protein Eg5.

7. Use of compounds of the formula IA4 according to Claim 1 or 2 or the mixture according to Claim 4 for the preparation of a medicament for the treatment and prophylaxis of cancer diseases.

8. Use according to Claim 7, where the cancer diseases are associated with a tumour from the group of tumours of the squamous epithelium, the bladder, the stomach, the kidneys, of head and neck, the oesophagus, the cervix, the thyroid, the intestine, the liver, the brain, the prostate, the urogenital tract, the lymphatic system, the stomach, the larynx and/or the lung.

9. Use according to Claim 8, where the tumour originates from the group of monocytic leukaemia, lung adenocarcinoma, small-cell lung carcinomas, pancreatic cancer, glioblastomas and breast carcinoma and colon carcinoma.

10. Use according to Claim 7, where the cancer disease to be treated is a tumour of the blood and immune system.

11. Use according to Claim 10, where the tumour originates from the group of acute myeloid leukaemia, chronic myeloid leukaemia, acute lymphatic leukaemia and/or chronic lymphatic leukaemia.

12. Use of compounds of the formula IA4 according to Claim 1 or 2 and/or physiologically acceptable salts and solvates thereof for the preparation of a medicament for the treatment of tumours in combination with a therapeutically effective amount of one or more compounds of the formula V, analogues and/or metabolites thereof, 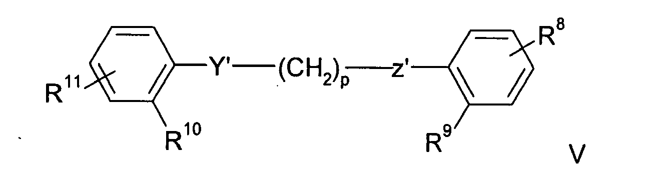 in which
Y' and Z' each, independently of one another, denote O or N,
R⁹ and R¹⁰ each, independently of one another, denote H, OH, halogen, OC₁₋₁₀-alkyl, OCF₃, NO₂ or NH₂,
n denotes an integer between 2 and 6 inclusive,
and
R⁸ and R¹¹ are each, independently of one another, in the meta- or para-position and are selected from the group: 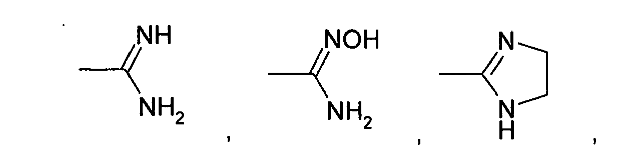 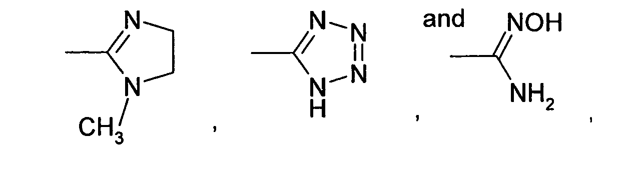 where
the compounds of the formula IA4 and the compounds of the formula V, analogues thereof and/or metabolites thereof are administered simultaneously or within 14 days of one another in amounts which are sufficient to inhibit the growth of a tumour or of other hyperproliferative cells.

13. Use according to Claim 12, where the compound of the formula V used is pentamidine or salts thereof.

14. Use of compounds of the formula IA4 according to Claim 1 or 2 and/or physiologically acceptable salts and solvates thereof for the preparation of a medicament for the treatment of tumours, where a therapeutically effective amount of a compound of the formula IA4 is administered in combination with radiotherapy and a compound from the group: 1) oestrogen receptor modulator, 2) androgen receptor modulator, 3) retinoid receptor modulator, 4) cytotoxic agent, 5) antiproliferative agent, 6) prenyl-protein transferase inhibitor, 7) HMG-CoA reductase inhibitor, 8) HIV protease inhibitor, 9) reverse transcriptase inhibitor and 10) further angiogenesis inhibitors.

## Revendications

1. Composés de formule IA4 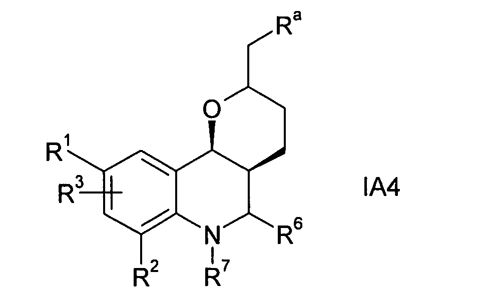 dans laquelle
R¹ désigne A, CF₃, OCF₃, SA, SCN, CH₂CN, OCOA, Hal ou SCF₃,
R² désigne F ou H,
R³ désigne F ou H,
R^{a} désigne 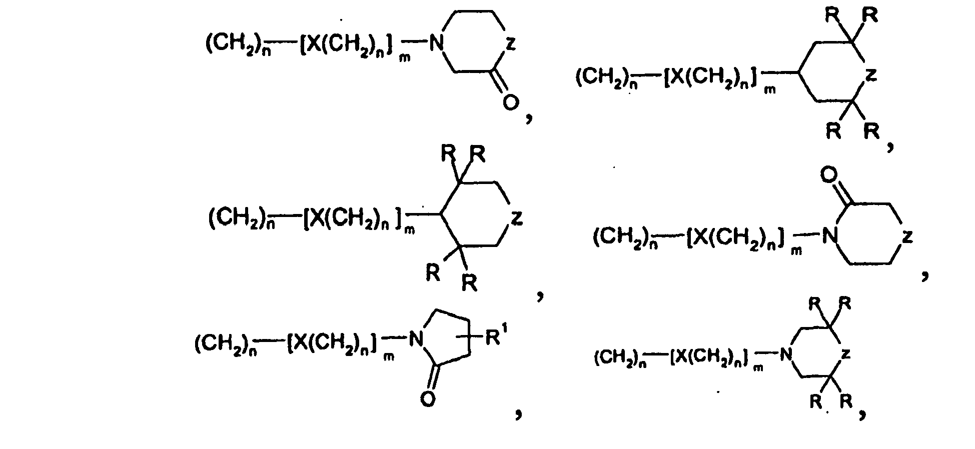 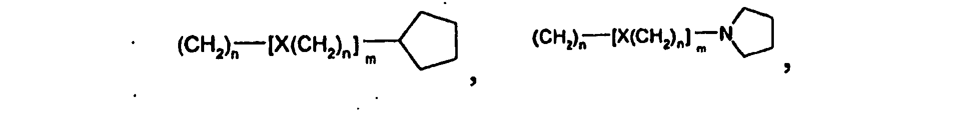 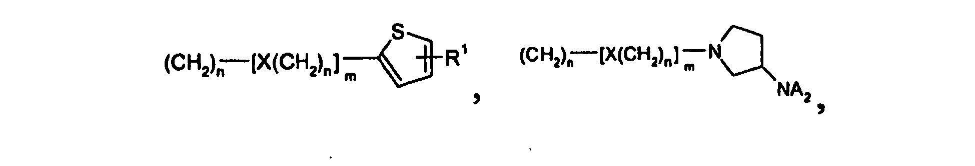 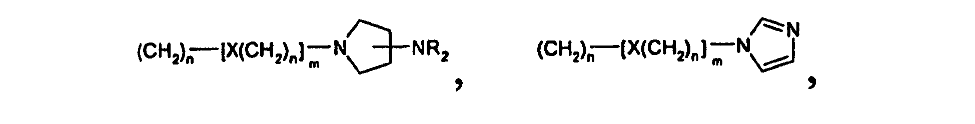 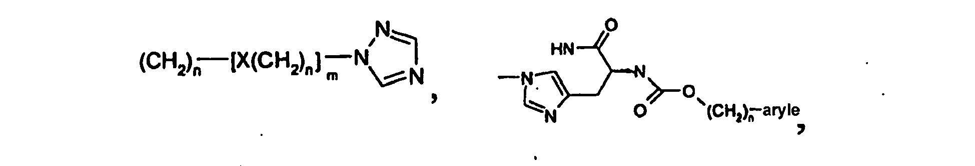 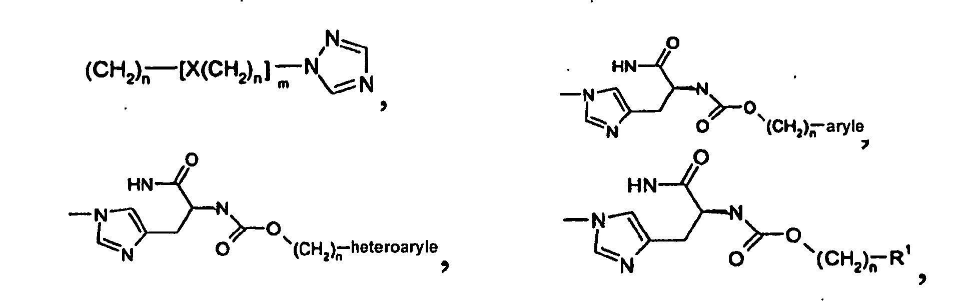 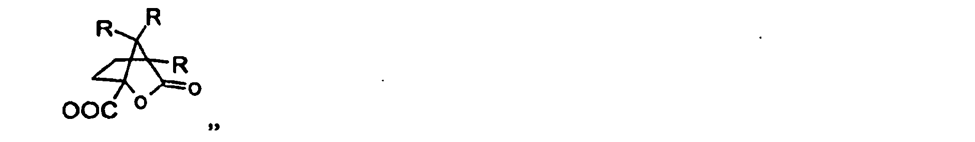 OR, NHR₂, NR₂, OR, NHR₂, NR₂, NR(CH₂)ₙ-aryle, NR(CH₂)ₙOR, COOR, un radical N-pyrrolidone, OCOR, NR(CH₂)ₙNR₂, N[(CH₂)ₙNR₂]CO(CH₂)ₙ-aryle, N[(CH₂)ₙNHCOOR]CO-aryle, R¹, N[CH₂(CH₂)ₙOR]₂, NR(CH₂)ₙNCOOR, X(CH₂)ₙX(CH₂)nXR, NR(CH₂)ₙX(CH₂)ₙOH, NR(CH₂)ₙO(CH₂)nOH, (CH₂)ₙCOOR, O(CO)NR(CH₂)ₙOR, O(CO)(CH₂)ₙNR₂, NR(CH₂)ₙNR₂, N[(CH₂)ₙNR₂]CO(CH₂)ₙ-aryle, N[(CH₂)ₙXR]CO(CH₂)ₙ-aryle, N[(CH₂)ₙXR]CO(CH₂)ₙ-hétéroaryle, N[(CH₂)ₙNR₂]CO(CH₂)ₙ-hétéroaryle, N[(CH₂)ₙNR₂]CO(CH₂)ₙR¹, N(R)(CH₂)ₙN(R)COOR, . XCOO(CH₂)ₙNR₂, OSO₂A, OSO₂CF₃, OSO₂Ar, OCONR₂, OCH₂(CH₂)ₙNR₂,
R désigne H ou A, dans le cas de radicaux géminés R désignent également ensemble -(CH₂)₅-, -(CH₂)₄-, -(CH₂)ₙ-X-(CH₂)ₙ ou -(CH₂)ₙ-Z-(CH₂)ₙ,
A désigne alkyle ou cycloalkyle, où un ou plusieurs atomes de H peuvent être remplacés par Hal,
Hal désigne F ou CI,
X désigne O, S ou NR,
Z désigne CH₂, X, CHCONH₂, CH(CH₂)ₙNRCOOR, CHNRCOOR, NCO, CH(CH₂)ₙCOOR, NCOOR, CH(CH₂)ₙOH, N(CH₂)ₙOH, CHNH₂, CH(CH₂)ₙNR₂, CH(CH₂)ₙNR₂, C(OH)R, CHNCOR, CH(CH₂)ₙ-aryle, CH(CH₂)ₙ-hétéroaryle, CH(CH₂)ₙR¹, N(CH₂)ₙCOOR, CH(CH₂)ₙX(CH₂)ₙ-aryle, CH(CH₂)ₙX(CH₂)ₙ-hétéroaryle, N(CH₂)ₙCONR₂, XCONR(CH₂)ₙNR₂, N[(CH₂)ₙXCOOR]CO(CH₂)ₙ-aryle, N[(CH₂)ₙXR]CO(CH₂)ₙ-aryle, N[(CH₂)ₙXR]CO(CH₂)ₙX- aryle, N[(CH₂)ₙXR]SO₂(CH₂)ₙ-aryle, N[(CH₂)ₙNRCOOR]CO(CH₂)ₙ-aryle, N[(CH₂)ₙNR₂]CO(CH₂)ₙ-aryle, N[(CH₂)ₙNR₂]CO(CH₂)ₙ-NR-aryle, N[(CH₂)ₙNR₂]SO₂(CH₂)ₙ-aryle, N[(CH₂)ₙXR]CO(CH₂)ₙ-hétéroaryle, N [(CH₂)ₙXR]CO(CH₂)ₙX-hétéroaryle, N[(CH₂)ₙXR]SO₂(CH₂)ₙ-hétéroaryle, N[(CH₂)ₙNRCOOR]CO(CH₂)ₙ-hétéroaryle, N[(CH₂)ₙNR₂]CO(CH₂)ₙ-hétéroaryle, N[(CH₂)ₙNR₂]CO(CH₂)ₙNR-hétéroaryle, N[(CH₂)ₙNR₂]SO₂(CH₂)ₙ-hétéroaryle, O(CH₂)ₙNR₂, X(CH₂)ₙNR₂, NCO(CH₂)ₙNR₂,
R⁶ désigne phényle, 2-, 3- ou 4-pyridyle, pyrimidyle, furyle ou thiényle, chacun d'entre eux étant non substitué ou mono- ou polysubstitué par Hal, NO₂, CN, OH, CF₃, OCH(CF₃)₂, OCOCH₃ ou A,
R⁷ désigne H,
aryle désigne phényle, naphtyle ou biphényle, chacun d'entre eux étant non substitué ou mono-, di- ou trisubstitué par Hal, A, OH, OA, NH₂, NO, CN, COOH, COOA, CONH₂, NHCOA, NHCONH₂, NHSO₂A, CHO, COA, SO₂NH₂, SO₂A, -CH₂-COOH ou -OCH₂-COOH,
hétéroaryle désigne hétérocycle mono- ou bicyclique aromatique ayant un ou plusieurs atomes de N, O et/ou S qui est non substitué ou mono-, di- ou trisubstitué par Hal, A, NO₂, NHA, NA₂, OA, COOA ou CN,
m désigne 0, 1 ou 2, et
n désigne 0, 1, 2, 3, 4, 5, 6 ou 7,
et les solvats, tautomères, sels et stéréoisomères de ceux-ci, y compris des mélanges de ceux-ci en toutes proportions.

2. Composés selon la revendication 1, dans lesquels alkyle désigne méthyle.

3. Médicaments comprenant au moins un composé de formule IA4 selon la revendication 1 ou 2 et/ou et des sels, solvats, tautomères et stéréoisomères de celui-ci, y compris des mélanges de ceux-ci en toutes proportions, et éventuellement des excipients et/ou des adjuvants.

4. Mélange comprenant un ou plusieurs composés de formule IA4 selon la revendication 1 ou 2, et une quantité d'un ou plusieurs composés de formule V, d'analogues et/ou de métabolites de ceux-ci, 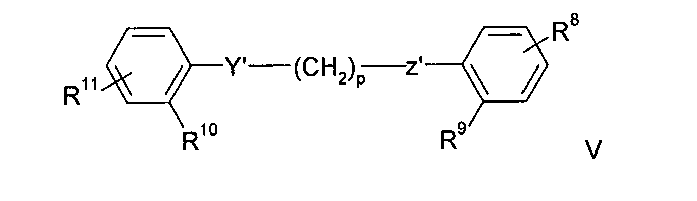 où
Y' et Z' désignent chacun, indépendamment l'un de l'autre, O ou N,
R⁹ et R¹⁰ désignent chacun, indépendamment l'un de l'autre, H, OH, halogène, OC₁₋₁₀-alkyle, OCF₃, NO₂ ou NH₂,
n désigne un nombre entier compris entre 2 et 6 inclus,
et
R⁸ et R¹¹ sont chacun, indépendamment l'un de l'autre, en position méta ou para et sont choisis dans le groupe constitué par :

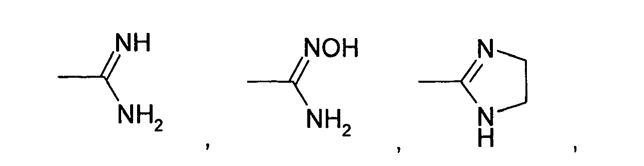

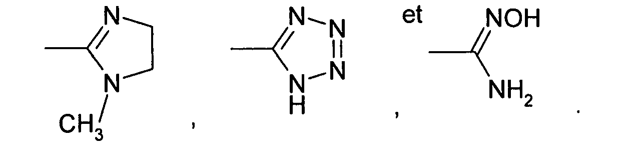

5. Mélange selon la revendication 4, dans lequel le composé de formule V sélectionné est la pentamidine ou des sels de celle-ci.

6. Utilisation de composés de formule IA4 selon la revendication 1 ou 2 et de sels, solvats, tautomères et stéréoisomères de ceux-ci, y compris des mélanges de ceux-ci en toutes proportions, ou du mélange selon la revendication 4, pour la préparation d'un médicament destiné au traitement de maladies pouvant être influencées par l'inhibition, la régulation et/ou la modulation de la protéine moteur mitotique Eg5.

7. Utilisation de composés de formule IA4 selon la revendication 1 ou 2 ou du mélange selon la revendication 4, pour la préparation d'un médicament destiné au traitement et à la prophylaxie de maladies cancéreuses.

8. Utilisation selon la revendication 7, dans laquelle les maladies cancéreuses sont associées à une tumeur issue du groupe des tumeurs de l'épithélium squameux, de la vessie, de l'estomac, du rein, de la tête et du cou, de l'oesophage, du col de l'utérus, de la thyroïde, des intestins, du foie, du cerveau, de la prostate, du tractus urogénital, du système lymphatique, de l'estomac, du larynx et/ou du poumon.

9. Utilisation selon la revendication 8, dans laquelle la tumeur est issue du groupe de la leucémie monocytique, de l'adénocarcinome du poumon, des carcinomes du poumon à petites cellules, du cancer du pancréas, des glioblastomes et du carcinome du sein et du carcinome du côlon.

10. Utilisation selon la revendication 7, dans laquelle la maladie cancéreuse à traiter est une tumeur du sang et du système immunitaire.

11. Utilisation selon la revendication 10, dans laquelle la tumeur est issue du groupe de la leucémie myéloïde aiguë, la leucémie myéloïde chronique, la leucémie lymphatique aiguë et/ou la leucémie lymphatique chronique.

12. Utilisation de composés de formule IA4 selon la revendication 1 ou 2 et/ou de sels et solvats physiologiquement acceptables de ceux-ci, pour la préparation d'un médicament destiné au traitement de tumeurs en association avec une quantité thérapeutiquement efficace d'un ou plusieurs composés de formule V, d'analogues et/ou de métabolites de ceux-ci, 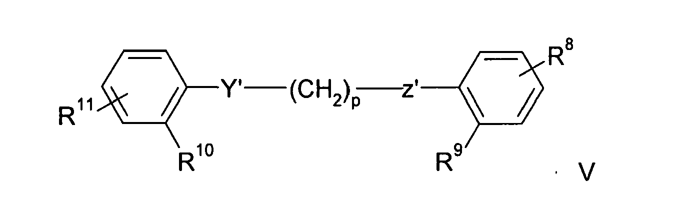 où
Y' et Z' désignent chacun, indépendamment l'un de l'autre, O ou N,
R⁹ et R¹⁰ désignent chacun, indépendamment l'un de l'autre, H, OH, halogène, OC₁₋₁₀-alkyle, OCF₃, NO₂ ou NH₂,
n désigne un nombre entier compris entre 2 et 6 inclus,
et
R⁸ et R¹¹ sont chacun, indépendamment l'un de l'autre, en position méta ou para et sont choisis dans le groupe constitué par : 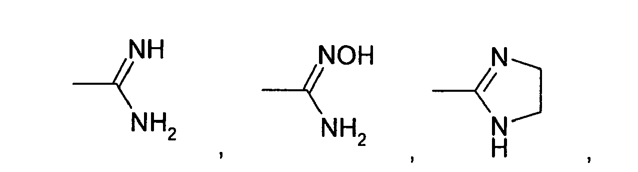 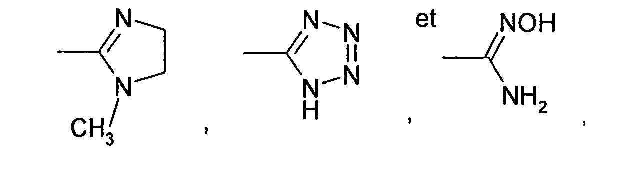 où
les composés de formule IA4 et les composés de formule V, les analogues de ceux-ci et/ou les métabolites de ceux-ci, sont administrés simultanément ou dans les 14 jours l'un par rapport à l'autre en des quantités qui sont suffisantes pour inhiber la croissance d'une tumeur ou d'autres cellules hyperprolifératives.

13. Utilisation selon la revendication 12, dans laquelle le composé de formule V utilisé est la pentamidine ou des sels de celle-ci.

14. Utilisation de composés de formule IA4 selon la revendication 1 ou 2 et/ou de sels et solvats physiologiquement acceptables de ceux-ci, pour la préparation d'un médicament destiné au traitement de tumeurs, dans laquelle une quantité thérapeutiquement efficace d'un composé de formule IA4 est administrée en association avec une radiothérapie et un composé issu du groupe : 1) un modulateur du récepteur des oestrogènes, 2) un modulateur du récepteur des androgènes, 3) un modulateur du récepteur des rétinoïdes, 4) un agent cytotoxique, 5) un agent antiprolifératif, 6) un inhibiteur de la prényl-protéine transférase, 7) un inhibiteur de la HMG-CoA réductase, 8) un inhibiteur de la protéase du VIH, 9) un inhibiteur de la transcriptase inverse et 10) d'autres inhibiteurs de l'angiogenèse.
